# EUROPEAN PATENT SPECIFICATION

(11) **EP 4 408 329 B1**
(45) Date of publication and mention of the grant of the patent: **10.06.2026**
(21) Application number: 22875262.2
(22) Date of filing: 14.09.2022
(51) Int. Cl.: A61B 34/30, A61B 34/00, A61B 90/30, A61B 90/98, A61B 34/37, A61B 1/00, A61B 34/10, A61B 34/20, A61B 90/00, A61G 13/10

(54) **REAL-TIME 3D ROBOTIC STATUS**
ECHTZEIT-3D-ROBOTERSTATUS
ÉTAT ROBOTIQUE 3D EN TEMPS RÉEL

(30) Priority: 28.09.2021 US 202163249491 P
(43) Date of publication of application: 07.08.2024
(73) Proprietor: Auris Health, Inc., Santa Clara, CA 95054 (US)
(72) Inventor: STACHULSKI, Melissa, Redwood City, California 94065 (US); SCHMITT, Fabien Y., Redwood City, California 94065 (US); JOHNSON, Eric Mark, Redwood City, California 94065 (US)
(74) Representative: Carpmaels & Ransford LLP
(86) International application number: PCT/IB2022/058653
(87) International publication number: WO 2023/052881

(56) References cited:
- WO-A1-2021/050087
- US-A1- 2017 181 798
- US-A1- 2019 005 848
- US-A1- 2019 183 591
- US-A1- 2020 069 373
- US-A1- 2021 068 907
- US-A1- 2021 251 706
- US-B2- 10 828 120

## Description

### TECHNICAL FIELD

The systems and methods disclosed herein are directed to robotic medical systems, and more particularly to communicating status of robotic medical systems for medical procedures.

### BACKGROUND

A robotically enabled medical system is capable of performing a variety of medical procedures, including both minimally invasive procedures, such as laparoscopy, and non-invasive procedures, such as endoscopy (e.g., bronchoscopy, ureteroscopy, gastroscopy, etc.).

Such robotic medical systems may include robotic arms configured to control the movement of medical tool(s) during a given medical procedure. In order to achieve a desired pose of a medical tool, a robotic arm may be placed into a particular pose during a set-up process or during teleoperation. Some robotically enabled medical systems may include an arm support (e.g., a bar) that is connected to respective bases of the robotic arms and supports the robotic arms. US2021251706A1 describes a robotic surgical system and method for providing a stadium view with arm set-up guidance is provided. In one embodiment, a robotic surgical system comprises a plurality of robotic arms, a display device, and a processor. The processor is configured to render, on the display device, a graphical representation of the plurality of robotic arms in their current positions, as well as user-guidance information on how to move the plurality of robotic arms to a different position. Other embodiments are provided.

### SUMMARY

The invention is defined in the appended set of claims.

Robotic medical systems are complex systems with many moving parts that can be controlled via various digital touchpoints located on the system and/or via user interfaces that are coupled to the system. Before a procedure starts, an operator (e.g., a physician assistant, medical personnel, etc.) may be required to set up the robotic arms and/or adjustable arm support(s) to a desired overall configuration. This usually involves adjusting each arm and/or arm support individually (e.g., separately). During surgery (e.g., teleoperation), a physician typically resides at a physician console and drives the robotic arms from the physician console (e.g., using haptic input devices (HID) located at the physician console, which in turn control movement of the robotic arms). After surgery, the physician assistant may be required to adjust the robotic arms into a stowing position. For each of these steps, it is important for the status of the robotic medical system (e.g., arm / arm support selections, progress, completion state, system errors, ways to resolve the errors, etc.) to be communicated to the user in a manner that is easily understandable to the user. In some circumstances, it would also be advantageous to have a medical robotic system that is capable of guiding the user through various workflow steps visually and intuitively.

Accordingly, an improved robotic medical system is desirable. In particular, there is a need for a robotic medical system that provides instant feedback about current status of the robotic medical system in a manner that is clear, concise, and easily understandable to the user. Real-time (e.g., immediate) feedback of the robotic medical system's status, selections, positional changes, and/or faults can increase the user's confidence and understanding of the system, improve the performance of teleoperation, and enhance user experience.

As disclosed herein, a robotic medical system is configured to display rendering(s) (e.g., two-dimensional (2-D) or three-dimensional (3-D) rendering(s)) that represent a status of the robotic medical system in real-time. The renderings (e.g., 2-D or 3-D rendering(s)) can include graphical representation(s) of one or more of robotic arms, adjustable arm supports, and/or patient support platform of the robotic medical system. In some embodiments disclosed herein, the robotic medical system can update the rendering(s) (e.g., 2-D or 3-D rendering(s)) in accordance with a pre-programmed workflow corresponding to a procedure, to guide a user (e.g., a physician and/or physician assistant) through the procedure via the rendering(s) (e.g., 2-D or 3-D rendering(s)). In some embodiments, the pre-programmed workflow can include a pre-operative stage, an intra-operative stage, and/or a post-operative stage. Each of the stages can include one or more respective steps. The rendering(s) (e.g., 2-D or 3-D rendering(s)) can be used to communicate real-time robotic status for each of the stages (and/or steps), and in particular positional changes of the robotic system.

As disclosed herein, the rendering(s) (e.g., 2-D rendering(s), 3-D rendering(s), 3-D models, etc.) can be used to communicate robotic status in response to both robotic control and manual control of the robotic medical system. The displayed rendering (e.g., 3-D rendering) can move and update to reflect positional changes to accurately reflect the actual system state and positions of robotic arms, arm supports, and/or patient support platform. In some embodiments disclosed herein, a field of view of the robotic system (e.g., as seen by a virtual camera of the robotic system) can dynamically change to include (e.g., focus on) a specific portion of the robotic medical system.

Accordingly, the systems and/or methods disclosed herein advantageously improves operator safety during setup and patient and/or operator safety during surgery. It also ensures that the operator is informed about the status of the system in real-time.

The systems, methods and devices of this disclosure each have several innovative aspects, no single one of which is solely responsible for the desirable attributes disclosed herein.

In accordance with some embodiments of the present disclosure, a robotic medical system includes one or more robotic arms. The robotic medical system includes one or more displays. The robotic medical system also includes one or more processors and memory. The memory stores instructions that, when executed by the one or more processors, cause the one or more processors to display a three-dimensional (3-D) rendering that includes a graphical representation of the one or more robotic arms, and update the 3-D rendering in accordance with a pre-programmed workflow corresponding to a procedure, to guide a user through the procedure.

In some embodiments, the robotic medical system further includes a patient support platform. The memory further stores instructions that, when executed by the one or more processors, cause the one or more processors to execute a spatial configuration adjustment of the one or more robotic arms relative to the patient support platform in accordance with the workflow. The memory further includes instructions that, when executed by the one or more processors, cause the one or more processors to update the 3-D rendering to reflect positional changes of the one or more robotic arms according to the spatial configuration adjustment.

In some embodiments, the memory further stores instructions that, when executed by the one or more processors, cause the one or more processors to cause movement of the patient support platform from a first position to a second position in accordance with the workflow, and update the 3-D rendering to reflect the movement of the patient support platform.

In some embodiments, the robotic medical system further includes one or more adjustable arm supports that are movably coupled to the one or more robotic arms. The memory further stores instructions that, when executed by the one or more processors, cause the one or more processors to cause movement of the one or more adjustable arm supports relative to the one or more robotic arms in accordance with the workflow, and update the 3-D rendering to reflect a positional change of the one or more adjustable arm supports.

In some embodiments, the memory further stores instructions that, when executed by the one or more processors, cause the one or more processors to receive user selection of a portion of the 3-D rendering. The memory further includes instructions that, when executed by the one or more processors, cause the one or more processors to, in accordance with the user selection, display the user-selected portion in a visually distinctive manner from other portions of the 3-D rendering.

In some embodiments, the pre-programmed workflow includes one or more stages, including: a pre-operative stage, an intra-operative stage, and/or a post-operative stage.

In some embodiments, a step of the pre-operative stage includes deploying the one or more robotic arms from a stowed position into a deployed position.

In some embodiments, a step of the pre-operative stage includes moving the one or more robotic arms into a draping pose.

In some embodiments, a step of the pre-operative stage includes placing the one or more robotic arms in a docked state.

In some embodiments, the robotic medical system further includes an adjustable arm support that is movably coupled to the one or more robotic arms. The robotic medical system also includes a patient support platform. A step of the intra-operative procedure includes leveling the adjustable arm support and the patient support platform.

In some embodiments, each of the one or more stages of the pre-programmed workflow includes one or more respective steps.

In some embodiments, the memory further stores instructions that, when executed by the one or more processors, cause the one or more processors to identify a step of the procedure to which the robotic medical system corresponds and cause movement of a portion of a first robotic arm of the one or more robotic arms in accordance with the identified step. The memory further includes instructions that, when executed by the one or more processors, cause the one or more processors to: during the movement, update the 3-D rendering to display the portion of the first robotic arm with a first visual representation that is visually distinct from other portions of the first robotic arm, and display a positional change of the portion of the first robotic arm according to the movement.

In some embodiments, displaying the portion of the first robotic arm with the first visual representation comprises displaying the portion with a first color that is distinct from a color corresponding to the other portions of the first robotic arm.

In some embodiments, the memory further stores instructions that, when executed by the one or more processors, cause the one or more processors to, in accordance with the movement, display a progress bar for visualizing progress of the identified step that is being executed.

In some embodiments, the memory further stores instructions that, when executed by the one or more processors, cause the one or more processors to, in accordance with the movement, produce an audio signal.

In some embodiments, the memory further stores instructions that, when executed by the one or more processors, cause the one or more processors to determine whether the step has been completed, and in accordance with a determination that the step has been completed, update the 3-D rendering to display the portion of the first robotic arm in a second visual representation that is distinct from the first visual representation.

In some embodiments, the first visual representation corresponds to a first color and the second visual representation corresponds to a second color that is distinct from the first color.

In some embodiments, the robotic medical system further includes an adjustable arm support that is movably coupled to the one or more robotic arms are coupled. The memory further stores instructions that, when executed by the one or more processors, cause the one or more processors to identify a step of procedure to which the robotic medical system corresponds and cause movement of the adjustable arm support in accordance with the identified step. The memory further includes instructions that, when executed by the one or more processors, cause the one or more processors to: during the movement, update the 3-D rendering to display the adjustable arm support with a first visual representation that is visually distinct from the one or more robotic arms, and display a positional change of the adjustable arm support according to the movement.

In some embodiments, displaying the adjustable arm support with the first visual representation comprises displaying the adjustable arm support with a first color that is distinct from a color corresponding to the one or more robotic arms.

In some embodiments, the robotic medical system further includes a patient support platform. The memory further stores instructions that, when executed by the one or more processors, cause the one or more processors to identify a step of procedure to which the robotic medical system corresponds and cause movement of at least a portion of the patient support platform. The memory further includes instructions that, when executed by the one or more processors, cause the one or more processors to: during the movement, update the 3-D rendering to, display the at least a portion of the patient support platform with a first visual representation that is visually distinct from the one or more robotic arms and/or other portions of the patient support platform, and display a positional change of the at least a portion of the patient support platform according to the movement.

In some embodiments, displaying the at least a portion of the patient support platform with the first visual representation comprises displaying the at least a portion of the patient support platform with a first color that is distinct from a color corresponding to the one or more robotic arms.

In some embodiments, the memory further stores instructions that, when executed by the one or more processors, cause the one or more processors to, in accordance with a determination that the robotic medical system is executing a specific step of the workflow, adjust a field of view of a virtual camera of the robotic medical system to include a specific portion of the one or more robotic arms.

In some embodiments, the memory further stores instructions that, when executed by the one or more processors, cause the one or more processors to: in accordance with a determination that the robotic medical system is executing a specific step of the workflow, generate a graphic and display the graphic as an overlay on the 3-D rendering.

In accordance with some embodiments of the present disclosure, a robotic medical system includes one or more robotic arms. The robotic medical system includes one or more displays. The robotic medical system also includes one or more processors and memory. The memory stores instructions that, when executed by the one or more processors, cause the one or more processors to display a three-dimensional (3-D) rendering that includes the one or more robotic arms and determine whether a manual action by a user has been completed. The memory also includes instructions that, when executed by the one or more processors, cause the one or more processors to, in accordance with a determination that the manual action is completed, update the 3-D rendering.

In some embodiments, the manual action corresponds to a step of a workflow for a medical procedure.

In some embodiments, the workflow includes one or more stages, including: a pre-operative stage, an intra-operative stage, and/or a post-operative stage.

In some embodiments, the manual action comprises docking each of the robotic arms to a respective cannula corresponding to the robotic arm.

In some embodiments, the manual action comprises attaching at least one of the robotic arms with a first medical tool.

In some embodiments, the manual action comprises attaching each of the robotic arms with a respective medical tool.

In some embodiments, the 3-D rendering includes one or more visual indicators. Each of the visual indicators corresponds to a respective one of the robotic arms. The memory further stores instructions that, when executed by the one or more processors, cause the one or more processors to: in accordance with a determination that the manual action is completed, update each of the visual indicators to indicate the completion of the manual action.

In some embodiments, the manual action includes attaching each of the robotic arms with a respective medical tool. The memory further stores instructions that, when executed by the one or more processors, cause the one or more processors to detect removal of a medical tool from a first robotic arm of the robotic arms, and in accordance with the detection, update a first visual indicator, corresponding to the first robotic arm, to indicate that the medical tool has been removed from the first robotic arm.

In some embodiments, the manual action includes manual movement of a portion of a first robotic arm of the one or more robotic arms from a first position to a second position.

In some embodiments, updating the 3-D rendering includes updating a position of the first robotic arm in the 3-D rendering from the first position to the second position.

In some embodiments, updating the 3-D rendering includes displaying the portion of the first robotic arm with a first visual representation that is visually distinct from other portions of the first robotic arm.

In some embodiments, displaying the portion of the first robotic arm with the first visual representation includes displaying the portion with a first color that is distinct from a color corresponding to the other portions of the first robotic arm.

In some embodiments, the memory further stores instructions that, when executed by the one or more processors, cause the one or more processors to detect a change in position of the portion of the first robotic arm as the manual movement is executed, and continuously update the 3-D rendering to reflect the change in position.

In some embodiments, the memory further stores instructions that, when executed by the one or more processors, cause the one or more processors to detect a change in position of the portion of the first robotic arm as the manual movement is executed, and in accordance with the executed movement, display a progress bar for visualizing progress of the manual action.

In some embodiments, the memory further stores instructions that, when executed by the one or more processors, cause the one or more processors to generate and output an audio signal as the manual action is executed.

In some embodiments, the memory further stores instructions that, when executed by the one or more processors, cause the one or more processors to: in accordance with a determination that the manual action is not completed, display an error state and information to correct the error.

In some embodiments, the robotic medical system further includes one of more adjustable arm supports that are moveably coupled to the one or more robotic arms. The 3-D rendering includes renderings of the one or more adjustable arm supports.

In some embodiments, the manual action includes movement of the one of more adjustable arm supports relative to the one or more robotic arms. Updating the 3-D rendering includes updating positions of the one or more adjustable arm supports in the renderings relative to the one or more robotic arms.

In some embodiments, the robotic medical system further includes a patient support platform. The 3-D rendering includes a rendering of the patient support platform.

In some embodiments, the manual action includes movement of at least a portion of the patient support platform from a first position to a second position. Updating the 3-D rendering includes updating a position of at least the portion of the patient support platform in the rendering from the first position to the second position.

Note that the various embodiments described above can be combined with any other embodiments described herein. The features and advantages described in the specification are not all inclusive and, in particular, many additional features and advantages will be apparent to one of ordinary skill in the art in view of the drawings, specification, and claims. Moreover, it should be noted that the language used in the specification has been principally selected for readability and instructional purposes and may not have been selected to delineate or circumscribe the inventive subject matter.

### BRIEF DESCRIPTION OF THE DRAWINGS

The disclosed aspects will hereinafter be described in conjunction with the appended drawings, provided to illustrate and not to limit the disclosed aspects, wherein like designations denote like elements.
FIG. 1 illustrates an embodiment of a cart-based robotic system arranged for diagnostic and/or therapeutic bronchoscopy procedure(s).
FIG. 2 depicts further aspects of the robotic system of FIG. 1.
FIG. 3 illustrates an embodiment of the robotic system of FIG. 1 arranged for ureteroscopy.
FIG. 4 illustrates an embodiment of the robotic system of FIG. 1 arranged for a vascular procedure.
FIG. 5 illustrates an embodiment of a table-based robotic system arranged for a bronchoscopy procedure.
FIG. 6 provides an alternative view of the robotic system of FIG. 5.
FIG. 7 illustrates an example system configured to stow robotic arm(s).
FIG. 8 illustrates an embodiment of a table-based robotic system configured for a ureteroscopy procedure.
FIG. 9 illustrates an embodiment of a table-based robotic system configured for a laparoscopic procedure.
FIG. 10 illustrates an embodiment of the table-based robotic system of FIGS. 5-9 with pitch or tilt adjustment.
FIG. 11 provides a detailed illustration of the interface between the table and the column of the table-based robotic system of FIGS. 5-10.
FIG. 12 illustrates an alternative embodiment of a table-based robotic system.
FIG. 13 illustrates an end view of the table-based robotic system of FIG. 12.
FIG. 14 illustrates an end view of a table-based robotic system with robotic arms attached thereto.
FIG. 15 illustrates an exemplary instrument driver.
FIG. 16 illustrates an exemplary medical instrument with a paired instrument driver.
FIG. 17 illustrates an alternative design for an instrument driver and instrument where the axes of the drive units are parallel to the axis of the elongated shaft of the instrument.
FIG. 18 illustrates an instrument having an instrument-based insertion architecture.
FIG. 19 illustrates an exemplary controller.
FIG. 20 depicts a block diagram illustrating a localization system that estimates a location of one or more elements of the robotic systems of FIGS. 1-10, such as the location of the instrument of FIGS. 16-18, in accordance with an example embodiment.
FIG. 21 illustrates an exemplary robotic system according to some embodiments.
FIG. 22 illustrates another view of an exemplary robotic system according to some embodiments.
FIGS. 23A to 23C illustrate different views of an exemplary robotic arm according to some embodiments.
FIGS. 24A to 24C illustrate three-dimensional (3-D) renderings of a robotic medical system during a setup stage of a procedure, in accordance with some embodiments.
FIGS. 25A and 25B illustrate 3-D renderings of a robotic medical system during a post-operative stage of a procedure, in accordance with some embodiments.
FIGS. 26A to 26D illustrate 3-D renderings that reflect real-time status of a robotic medical system arising from manual interactions according to some embodiments.
FIGS. 27A and 27B illustrate the use of 3-D renderings to communicate an error state of the robotic medical system in accordance with some embodiments.
FIGS. 28A and 28B illustrate 3-D renderings depicting a real-time status of the robotic medical system during an intra-operative stage in accordance with some embodiments.
FIGS. 29A to 29C illustrate the use of pre-rendered images to convey a status of the robotic medical system in accordance with some embodiments.
FIGS. 30A to 30E illustrate a flowchart diagram for a method performed by one or more processors of a robotic system, in accordance with some embodiments.
FIGS. 31A to 31C illustrate a flowchart diagram for a method performed by one or more processors of a robotic system, in accordance with some embodiments.
FIG. 32 is a schematic diagram illustrating electronic components of a robotic medical system in accordance with some embodiments.

### DETAILED DESCRIPTION

### 1. Overview.

Aspects of the present disclosure may be integrated into a robotically enabled medical system capable of performing a variety of medical procedures, including both minimally invasive, such as laparoscopy, and non-invasive, such as endoscopy, procedures. Among endoscopy procedures, the system may be capable of performing bronchoscopy, ureteroscopy, gastroscopy, etc.

In addition to performing the breadth of procedures, the system may provide additional benefits, such as enhanced imaging and guidance to assist the physician. Additionally, the system may provide the physician with the ability to perform the procedure from an ergonomic position without the need for awkward arm motions and positions. Still further, the system may provide the physician with the ability to perform the procedure with improved ease of use such that one or more of the instruments of the system can be controlled by a single user.

Various embodiments will be described below in conjunction with the drawings for purposes of illustration. It should be appreciated that many other embodiments of the disclosed concepts are possible, and various advantages can be achieved with the disclosed embodiments. Headings are included herein for reference and to aid in locating various sections. These headings are not intended to limit the scope of the concepts described with respect thereto. Such concepts may have applicability throughout the entire specification.

### A. Robotic System - Cart.

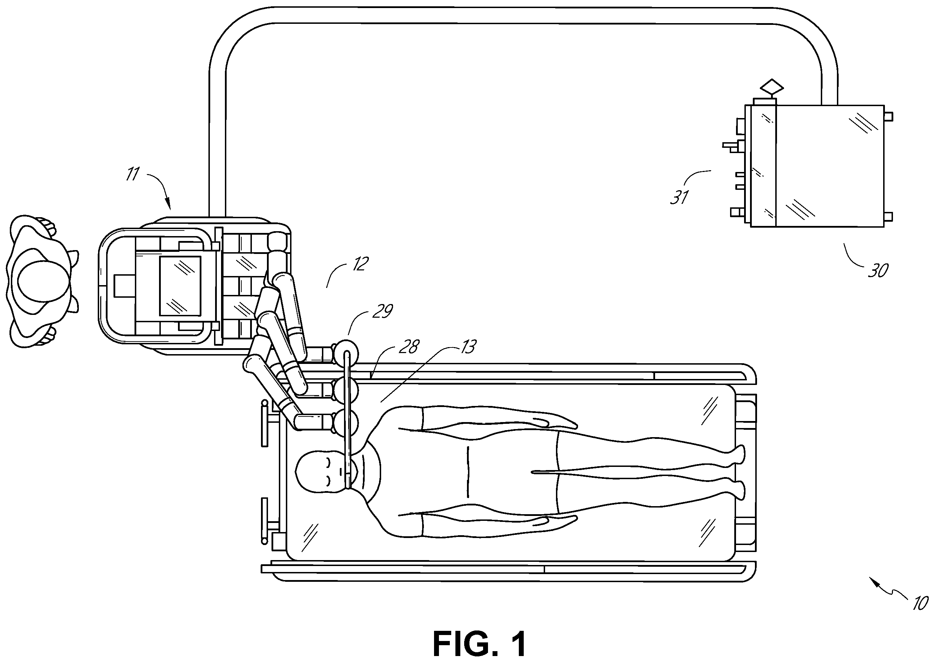

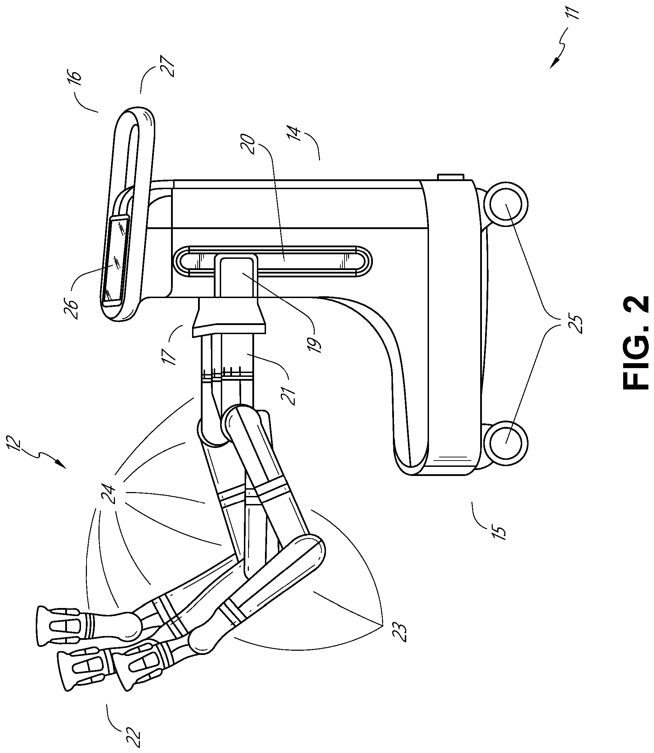

The robotically enabled medical system may be configured in a variety of ways depending on the particular procedure. FIG. 1 illustrates an embodiment of a cart-based robotically enabled system 10 arranged for a diagnostic and/or therapeutic bronchoscopy procedure. During a bronchoscopy, the system 10 may comprise a cart 11 having one or more robotic arms 12 to deliver a medical instrument, such as a steerable endoscope 13, which may be a procedure-specific bronchoscope for bronchoscopy, to a natural orifice access point (i.e., the mouth of the patient positioned on a table in the present example) to deliver diagnostic and/or therapeutic tools. As shown, the cart 11 may be positioned proximate to the patient's upper torso in order to provide access to the access point. Similarly, the robotic arms 12 may be actuated to position the bronchoscope relative to the access point. The arrangement in FIG. 1 may also be utilized when performing a gastro-intestinal (GI) procedure with a gastroscope, a specialized endoscope for GI procedures. FIG. 2 depicts an example embodiment of the cart in greater detail.

With continued reference to FIG. 1, once the cart 11 is properly positioned, the robotic arms 12 may insert the steerable endoscope 13 into the patient robotically, manually, or a combination thereof. As shown, the steerable endoscope 13 may comprise at least two telescoping parts, such as an inner leader portion and an outer sheath portion, each portion coupled to a separate instrument driver from the set of instrument drivers 28, each instrument driver coupled to the distal end of an individual robotic arm. This linear arrangement of the instrument drivers 28, which facilitates coaxially aligning the leader portion with the sheath portion, creates a "virtual rail" 29 that may be repositioned in space by manipulating the one or more robotic arms 12 into different angles and/or positions. The virtual rails described herein are depicted in the Figures using dashed lines, and accordingly the dashed lines do not depict any physical structure of the system. Translation of the instrument drivers 28 along the virtual rail 29 telescopes the inner leader portion relative to the outer sheath portion or advances or retracts the endoscope 13 from the patient. The angle of the virtual rail 29 may be adjusted, translated, and pivoted based on clinical application or physician preference. For example, in bronchoscopy, the angle and position of the virtual rail 29 as shown represents a compromise between providing physician access to the endoscope 13 while minimizing friction that results from bending the endoscope 13 into the patient's mouth.

The endoscope 13 may be directed down the patient's trachea and lungs after insertion using precise commands from the robotic system until reaching the target destination or operative site. In order to enhance navigation through the patient's lung network and/or reach the desired target, the endoscope 13 may be manipulated to telescopically extend the inner leader portion from the outer sheath portion to obtain enhanced articulation and greater bend radius. The use of separate instrument drivers 28 also allows the leader portion and sheath portion to be driven independent of each other.

For example, the endoscope 13 may be directed to deliver a biopsy needle to a target, such as, for example, a lesion or nodule within the lungs of a patient. The needle may be deployed down a working channel that runs the length of the endoscope to obtain a tissue sample to be analyzed by a pathologist. Depending on the pathology results, additional tools may be deployed down the working channel of the endoscope for additional biopsies. After identifying a nodule to be malignant, the endoscope 13 may endoscopically deliver tools to resect the potentially cancerous tissue. In some instances, diagnostic and therapeutic treatments can be delivered in separate procedures. In those circumstances, the endoscope 13 may also be used to deliver a fiducial to "mark" the location of the target nodule as well. In other instances, diagnostic and therapeutic treatments may be delivered during the same procedure.

The system 10 may also include a movable tower 30, which may be connected via support cables to the cart 11 to provide support for controls, electronics, fluidics, optics, sensors, and/or power to the cart 11. Placing such functionality in the tower 30 allows for a smaller form factor cart 11 that may be more easily adjusted and/or re-positioned by an operating physician and his/her staff. Additionally, the division of functionality between the cart / table and the support tower 30 reduces operating room clutter and facilitates improving clinical workflow. While the cart 11 may be positioned close to the patient, the tower 30 may be stowed in a remote location to stay out of the way during a procedure.

In support of the robotic systems described above, the tower 30 may include component(s) of a computer-based control system that stores computer program instructions, for example, within a non-transitory computer-readable storage medium such as a persistent magnetic storage drive, solid state drive, etc. The execution of those instructions, whether the execution occurs in the tower 30 or the cart 11, may control the entire system or sub-system(s) thereof. For example, when executed by a processor of the computer system, the instructions may cause the components of the robotics system to actuate the relevant carriages and arm mounts, actuate the robotics arms, and control the medical instruments. For example, in response to receiving the control signal, the motors in the joints of the robotics arms may position the arms into a certain posture.

The tower 30 may also include a pump, flow meter, valve control, and/or fluid access in order to provide controlled irrigation and aspiration capabilities to the system that may be deployed through the endoscope 13. These components may also be controlled using the computer system of tower 30. In some embodiments, irrigation and aspiration capabilities may be delivered directly to the endoscope 13 through separate cable(s).

The tower 30 may include a voltage and surge protector designed to provide filtered and protected electrical power to the cart 11, thereby avoiding placement of a power transformer and other auxiliary power components in the cart 11, resulting in a smaller, more moveable cart 11.

The tower 30 may also include support equipment for the sensors deployed throughout the robotic system 10. For example, the tower 30 may include opto-electronics equipment for detecting, receiving, and processing data received from the optical sensors or cameras throughout the robotic system 10. In combination with the control system, such opto-electronics equipment may be used to generate real-time images for display in any number of consoles deployed throughout the system, including in the tower 30. Similarly, the tower 30 may also include an electronic subsystem for receiving and processing signals received from deployed electromagnetic (EM) sensors. The tower 30 may also be used to house and position an EM field generator for detection by EM sensors in or on the medical instrument.

The tower 30 may also include a console 31 in addition to other consoles available in the rest of the system, e.g., console mounted on top of the cart. The console 31 may include a user interface and a display screen, such as a touchscreen, for the physician operator. Consoles in system 10 are generally designed to provide both robotic controls as well as pre-operative and real-time information of the procedure, such as navigational and localization information of the endoscope 13. When the console 31 is not the only console available to the physician, it may be used by a second operator, such as a nurse, to monitor the health or vitals of the patient and the operation of system, as well as provide procedure-specific data, such as navigational and localization information. In other embodiments, the console 30 is housed in a body that is separate from the tower 30.

The tower 30 may be coupled to the cart 11 and endoscope 13 through one or more cables or connections (not shown). In some embodiments, the support functionality from the tower 30 may be provided through a single cable to the cart 11, simplifying and de-cluttering the operating room. In other embodiments, specific functionality may be coupled in separate cabling and connections. For example, while power may be provided through a single power cable to the cart, the support for controls, optics, fluidics, and/or navigation may be provided through a separate cable.

FIG. 2 provides a detailed illustration of an embodiment of the cart from the cart-based robotically enabled system shown in FIG. 1. The cart 11 generally includes an elongated support structure 14 (often referred to as a "column"), a cart base 15, and a console 16 at the top of the column 14. The column 14 may include one or more carriages, such as a carriage 17 (alternatively "arm support") for supporting the deployment of one or more robotic arms 12 (three shown in FIG. 2). The carriage 17 may include individually configurable arm mounts that rotate along a perpendicular axis to adjust the base of the robotic arms 12 for better positioning relative to the patient. The carriage 17 also includes a carriage interface 19 that allows the carriage 17 to vertically translate along the column 14.

The carriage interface 19 is connected to the column 14 through slots, such as slot 20, that are positioned on opposite sides of the column 14 to guide the vertical translation of the carriage 17. The slot 20 contains a vertical translation interface to position and hold the carriage at various vertical heights relative to the cart base 15. Vertical translation of the carriage 17 allows the cart 11 to adjust the reach of the robotic arms 12 to meet a variety of table heights, patient sizes, and physician preferences. Similarly, the individually configurable arm mounts on the carriage 17 allow the robotic arm base 21 of robotic arms 12 to be angled in a variety of configurations.

In some embodiments, the slot 20 may be supplemented with slot covers that are flush and parallel to the slot surface to prevent dirt and fluid ingress into the internal chambers of the column 14 and the vertical translation interface as the carriage 17 vertically translates. The slot covers may be deployed through pairs of spring spools positioned near the vertical top and bottom of the slot 20. The covers are coiled within the spools until deployed to extend and retract from their coiled state as the carriage 17 vertically translates up and down. The spring-loading of the spools provides force to retract the cover into a spool when carriage 17 translates towards the spool, while also maintaining a tight seal when the carriage 17 translates away from the spool. The covers may be connected to the carriage 17 using, for example, brackets in the carriage interface 19 to ensure proper extension and retraction of the cover as the carriage 17 translates.

The column 14 may internally comprise mechanisms, such as gears and motors, that are designed to use a vertically aligned lead screw to translate the carriage 17 in a mechanized fashion in response to control signals generated in response to user inputs, e.g., inputs from the console 16.

The robotic arms 12 may generally comprise robotic arm bases 21 and end effectors 22, separated by a series of linkages 23 that are connected by a series of joints 24, each joint comprising an independent actuator, each actuator comprising an independently controllable motor. Each independently controllable joint represents an independent degree of freedom available to the robotic arm. Each of the arms 12 have seven joints, and thus provide seven degrees of freedom. A multitude of joints result in a multitude of degrees of freedom, allowing for "redundant" degrees of freedom. Redundant degrees of freedom allow the robotic arms 12 to position their respective end effectors 22 at a specific position, orientation, and trajectory in space using different linkage positions and joint angles. This allows for the system to position and direct a medical instrument from a desired point in space while allowing the physician to move the arm joints into a clinically advantageous position away from the patient to create greater access, while avoiding arm collisions.

The cart base 15 balances the weight of the column 14, carriage 17, and arms 12 over the floor. Accordingly, the cart base 15 houses heavier components, such as electronics, motors, power supply, as well as components that either enable movement and/or immobilize the cart. For example, the cart base 15 includes rollable wheel-shaped casters 25 that allow for the cart to easily move around the room prior to a procedure. After reaching the appropriate position, the casters 25 may be immobilized using wheel locks to hold the cart 11 in place during the procedure.

Positioned at the vertical end of column 14, the console 16 allows for both a user interface for receiving user input and a display screen (or a dual-purpose device such as, for example, a touchscreen 26) to provide the physician user with both pre-operative and intra-operative data. Potential pre-operative data on the touchscreen 26 may include pre-operative plans, navigation and mapping data derived from pre-operative computerized tomography (CT) scans, and/or notes from pre-operative patient interviews. Intra-operative data on display may include optical information provided from the tool, sensor and coordinate information from sensors, as well as vital patient statistics, such as respiration, heart rate, and/or pulse. The console 16 may be positioned and tilted to allow a physician to access the console from the side of the column 14 opposite carriage 17. From this position, the physician may view the console 16, robotic arms 12, and patient while operating the console 16 from behind the cart 11. As shown, the console 16 also includes a handle 27 to assist with maneuvering and stabilizing cart 11.

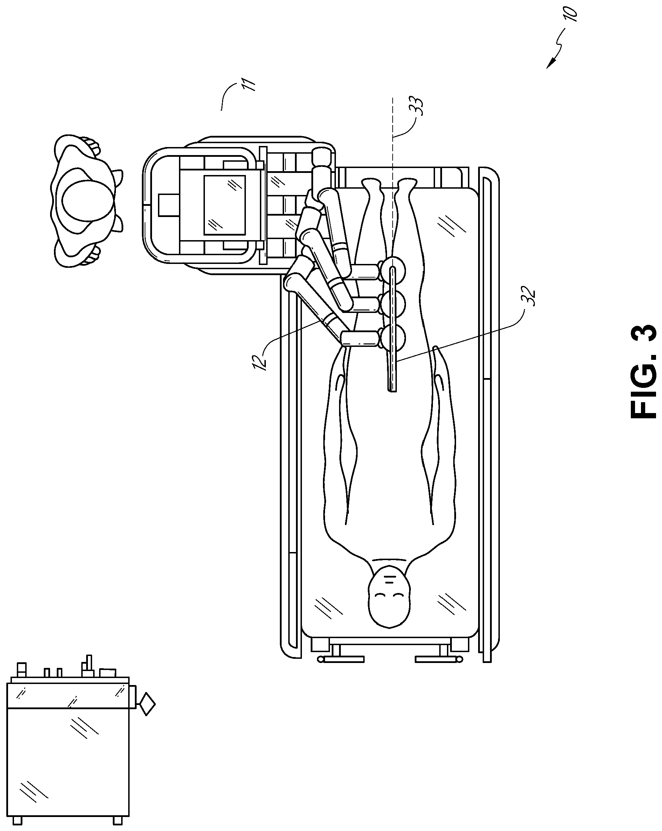

FIG. 3 illustrates an embodiment of a robotically enabled system 10 arranged for ureteroscopy. In a ureteroscopic procedure, the cart 11 may be positioned to deliver a ureteroscope 32, a procedure-specific endoscope designed to traverse a patient's urethra and ureter, to the lower abdominal area of the patient. In a ureteroscopy, it may be desirable for the ureteroscope 32 to be directly aligned with the patient's urethra to reduce friction and forces on the sensitive anatomy in the area. As shown, the cart 11 may be aligned at the foot of the table to allow the robotic arms 12 to position the ureteroscope 32 for direct linear access to the patient's urethra. From the foot of the table, the robotic arms 12 may insert the ureteroscope 32 along the virtual rail 33 directly into the patient's lower abdomen through the urethra.

After insertion into the urethra, using similar control techniques as in bronchoscopy, the ureteroscope 32 may be navigated into the bladder, ureters, and/or kidneys for diagnostic and/or therapeutic applications. For example, the ureteroscope 32 may be directed into the ureter and kidneys to break up kidney stone build up using a laser or ultrasonic lithotripsy device deployed down the working channel of the ureteroscope 32. After lithotripsy is complete, the resulting stone fragments may be removed using baskets deployed down the ureteroscope 32.

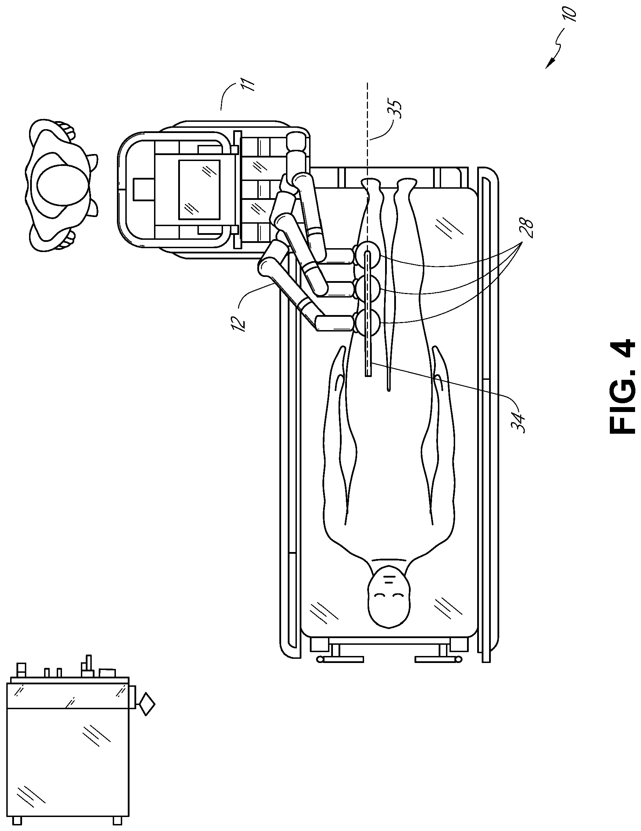

FIG. 4 illustrates an embodiment of a robotically enabled system similarly arranged for a vascular procedure. In a vascular procedure, the system 10 may be configured such that the cart 11 may deliver a medical instrument 34, such as a steerable catheter, to an access point in the femoral artery in the patient's leg. The femoral artery presents both a larger diameter for navigation as well as a relatively less circuitous and tortuous path to the patient's heart, which simplifies navigation. As in a ureteroscopic procedure, the cart 11 may be positioned towards the patient's legs and lower abdomen to allow the robotic arms 12 to provide a virtual rail 35 with direct linear access to the femoral artery access point in the patient's thigh / hip region. After insertion into the artery, the medical instrument 34 may be directed and inserted by translating the instrument drivers 28. Alternatively, the cart may be positioned around the patient's upper abdomen in order to reach alternative vascular access points, such as, for example, the carotid and brachial arteries near the shoulder and wrist.

### B. Robotic System - Table.

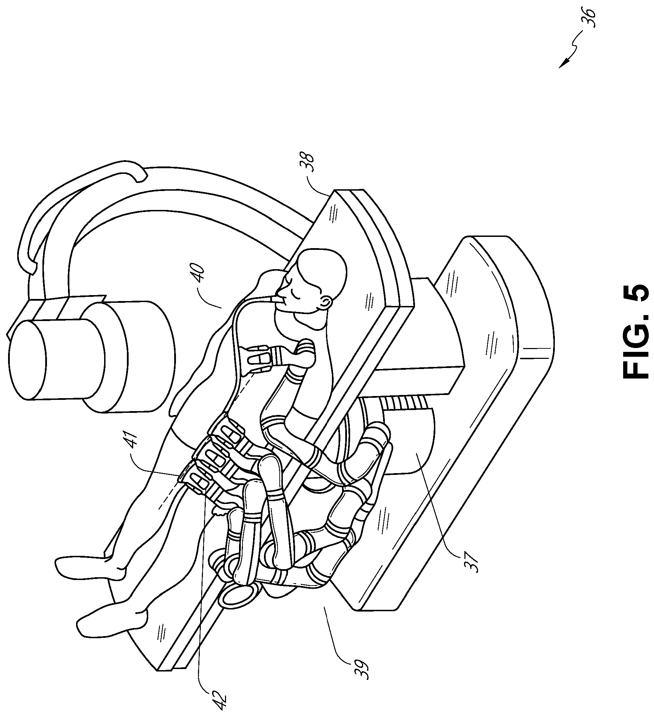

Embodiments of the robotically enabled medical system may also incorporate the patient's table. Incorporation of the table reduces the amount of capital equipment within the operating room by removing the cart, which allows greater access to the patient. FIG. 5 illustrates an embodiment of such a robotically enabled system arranged for a bronchoscopy procedure. System 36 includes a support structure or column 37 for supporting platform 38 (shown as a "table" or "bed") over the floor. Much like in the cart-based systems, the end effectors of the robotic arms 39 of the system 36 comprise instrument drivers 42 that are designed to manipulate an elongated medical instrument, such as a bronchoscope 40 in FIG. 5, through or along a virtual rail 41 formed from the linear alignment of the instrument drivers 42. In practice, a C-arm for providing fluoroscopic imaging may be positioned over the patient's upper abdominal area by placing the emitter and detector around table 38.

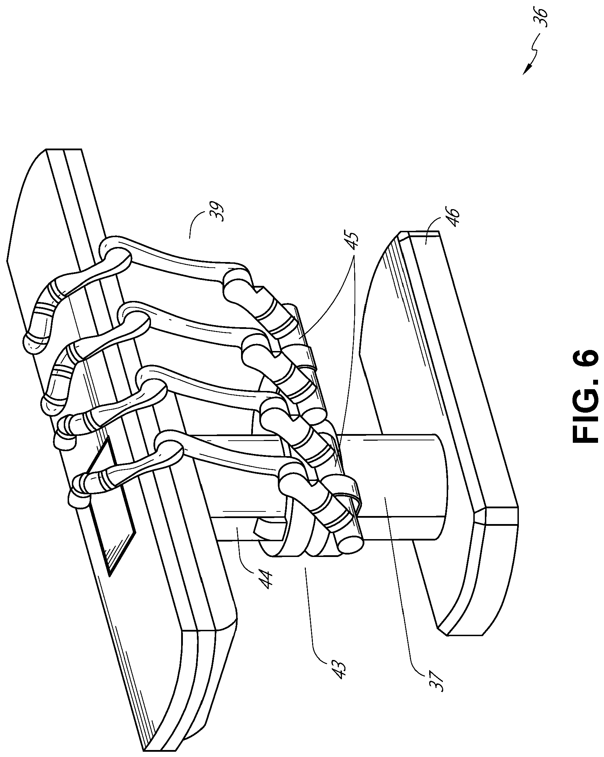

FIG. 6 provides an alternative view of the system 36 without the patient and medical instrument for discussion purposes. As shown, the column 37 may include one or more carriages 43 shown as ring-shaped in the system 36, from which the one or more robotic arms 39 may be based. The carriages 43 may translate along a vertical column interface 44 that runs the length of the column 37 to provide different vantage points from which the robotic arms 39 may be positioned to reach the patient. The carriage(s) 43 may rotate around the column 37 using a mechanical motor positioned within the column 37 to allow the robotic arms 39 to have access to multiples sides of the table 38, such as, for example, both sides of the patient. In embodiments with multiple carriages, the carriages may be individually positioned on the column and may translate and/or rotate independent of the other carriages. While carriages 43 need not surround the column 37 or even be circular, the ring-shape as shown facilitates rotation of the carriages 43 around the column 37 while maintaining structural balance. Rotation and translation of the carriages 43 allows the system to align the medical instruments, such as endoscopes and laparoscopes, into different access points on the patient. In other embodiments (not shown), the system 36 can include a patient table or bed with adjustable arm supports in the form of bars or rails extending alongside it. One or more robotic arms 39 (e.g., via a shoulder with an elbow joint) can be attached to the adjustable arm supports, which can be vertically adjusted. By providing vertical adjustment, the robotic arms 39 are advantageously capable of being stowed compactly beneath the patient table or bed, and subsequently raised during a procedure.

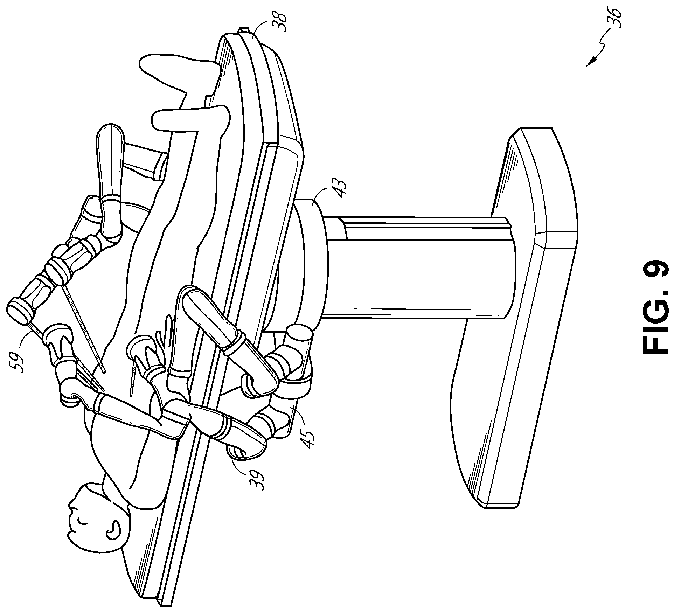

The arms 39 may be mounted on the carriages through a set of arm mounts 45 comprising a series of joints that may individually rotate and/or telescopically extend to provide additional configurability to the robotic arms 39. Additionally, the arm mounts 45 may be positioned on the carriages 43 such that, when the carriages 43 are appropriately rotated, the arm mounts 45 may be positioned on either the same side of table 38 (as shown in FIG. 6), on opposite sides of table 38 (as shown in FIG. 9), or on adjacent sides of the table 38 (not shown).

The column 37 structurally provides support for the table 38, and a path for vertical translation of the carriages. Internally, the column 37 may be equipped with lead screws for guiding vertical translation of the carriages, and motors to mechanize the translation of said carriages based the lead screws. The column 37 may also convey power and control signals to the carriage 43 and robotic arms 39 mounted thereon.

The table base 46 serves a similar function as the cart base 15 in cart 11 shown in FIG. 2, housing heavier components to balance the table/bed 38, the column 37, the carriages 43, and the robotic arms 39. The table base 46 may also incorporate rigid casters to provide stability during procedures. Deployed from the bottom of the table base 46, the casters may extend in opposite directions on both sides of the base 46 and retract when the system 36 needs to be moved.

Continuing with FIG. 6, the system 36 may also include a tower (not shown) that divides the functionality of system 36 between table and tower to reduce the form factor and bulk of the table. As in earlier disclosed embodiments, the tower may provide a variety of support functionalities to table, such as processing, computing, and control capabilities, power, fluidics, and/or optical and sensor processing. The tower may also be movable to be positioned away from the patient to improve physician access and de-clutter the operating room. Additionally, placing components in the tower allows for more storage space in the table base for potential stowage of the robotic arms. The tower may also include a master controller or console that provides both a user interface for user input, such as keyboard and/or pendant, as well as a display screen (or touchscreen) for pre-operative and intra-operative information, such as real-time imaging, navigation, and tracking information. In some embodiments, the tower may also contain holders for gas tanks to be used for insufflation.

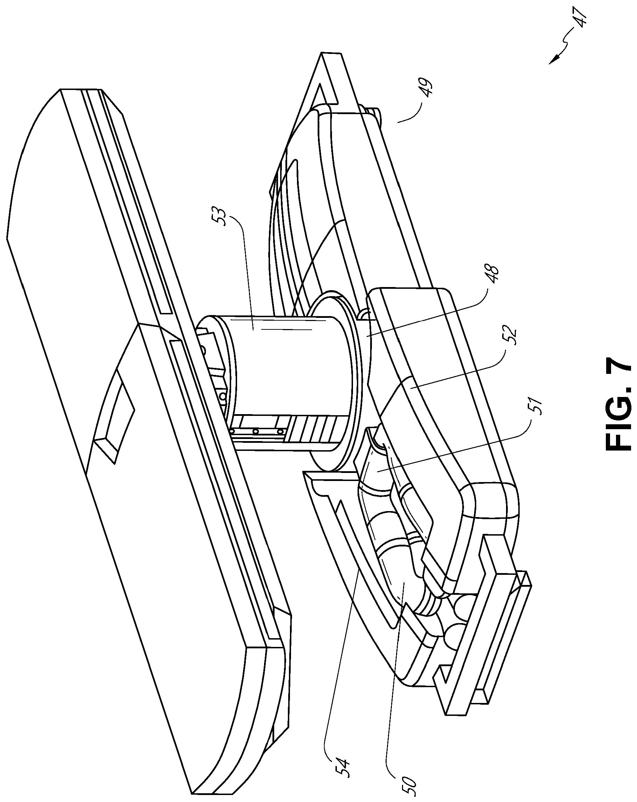

In some embodiments, a table base may stow and store the robotic arms when not in use. FIG. 7 illustrates a system 47 that stows robotic arms in an embodiment of the table-based system. In system 47, carriages 48 may be vertically translated into base 49 to stow robotic arms 50, arm mounts 51, and the carriages 48 within the base 49. Base covers 52 may be translated and retracted open to deploy the carriages 48, arm mounts 51, and arms 50 around column 53, and closed to stow to protect them when not in use. The base covers 52 may be sealed with a membrane 54 along the edges of its opening to prevent dirt and fluid ingress when closed.

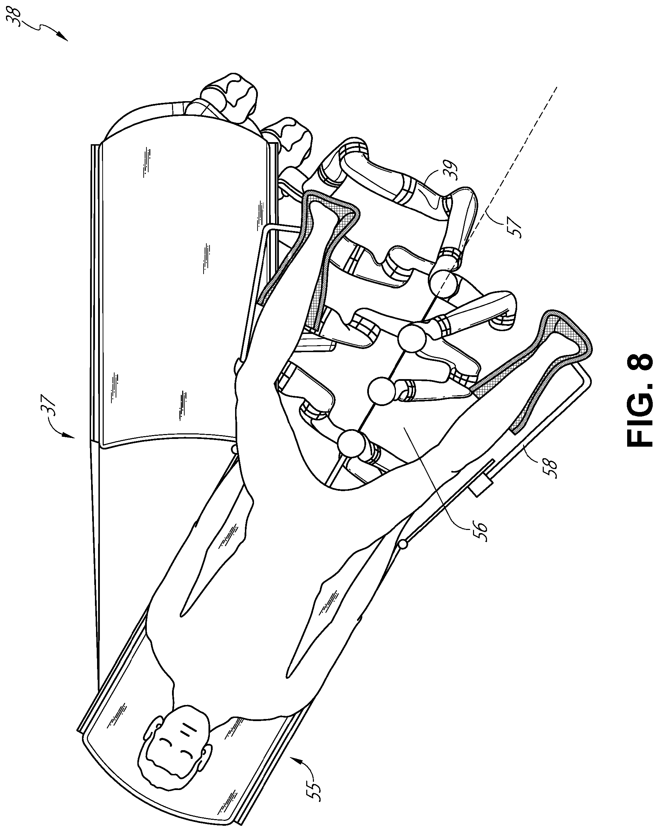

FIG. 8 illustrates an embodiment of a robotically enabled table-based system configured for a ureteroscopy procedure. In a ureteroscopy, the table 38 may include a swivel portion 55 for positioning a patient off-angle from the column 37 and table base 46. The swivel portion 55 may rotate or pivot around a pivot point (e.g., located below the patient's head) in order to position the bottom portion of the swivel portion 55 away from the column 37. For example, the pivoting of the swivel portion 55 allows a C-arm (not shown) to be positioned over the patient's lower abdomen without competing for space with the column (not shown) below table 38. By rotating the carriage 35 (not shown) around the column 37, the robotic arms 39 may directly insert a ureteroscope 56 along a virtual rail 57 into the patient's groin area to reach the urethra. In a ureteroscopy, stirrups 58 may also be fixed to the swivel portion 55 of the table 38 to support the position of the patient's legs during the procedure and allow clear access to the patient's groin area.

In a laparoscopic procedure, through small incision(s) in the patient's abdominal wall, minimally invasive instruments may be inserted into the patient's anatomy. In some embodiments, the minimally invasive instruments comprise an elongated rigid member, such as a shaft, which is used to access anatomy within the patient. After inflation of the patient's abdominal cavity, the instruments may be directed to perform surgical or medical tasks, such as grasping, cutting, ablating, suturing, etc. In some embodiments, the instruments can comprise a scope, such as a laparoscope. FIG. 9 illustrates an embodiment of a robotically enabled table-based system configured for a laparoscopic procedure. As shown in FIG. 9, the carriages 43 of the system 36 may be rotated and vertically adjusted to position pairs of the robotic arms 39 on opposite sides of the table 38, such that instrument 59 may be positioned using the arm mounts 45 to be passed through minimal incisions on both sides of the patient to reach his/her abdominal cavity.

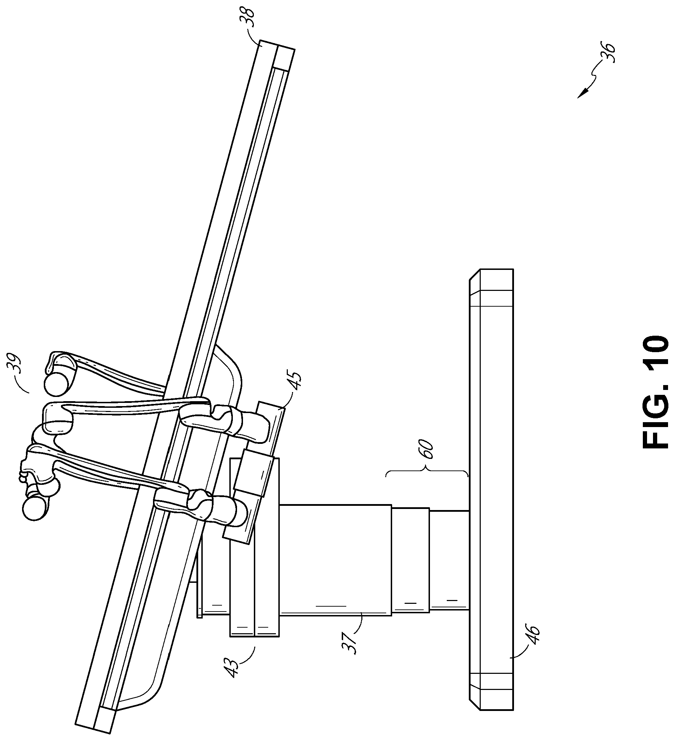

To accommodate laparoscopic procedures, the robotically enabled table system may also tilt the platform to a desired angle. FIG. 10 illustrates an embodiment of the robotically enabled medical system with pitch or tilt adjustment. As shown in FIG. 10, the system 36 may accommodate tilt of the table 38 to position one portion of the table at a greater distance from the floor than the other. Additionally, the arm mounts 45 may rotate to match the tilt such that the arms 39 maintain the same planar relationship with table 38. To accommodate steeper angles, the column 37 may also include telescoping portions 60 that allow vertical extension of column 37 to keep the table 38 from touching the floor or colliding with base 46.

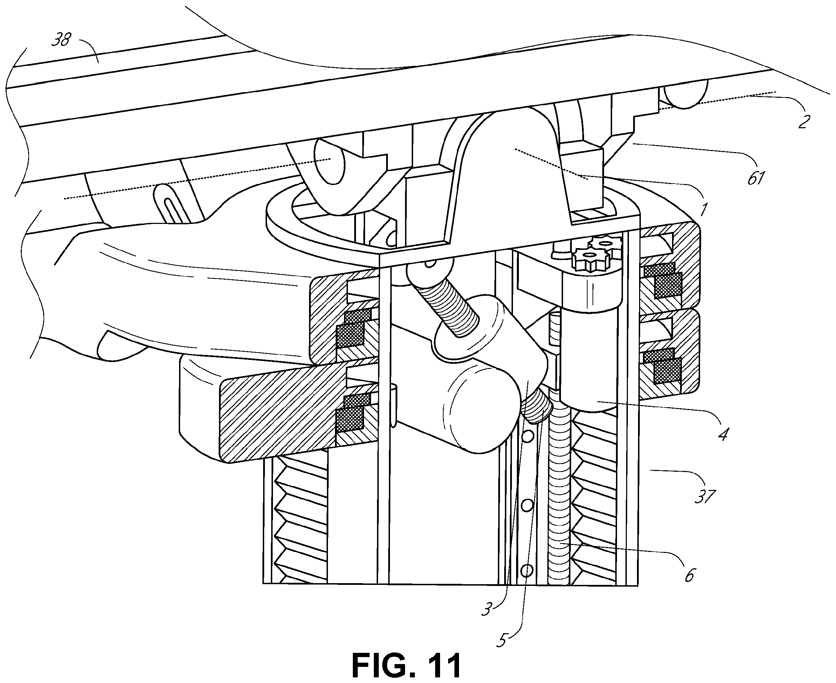

FIG. 11 provides a detailed illustration of the interface between the table 38 and the column 37. Pitch rotation mechanism 61 may be configured to alter the pitch angle of the table 38 relative to the column 37 in multiple degrees of freedom. The pitch rotation mechanism 61 may be enabled by the positioning of orthogonal axes 1, 2 at the column-table interface, each axis actuated by a separate motor 3, 4 responsive to an electrical pitch angle command. Rotation along one screw 5 would enable tilt adjustments in one axis 1, while rotation along the other screw 6 would enable tilt adjustments along the other axis 2. In some embodiments, a ball joint can be used to alter the pitch angle of the table 38 relative to the column 37 in multiple degrees of freedom.

For example, pitch adjustments are particularly useful when trying to position the table in a Trendelenburg position, i.e., position the patient's lower abdomen at a higher position from the floor than the patient's lower abdomen, for lower abdominal surgery. The Trendelenburg position causes the patient's internal organs to slide towards his/her upper abdomen through the force of gravity, clearing out the abdominal cavity for minimally invasive tools to enter and perform lower abdominal surgical or medical procedures, such as laparoscopic prostatectomy.

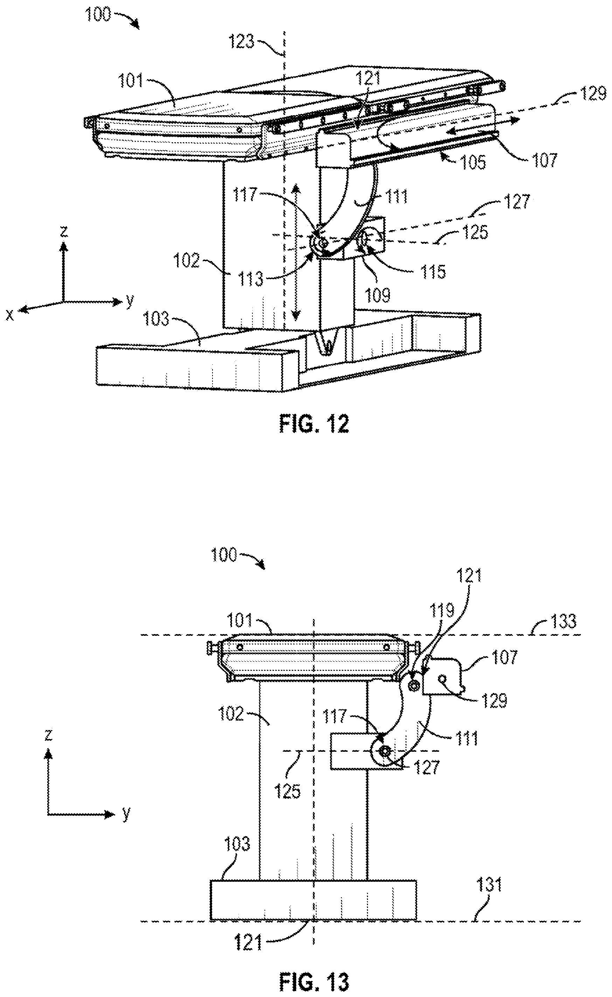

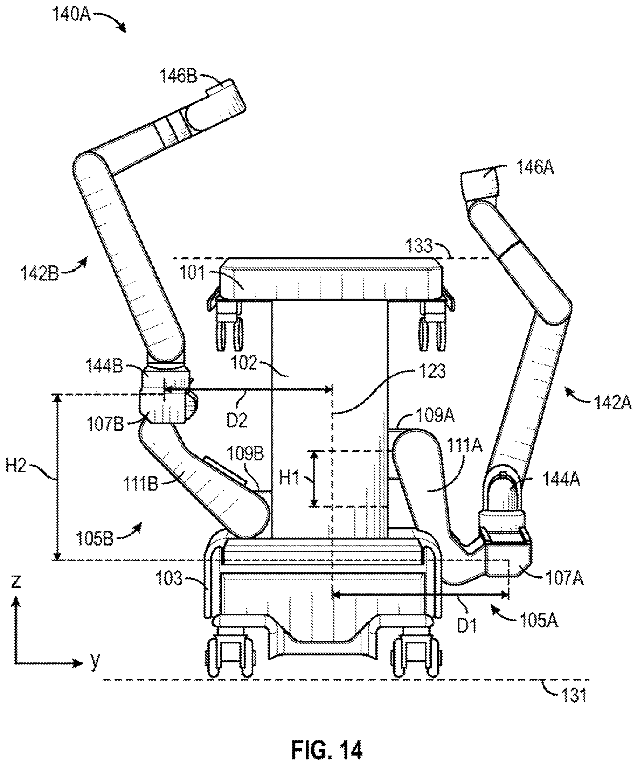

FIGS. 12 and 13 illustrate isometric and end views of an alternative embodiment of a table-based surgical robotics system 100. The surgical robotics system 100 includes one or more adjustable arm supports 105 that can be configured to support one or more robotic arms (see, for example, FIG. 14) relative to a table 101. In the illustrated embodiment, a single adjustable arm support 105 is shown, though an additional arm support can be provided on an opposite side of the table 101. The adjustable arm support 105 can be configured so that it can move relative to the table 101 to adjust and/or vary the position of the adjustable arm support 105 and/or any robotic arms mounted thereto relative to the table 101. For example, the adjustable arm support 105 may be adjusted one or more degrees of freedom relative to the table 101. The adjustable arm support 105 provides high versatility to the system 100, including the ability to easily stow the one or more adjustable arm supports 105 and any robotics arms attached thereto beneath the table 101. The adjustable arm support 105 can be elevated from the stowed position to a position below an upper surface of the table 101. In other embodiments, the adjustable arm support 105 can be elevated from the stowed position to a position above an upper surface of the table 101.

The adjustable arm support 105 can provide several degrees of freedom, including lift, lateral translation, tilt, etc. In the illustrated embodiment of FIGS. 12 and 13, the arm support 105 is configured with four degrees of freedom, which are illustrated with arrows in FIG. 12. A first degree of freedom allows for adjustment of the adjustable arm support 105 in the z-direction ("Z-lift"). For example, the adjustable arm support 105 can include a carriage 109 configured to move up or down along or relative to a column 102 supporting the table 101. A second degree of freedom can allow the adjustable arm support 105 to tilt. For example, the adjustable arm support 105 can include a rotary joint, which can allow the adjustable arm support 105 to be aligned with the bed in a Trendelenburg position. A third degree of freedom can allow the adjustable arm support 105 to "pivot up," which can be used to adjust a distance between a side of the table 101 and the adjustable arm support 105. A fourth degree of freedom can permit translation of the adjustable arm support 105 along a longitudinal length of the table.

The surgical robotics system 100 in FIGS. 12 and 13 can comprise a table supported by a column 102 that is mounted to a base 103. The base 103 and the column 102 support the table 101 relative to a support surface. A floor axis 131 and a support axis 133 are shown in FIG. 13.

The adjustable arm support 105 can be mounted to the column 102. In other embodiments, the arm support 105 can be mounted to the table 101 or base 103. The adjustable arm support 105 can include a carriage 109, a bar or rail connector 111 and a bar or rail 107. In some embodiments, one or more robotic arms mounted to the rail 107 can translate and move relative to one another.

The carriage 109 can be attached to the column 102 by a first joint 113, which allows the carriage 109 to move relative to the column 102 (e.g., such as up and down a first or vertical axis 123). The first joint 113 can provide the first degree of freedom ("Z-lift") to the adjustable arm support 105. The adjustable arm support 105 can include a second joint 115, which provides the second degree of freedom (tilt) for the adjustable arm support 105. The adjustable arm support 105 can include a third joint 117, which can provide the third degree of freedom ("pivot up") for the adjustable arm support 105. An additional joint 119 (shown in FIG. 13) can be provided that mechanically constrains the third joint 117 to maintain an orientation of the rail 107 as the rail connector 111 is rotated about a third axis 127. The adjustable arm support 105 can include a fourth joint 121, which can provide a fourth degree of freedom (translation) for the adjustable arm support 105 along a fourth axis 129.

FIG. 14 illustrates an end view of the surgical robotics system 140A with two adjustable arm supports 105A, 105B mounted on opposite sides of a table 101. A first robotic arm 142A is attached to the bar or rail 107A of the first adjustable arm support 105B. The first robotic arm 142A includes a base 144A attached to the rail 107A. The distal end of the first robotic arm 142A includes an instrument drive mechanism 146A that can attach to one or more robotic medical instruments or tools. Similarly, the second robotic arm 142B includes a base 144B attached to the rail 107B. The distal end of the second robotic arm 142B includes an instrument drive mechanism 146B. The instrument drive mechanism 146B can be configured to attach to one or more robotic medical instruments or tools.

In some embodiments, one or more of the robotic arms 142A, 142B comprises an arm with seven or more degrees of freedom. In some embodiments, one or more of the robotic arms 142A, 142B can include eight degrees of freedom, including an insertion axis (1-degree of freedom including insertion), a wrist (3-degrees of freedom including wrist pitch, yaw and roll), an elbow (1-degree of freedom including elbow pitch), a shoulder (2-degrees of freedom including shoulder pitch and yaw), and base 144A, 144B (1-degree of freedom including translation). In some embodiments, the insertion degree of freedom can be provided by the robotic arm 142A, 142B, while in other embodiments, the instrument itself provides insertion via an instrument-based insertion architecture.

### C. Instrument Driver & Interface.

The end effectors of the system's robotic arms comprise (i) an instrument driver (alternatively referred to as "instrument drive mechanism" or "instrument device manipulator") that incorporate electro-mechanical means for actuating the medical instrument and (ii) a removable or detachable medical instrument, which may be devoid of any electro-mechanical components, such as motors. This dichotomy may be driven by the need to sterilize medical instruments used in medical procedures, and the inability to adequately sterilize expensive capital equipment due to their intricate mechanical assemblies and sensitive electronics. Accordingly, the medical instruments may be designed to be detached, removed, and interchanged from the instrument driver (and thus the system) for individual sterilization or disposal by the physician or the physician's staff. In contrast, the instrument drivers need not be changed or sterilized, and may be draped for protection.

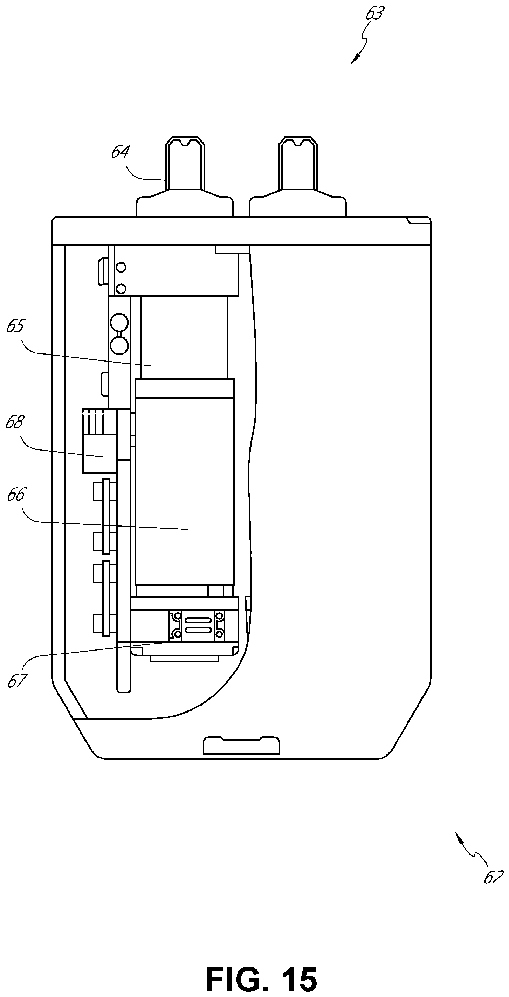

FIG. 15 illustrates an example instrument driver. Positioned at the distal end of a robotic arm, instrument driver 62 comprises of one or more drive units 63 arranged with parallel axes to provide controlled torque to a medical instrument via drive shafts 64. Each drive unit 63 comprises an individual drive shaft 64 for interacting with the instrument, a gear head 65 for converting the motor shaft rotation to a desired torque, a motor 66 for generating the drive torque, an encoder 67 to measure the speed of the motor shaft and provide feedback to the control circuitry, and control circuity 68 for receiving control signals and actuating the drive unit. Each drive unit 63 being independent controlled and motorized, the instrument driver 62 may provide multiple (four as shown in FIG. 15) independent drive outputs to the medical instrument. In operation, the control circuitry 68 would receive a control signal, transmit a motor signal to the motor 66, compare the resulting motor speed as measured by the encoder 67 with the desired speed, and modulate the motor signal to generate the desired torque.

For procedures that require a sterile environment, the robotic system may incorporate a drive interface, such as a sterile adapter connected to a sterile drape, that sits between the instrument driver and the medical instrument. The chief purpose of the sterile adapter is to transfer angular motion from the drive shafts of the instrument driver to the drive inputs of the instrument while maintaining physical separation, and thus sterility, between the drive shafts and drive inputs. Accordingly, an example sterile adapter may comprise of a series of rotational inputs and outputs intended to be mated with the drive shafts of the instrument driver and drive inputs on the instrument. Connected to the sterile adapter, the sterile drape, comprised of a thin, flexible material such as transparent or translucent plastic, is designed to cover the capital equipment, such as the instrument driver, robotic arm, and cart (in a cart-based system) or table (in a table-based system). Use of the drape would allow the capital equipment to be positioned proximate to the patient while still being located in an area not requiring sterilization (i.e., non-sterile field). On the other side of the sterile drape, the medical instrument may interface with the patient in an area requiring sterilization (i.e., sterile field).

### D. Medical Instrument.

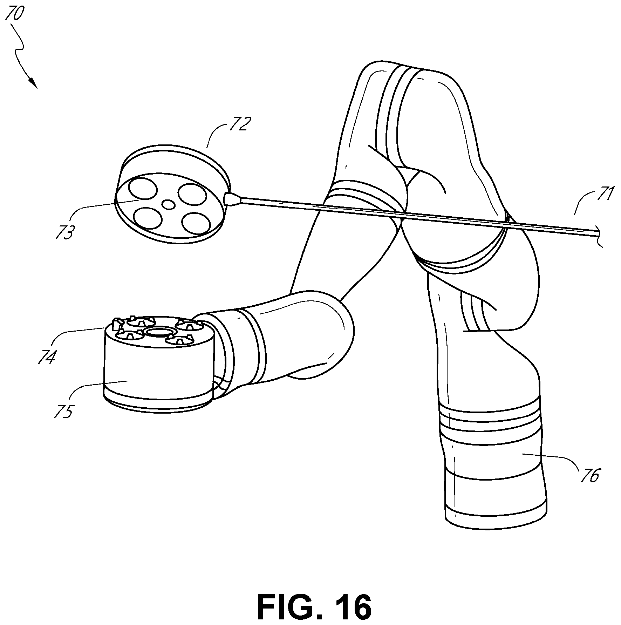

FIG. 16 illustrates an example medical instrument with a paired instrument driver. Like other instruments designed for use with a robotic system, medical instrument 70 comprises an elongated shaft 71 (or elongate body) and an instrument base 72. The instrument base 72, also referred to as an "instrument handle" due to its intended design for manual interaction by the physician, may generally comprise rotatable drive inputs 73, e.g., receptacles, pulleys or spools, that are designed to be mated with drive outputs 74 that extend through a drive interface on instrument driver 75 at the distal end of robotic arm 76. When physically connected, latched, and/or coupled, the mated drive inputs 73 of instrument base 72 may share axes of rotation with the drive outputs 74 in the instrument driver 75 to allow the transfer of torque from drive outputs 74 to drive inputs 73. In some embodiments, the drive outputs 74 may comprise splines that are designed to mate with receptacles on the drive inputs 73.

The elongated shaft 71 is designed to be delivered through either an anatomical opening or lumen, e.g., as in endoscopy, or a minimally invasive incision, e.g., as in laparoscopy. The elongated shaft 71 may be either flexible (e.g., having properties similar to an endoscope) or rigid (e.g., having properties similar to a laparoscope) or contain a customized combination of both flexible and rigid portions. When designed for laparoscopy, the distal end of a rigid elongated shaft may be connected to an end effector extending from a jointed wrist formed from a clevis with at least one degree of freedom and a surgical tool or medical instrument, such as, for example, a grasper or scissors, that may be actuated based on force from the tendons as the drive inputs rotate in response to torque received from the drive outputs 74 of the instrument driver 75. When designed for endoscopy, the distal end of a flexible elongated shaft may include a steerable or controllable bending section that may be articulated and bent based on torque received from the drive outputs 74 of the instrument driver 75.

Torque from the instrument driver 75 is transmitted down the elongated shaft 71 using tendons along the shaft 71. These individual tendons, such as pull wires, may be individually anchored to individual drive inputs 73 within the instrument handle 72. From the handle 72, the tendons are directed down one or more pull lumens along the elongated shaft 71 and anchored at the distal portion of the elongated shaft 71, or in the wrist at the distal portion of the elongated shaft. During a surgical procedure, such as a laparoscopic, endoscopic or hybrid procedure, these tendons may be coupled to a distally mounted end effector, such as a wrist, grasper, or scissor. Under such an arrangement, torque exerted on drive inputs 73 would transfer tension to the tendon, thereby causing the end effector to actuate in some way. In some embodiments, during a surgical procedure, the tendon may cause a joint to rotate about an axis, thereby causing the end effector to move in one direction or another. Alternatively, the tendon may be connected to one or more jaws of a grasper at distal end of the elongated shaft 71, where tension from the tendon cause the grasper to close.

In endoscopy, the tendons may be coupled to a bending or articulating section positioned along the elongated shaft 71 (e.g., at the distal end) via adhesive, control ring, or other mechanical fixation. When fixedly attached to the distal end of a bending section, torque exerted on drive inputs 73 would be transmitted down the tendons, causing the softer, bending section (sometimes referred to as the articulable section or region) to bend or articulate. Along the non-bending sections, it may be advantageous to spiral or helix the individual pull lumens that direct the individual tendons along (or inside) the walls of the endoscope shaft to balance the radial forces that result from tension in the pull wires. The angle of the spiraling and/or spacing there between may be altered or engineered for specific purposes, wherein tighter spiraling exhibits lesser shaft compression under load forces, while lower amounts of spiraling results in greater shaft compression under load forces, but also exhibits limits bending. On the other end of the spectrum, the pull lumens may be directed parallel to the longitudinal axis of the elongated shaft 71 to allow for controlled articulation in the desired bending or articulable sections.

In endoscopy, the elongated shaft 71 houses a number of components to assist with the robotic procedure. The shaft may comprise of a working channel for deploying surgical tools (or medical instruments), irrigation, and/or aspiration to the operative region at the distal end of the shaft 71. The shaft 71 may also accommodate wires and/or optical fibers to transfer signals to/from an optical assembly at the distal tip, which may include of an optical camera. The shaft 71 may also accommodate optical fibers to carry light from proximally located light sources, such as light emitting diodes, to the distal end of the shaft.

At the distal end of the instrument 70, the distal tip may also comprise the opening of a working channel for delivering tools for diagnostic and/or therapy, irrigation, and aspiration to an operative site. The distal tip may also include a port for a camera, such as a fiberscope or a digital camera, to capture images of an internal anatomical space. Relatedly, the distal tip may also include ports for light sources for illuminating the anatomical space when using the camera.

In the example of FIG. 16, the drive shaft axes, and thus the drive input axes, are orthogonal to the axis of the elongated shaft. This arrangement, however, complicates roll capabilities for the elongated shaft 71. Rolling the elongated shaft 71 along its axis while keeping the drive inputs 73 static results in undesirable tangling of the tendons as they extend off the drive inputs 73 and enter pull lumens within the elongated shaft 71. The resulting entanglement of such tendons may disrupt any control algorithms intended to predict movement of the flexible elongated shaft during an endoscopic procedure.

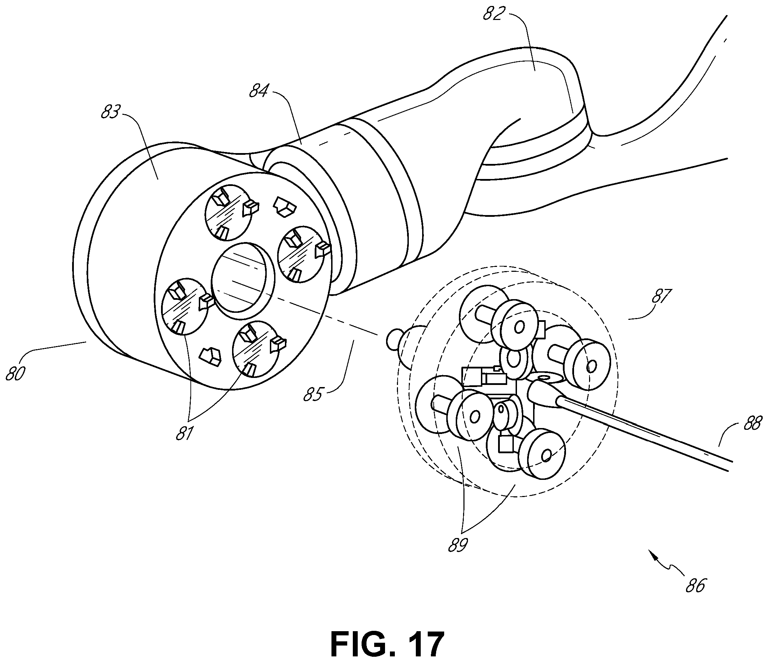

FIG. 17 illustrates an alternative design for an instrument driver and instrument where the axes of the drive units are parallel to the axis of the elongated shaft of the instrument. As shown, a circular instrument driver 80 comprises four drive units with their drive outputs 81 aligned in parallel at the end of a robotic arm 82. The drive units, and their respective drive outputs 81, are housed in a rotational assembly 83 of the instrument driver 80 that is driven by one of the drive units within the assembly 83. In response to torque provided by the rotational drive unit, the rotational assembly 83 rotates along a circular bearing that connects the rotational assembly 83 to the non-rotational portion 84 of the instrument driver. Power and controls signals may be communicated from the non-rotational portion 84 of the instrument driver 80 to the rotational assembly 83 through electrical contacts and may be maintained through rotation by a brushed slip ring connection (not shown). In other embodiments, the rotational assembly 83 may be responsive to a separate drive unit that is integrated into the non-rotatable portion 84, and thus not in parallel to the other drive units. The rotational mechanism 83 allows the instrument driver 80 to rotate the drive units, and their respective drive outputs 81, as a single unit around an instrument driver axis 85.

Like earlier disclosed embodiments, an instrument 86 may comprise an elongated shaft portion 88 and an instrument base 87 (shown with a transparent external skin for discussion purposes) comprising a plurality of drive inputs 89 (such as receptacles, pulleys, and spools) that are configured to receive the drive outputs 81 in the instrument driver 80. Unlike prior disclosed embodiments, instrument shaft 88 extends from the center of instrument base 87 with an axis substantially parallel to the axes of the drive inputs 89, rather than orthogonal as in the design of FIG. 16.

When coupled to the rotational assembly 83 of the instrument driver 80, the medical instrument 86, comprising instrument base 87 and instrument shaft 88, rotates in combination with the rotational assembly 83 about the instrument driver axis 85. Since the instrument shaft 88 is positioned at the center of instrument base 87, the instrument shaft 88 is coaxial with instrument driver axis 85 when attached. Thus, rotation of the rotational assembly 83 causes the instrument shaft 88 to rotate about its own longitudinal axis. Moreover, as the instrument base 87 rotates with the instrument shaft 88, any tendons connected to the drive inputs 89 in the instrument base 87 are not tangled during rotation. Accordingly, the parallelism of the axes of the drive outputs 81, drive inputs 89, and instrument shaft 88 allows for the shaft rotation without tangling any control tendons.

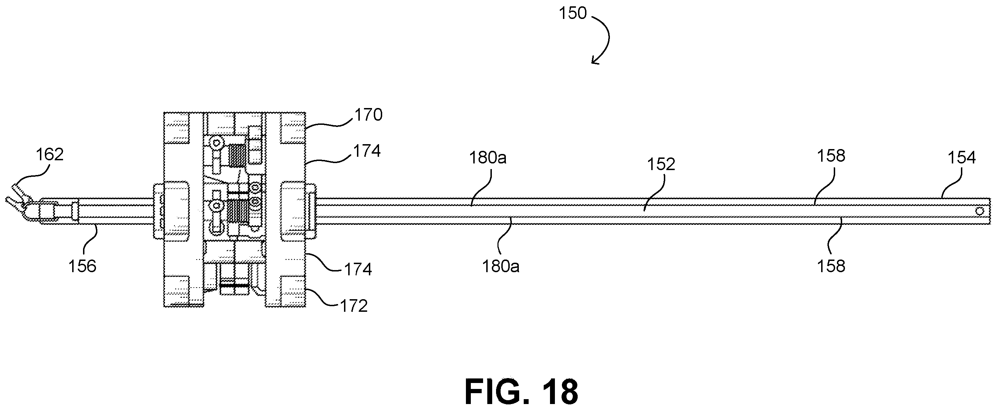

FIG. 18 illustrates an instrument having an instrument-based insertion architecture in accordance with some embodiments. The instrument 150 can be coupled to any of the instrument drivers discussed above. The instrument 150 comprises an elongated shaft 152, an end effector 162 connected to the shaft 152, and a handle 170 coupled to the shaft 152. The elongated shaft 152 comprises a tubular member having a proximal portion 154 and a distal portion 156. The elongated shaft 152 comprises one or more channels or grooves 158 along its outer surface. The grooves 158 are configured to receive one or more wires or cables 180 therethrough. One or more cables 180 thus run along an outer surface of the elongated shaft 152. In other embodiments, cables 180 can also run through the elongated shaft 152. Manipulation of the one or more cables 180 (e.g., via an instrument driver) results in actuation of the end effector 162.

The instrument handle 170, which may also be referred to as an instrument base, may generally comprise an attachment interface 172 having one or more mechanical inputs 174, e.g., receptacles, pulleys or spools, that are designed to be reciprocally mated with one or more torque couplers on an attachment surface of an instrument driver.

In some embodiments, the instrument 150 comprises a series of pulleys or cables that enable the elongated shaft 152 to translate relative to the handle 170. In other words, the instrument 150 itself comprises an instrument-based insertion architecture that accommodates insertion of the instrument, thereby minimizing the reliance on a robot arm to provide insertion of the instrument 150. In other embodiments, a robotic arm can be largely responsible for instrument insertion.

### E. Controller.

Any of the robotic systems described herein can include an input device or controller for manipulating an instrument attached to a robotic arm. In some embodiments, the controller can be coupled (e.g., communicatively, electronically, electrically, wirelessly and/or mechanically) with an instrument such that manipulation of the controller causes a corresponding manipulation of the instrument e.g., via master slave control.

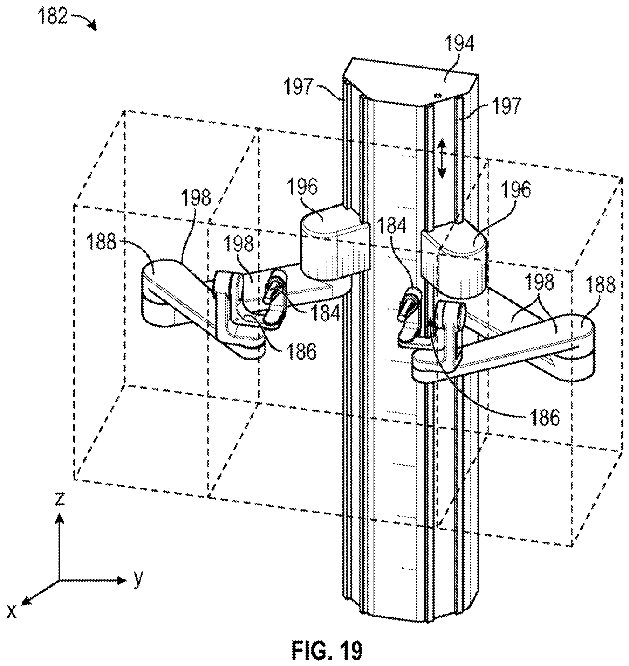

FIG. 19 is a perspective view of an embodiment of a controller 182. In the present embodiment, the controller 182 comprises a hybrid controller that can have both impedance and admittance control. In other embodiments, the controller 182 can utilize just impedance or passive control. In other embodiments, the controller 182 can utilize just admittance control. By being a hybrid controller, the controller 182 advantageously can have a lower perceived inertia while in use.

In the illustrated embodiment, the controller 182 is configured to allow manipulation of two medical instruments and includes two handles 184. Each of the handles 184 is connected to a gimbal 186. Each gimbal 186 is connected to a positioning platform 188.

As shown in FIG. 19, each positioning platform 188 includes a SCARA arm (selective compliance assembly robot arm) 198 coupled to a column 194 by a prismatic joint 196. The prismatic joints 196 are configured to translate along the column 194 (e.g., along rails 197) to allow each of the handles 184 to be translated in the z-direction, providing a first degree of freedom. The SCARA arm 198 is configured to allow motion of the handle 184 in an x-y plane, providing two additional degrees of freedom.

In some embodiments, one or more load cells are positioned in the controller. For example, in some embodiments, a load cell (not shown) is positioned in the body of each of the gimbals 186. By providing a load cell, portions of the controller 182 are capable of operating under admittance control, thereby advantageously reducing the perceived inertia of the controller while in use. In some embodiments, the positioning platform 188 is configured for admittance control, while the gimbal 186 is configured for impedance control. In other embodiments, the gimbal 186 is configured for admittance control, while the positioning platform 188 is configured for impedance control. Accordingly, for some embodiments, the translational or positional degrees of freedom of the positioning platform 188 can rely on admittance control, while the rotational degrees of freedom of the gimbal 186 rely on impedance control.

### F. Navigation and Control.

Traditional endoscopy may involve the use of fluoroscopy (e.g., as may be delivered through a C-arm) and other forms of radiation-based imaging modalities to provide endoluminal guidance to an operator physician. In contrast, the robotic systems contemplated by this disclosure can provide for non-radiation-based navigational and localization means to reduce physician exposure to radiation and reduce the amount of equipment within the operating room. As used herein, the term "localization" may refer to determining and/or monitoring the position of objects in a reference coordinate system. Technologies such as pre-operative mapping, computer vision, real-time EM tracking, and robot command data may be used individually or in combination to achieve a radiation-free operating environment. In other cases, where radiation-based imaging modalities are still used, the pre-operative mapping, computer vision, real-time EM tracking, and robot command data may be used individually or in combination to improve upon the information obtained solely through radiation-based imaging modalities.

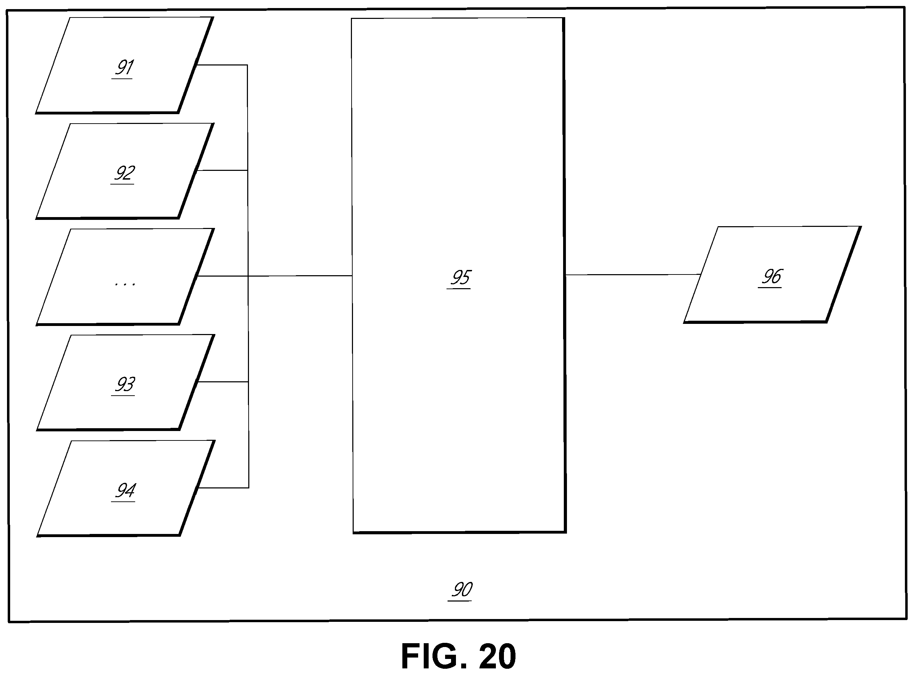

FIG. 20 is a block diagram illustrating a localization system 90 that estimates a location of one or more elements of the robotic system, such as the location of the instrument, in accordance to an example embodiment. The localization system 90 may be a set of one or more computer devices configured to execute one or more instructions. The computer devices may be embodied by a processor (or processors) and computer-readable memory in one or more components discussed above. By way of example and not limitation, the computer devices may be in the tower 30 shown in FIG. 1, the cart shown in FIGS. 1-4, the beds shown in FIGS. 5-14, etc.

As shown in FIG. 20, the localization system 90 may include a localization module 95 that processes input data 91-94 to generate location data 96 for the distal tip of a medical instrument. The location data 96 may be data or logic that represents a location and/or orientation of the distal end of the instrument relative to a frame of reference. The frame of reference can be a frame of reference relative to the anatomy of the patient or to a known object, such as an EM field generator (see discussion below for the EM field generator).

The various input data 91-94 are now described in greater detail. Pre-operative mapping may be accomplished through the use of the collection of low dose CT scans. Pre-operative CT scans are reconstructed into three-dimensional images, which are visualized, e.g., as "slices" of a cutaway view of the patient's internal anatomy. When analyzed in the aggregate, image-based models for anatomical cavities, spaces and structures of the patient's anatomy, such as a patient lung network, may be generated. Techniques such as center-line geometry may be determined and approximated from the CT images to develop a three-dimensional volume of the patient's anatomy, referred to as model data 91 (also referred to as "preoperative model data" when generated using only preoperative CT scans). The use of center-line geometry is discussed in U.S. Pat. App. No. 14/523,760. Network topological models may also be derived from the CT-images and are particularly appropriate for bronchoscopy.

In some embodiments, the instrument may be equipped with a camera to provide vision data 92. The localization module 95 may process the vision data to enable one or more vision-based location tracking. For example, the preoperative model data may be used in conjunction with the vision data 92 to enable computer vision-based tracking of the medical instrument (e.g., an endoscope or an instrument advance through a working channel of the endoscope). For example, using the preoperative model data 91, the robotic system may generate a library of expected endoscopic images from the model based on the expected path of travel of the endoscope, each image linked to a location within the model. Intra-operatively, this library may be referenced by the robotic system in order to compare real-time images captured at the camera (e.g., a camera at a distal end of the endoscope) to those in the image library to assist localization.

Other computer vision-based tracking techniques use feature tracking to determine motion of the camera, and thus the endoscope. Some features of the localization module 95 may identify circular geometries in the preoperative model data 91 that correspond to anatomical lumens and track the change of those geometries to determine which anatomical lumen was selected, as well as the relative rotational and/or translational motion of the camera. Use of a topological map may further enhance vision-based algorithms or techniques.

Optical flow, another computer vision-based technique, may analyze the displacement and translation of image pixels in a video sequence in the vision data 92 to infer camera movement. Examples of optical flow techniques may include motion detection, object segmentation calculations, luminance, motion compensated encoding, stereo disparity measurement, etc. Through the comparison of multiple frames over multiple iterations, movement and location of the camera (and thus the endoscope) may be determined.

The localization module 95 may use real-time EM tracking to generate a real-time location of the endoscope in a global coordinate system that may be registered to the patient's anatomy, represented by the preoperative model. In EM tracking, an EM sensor (or tracker) comprising of one or more sensor coils embedded in one or more locations and orientations in a medical instrument (e.g., an endoscopic tool) measures the variation in the EM field created by one or more static EM field generators positioned at a known location. The location information detected by the EM sensors is stored as EM data 93. The EM field generator (or transmitter), may be placed close to the patient to create a low intensity magnetic field that the embedded sensor may detect. The magnetic field induces small currents in the sensor coils of the EM sensor, which may be analyzed to determine the distance and angle between the EM sensor and the EM field generator. These distances and orientations may be intra-operatively "registered" to the patient anatomy (e.g., the preoperative model) in order to determine the geometric transformation that aligns a single location in the coordinate system with a position in the pre-operative model of the patient's anatomy. Once registered, an embedded EM tracker in one or more positions of the medical instrument (e.g., the distal tip of an endoscope) may provide real-time indications of the progression of the medical instrument through the patient's anatomy.

Robotic command and kinematics data 94 may also be used by the localization module 95 to provide localization data 96 for the robotic system. Device pitch and yaw resulting from articulation commands may be determined during pre-operative calibration. Intra-operatively, these calibration measurements may be used in combination with known insertion depth information to estimate the position of the instrument. Alternatively, these calculations may be analyzed in combination with EM, vision, and/or topological modeling to estimate the position of the medical instrument within the network.

As FIG. 20 shows, a number of other input data can be used by the localization module 95. For example, although not shown in FIG. 20, an instrument utilizing shape-sensing fiber can provide shape data that the localization module 95 can use to determine the location and shape of the instrument.

The localization module 95 may use the input data 91-94 in combination(s). In some cases, such a combination may use a probabilistic approach where the localization module 95 assigns a confidence weight to the location determined from each of the input data 91-94. Thus, where the EM data may not be reliable (as may be the case where there is EM interference) the confidence of the location determined by the EM data 93 can be decrease and the localization module 95 may rely more heavily on the vision data 92 and/or the robotic command and kinematics data 94.

As discussed above, the robotic systems discussed herein may be designed to incorporate a combination of one or more of the technologies above. The robotic system's computer-based control system, based in the tower, bed and/or cart, may store computer program instructions, for example, within a non-transitory computer-readable storage medium such as a persistent magnetic storage drive, solid state drive, or the like, that, upon execution, cause the system to receive and analyze sensor data and user commands, generate control signals throughout the system, and display the navigational and localization data, such as the position of the instrument within the global coordinate system, anatomical map, etc.

### 2. Introduction to Systems, Devices, and Methods for Real-Time 3D Robotic Status

This application discloses robotic medical systems that use real-time renderings (e.g., three-dimensional (3-D) renderings, such as 3-D images, models, information, etc.) to communicate a status of the robotic medical systems to a physician / physician assistant.

As described herein, robotic medical systems may be configured to execute pre-programmed workflow corresponding to a procedure, to guide the physician / physician assistant through the procedure. In some embodiments, the pre-programmed workflow can include one or more stages, such as a pre-operative stage, an intra-operative stage, and/or a post-operative (e.g., teardown) stage. Each of the stages can include one or more respective steps. The 3-D renderings can be used to communicate real-time status of the robotic medical system during the one or more stages, and in particular positional changes of the robotic medical system in real time.

As described herein, the 3-D renderings can be used to communicate a real-time status of the robotic medical system in response to robotic control and/or manual control of the robotic medical system.

In some embodiments, the 3-D renderings can be used to communicate a status of the robotic medical system in response to manual control of the robotic medical system.

### A. Robotic System

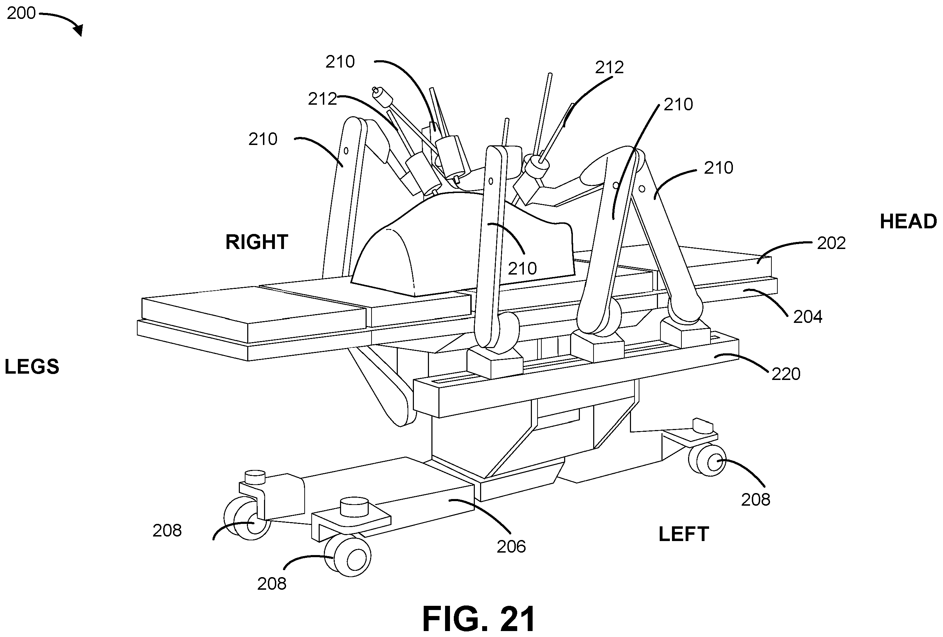

FIG. 21 illustrates an exemplary robotic medical system 200 according to some embodiments. In some embodiments, the robotic medical system 200 is a robotic medical system (e.g., robotic surgery system). In the example of FIG. 21, the robotic medical system 200 comprises a patient support platform 202 (e.g., a patient platform, a table, a bed, etc.). The two ends along the length of the patient support platform 202 are respectively referred to as "head" and "leg". The two sides of the patient support platform 202 are respectively referred to as "left" and "right." The patient support platform 202 includes a support 204 (e.g., a rigid frame) for the patient support platform 202.

The robotic medical system 200 also comprises a base 206 for supporting the robotic medical system 200. The base 206 includes wheels 208 that allow the robotic medical system 200 to be easily movable or repositionable in a physical environment. In some embodiments, the wheels 208 are omitted from the robotic medical system 200 or are retractable, and the base 206 can rest directly on the ground or floor. In some embodiments, the wheels 208 are replaced with feet.

The robotic medical system 200 includes one or more robotic arms 210. The robotic arms 210 can be configured to perform robotic medical procedures as described above with reference to FIGS. 1-20. Although FIG. 21 shows five robotic arms 210, it should be appreciated that the robotic medical system 200 may include any number of robotic arms, including less than five or six or more.

The robotic medical system 200 also includes one or more bars 220 (e.g., adjustable arm support or an adjustable bar) that support the robotic arms 210. Each of the robotic arms 210 is supported on, and movably coupled to, a bar 220, by a respective base joint of the robotic arm. In some embodiments, and as described in FIG. 12, bar 220 can provide several degrees of freedom, including lift, lateral translation, tilt, etc. In some embodiments, each of the robotic arms 210 and/or the adjustable arm supports 220 is also referred to as a respective kinematic chain.

FIG. 21 shows three robotic arms 210 supported by the bar 220 that is in the field of view of the figure. The two remaining robotic arms are supported by another bar that is located across the other length of the patient support platform 202.

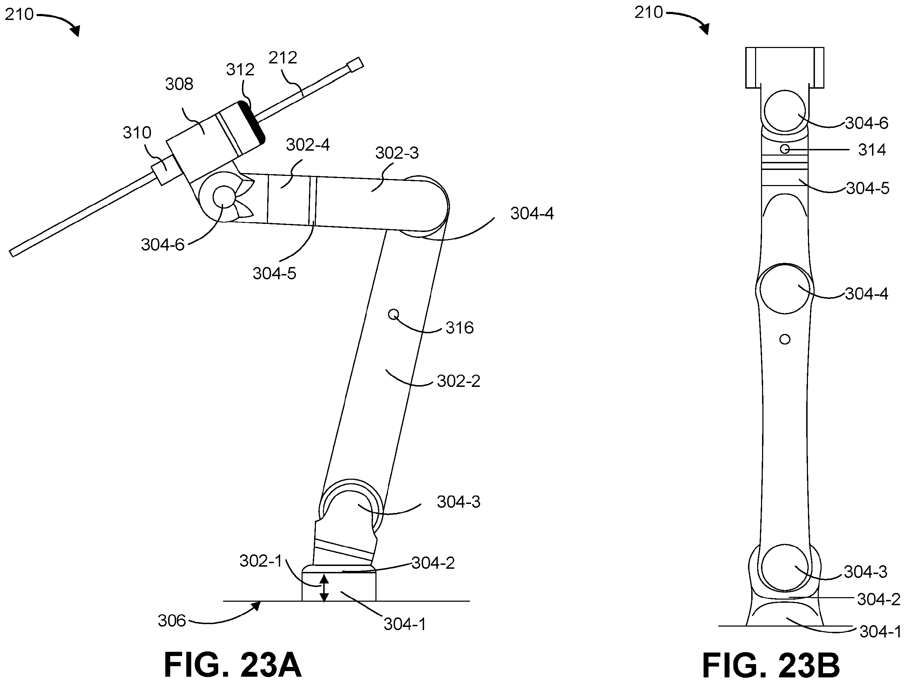

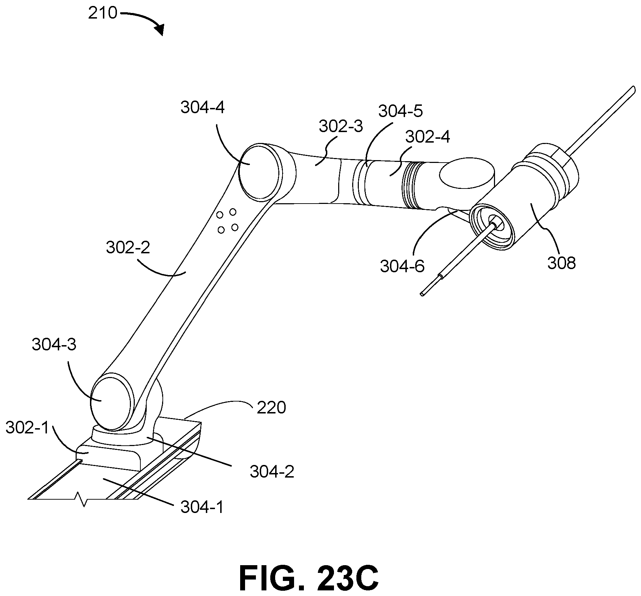

In some embodiments, the adjustable arm supports 220 can be configured to provide a base position for one or more of the robotic arms 210 for a robotic medical procedure. A robotic arm 210 can be positioned relative to the patient support platform 202 by translating the robotic arm 210 along a length of its underlying bar 220 and/or by adjusting a position and/or orientation of the robotic arm 210 via one or more joints and/or links (see, e.g., FIG. 23). In some embodiments, the bar pose can be changed via manual manipulation, teleoperation, and/or power assisted motion.

In some embodiments, the adjustable arm support 220 can be translated along a length of the patient support platform 202. In some embodiments, translation of the bar 220 along a length of the patient support platform 202 causes one or more of the robotic arms 210 supported by the bar 220 to be simultaneously translated with the bar or relative to the bar. In some embodiments, the bar 220 can be translated while keeping one or more of the robotic arms stationary with respect to the base 206 of the robotic medical system 200.

In the example of FIG. 21, the adjustable arm support 220 is located along a length of the patient support platform 202. In some embodiments, the adjustable arm support 220 may extend across a partial or full length of the patient support platform 202, and/or across a partial or full width of the patient support platform 202.

During a robotic medical procedure, one or more of the robotic arms 210 can also be configured to hold instruments 212 (e.g., robotically controlled medical instruments or tools, such as an endoscope and/or any other instruments (e.g., sensors, illumination instrument, cutting instrument, etc.) that may be used during surgery), and/or be coupled to one or more accessories, including one or more cannulas, in accordance with some embodiments.

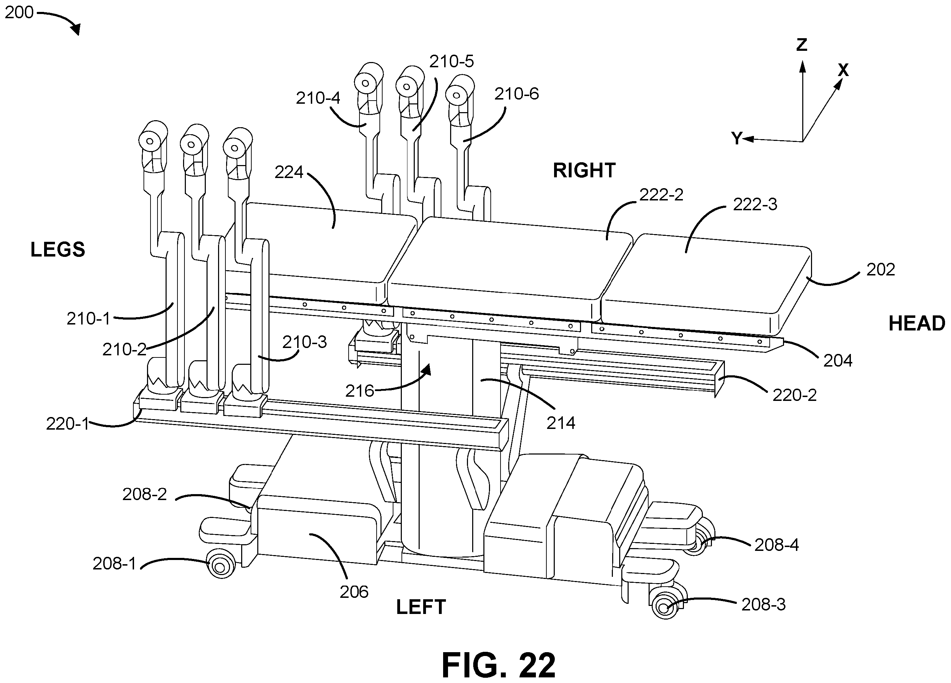

FIG. 22 illustrates another view of the exemplary robotic medical system 200 in FIG. 21 according to some embodiments. In this example, the robotic medical system 200 includes six robotic arms 210-1, 210-2, 210-3, 210-4, 210-5, and 210-6. The patient platform 202 is supported by a column 214 that extends between the base 206 and the patient platform 202. In some embodiments, the patient platform 202 comprises a tilt mechanism 216. The tilt mechanism 216 can be positioned between the column 214 and the patient platform 202 to allow the patient platform 202 to pivot, rotate, or tilt relative to the column 214. The tilt mechanism 216 can be configured to allow for lateral and/or longitudinal tilt of the patient platform 202. In some embodiments, the tilt mechanism 216 allows for simultaneous lateral and longitudinal tilt of the patient platform 202.

FIG. 22 shows the patient platform 202 in an untilted state or position. In some embodiments, the untilted state or position is a default position of the patient platform 202. In some embodiments, the default position of the patient platform 202 is a substantially horizontal position as shown in FIG. 22. As illustrated, in the untilted state, the patient platform 202 can be positioned horizontally or parallel to a surface that supports the robotic medical system 200 (e.g., the ground or floor). In some embodiments, the term "untilted" refers to a state in which the angle between the default position and the current position is less than a threshold angle (e.g., less than 5 degrees, or less than an angle that would cause the patient to shift on the patient platform, etc.). In some embodiments, the term "untilted" refers to a state in which the patient platform is substantially perpendicular to the direction of gravity, irrespective of the angle formed by the surface that supports the robotic medical system relative to gravity.

With continued reference to FIG. 22, in the illustrated example of the robotic medical system 200, the patient platform 202 comprises a support 204. In some embodiments, the support 204 includes a rigid support structure or frame, and can support one or more surfaces, pads, or cushions 222. An upper surface of the patient platform 202 can include a support surface 224. During a medical procedure, a patient can be placed on the support surface 224.

FIG. 22 shows the robotic arms 210 and the adjustable arm supports 220 in an exemplary deployed configuration in which the robotic arms 210 reach above the patient platform 202. In some embodiments, due to the configuration of the robotic medical system 200, which enables stowage of different components beneath the patient platform 202, the robotic arms 210 and the arm supports 220 can occupy a space underneath the patient platform 202. Thus, in some embodiments, the tilt mechanism 216 has a low-profile and/or low volume in order to increase the space available for storage below.

FIG. 22 also illustrates an example, x, y, and z coordinate system that may be used to describe certain features of the embodiments disclosed herein. It will be appreciated that this coordinate system is provided for purposes of example and explanation only and that other coordinate systems may be used. In the illustrated example, the x-direction or x-axis extends in a lateral direction across the patient platform 202 when the patient platform 202 is in an untilted state. In some configurations, the x-direction extends across the patient platform 202 from one lateral side (e.g., the right side) to the other lateral side (e.g., the left side) when the patient platform 202 is in an untilted state. The y-direction or y-axis extends in a longitudinal direction along the patient platform 202 when the patient platform 202 is in an untilted state. That is, the y-direction extends along the patient platform 202 from one longitudinal end (e.g., the head end) to the other longitudinal end (e.g., the legs end) when the patient platform 202 is in an untilted state. In an untilted state, the patient platform 202 can lie in or be parallel to the x-y plane, which can be parallel to the floor or ground. In the illustrated example, the z-direction or z-axis extends along the column 214 in a vertical direction. In some embodiments, the tilt mechanism 216 is configured to laterally tilt the patient platform 202 by rotating the patient platform 202 about a lateral tilt axis that is parallel to the y-axis. The tilt mechanism 216 can further be configured to longitudinally tilt the patient platform 202 by rotating the patient platform 202 about a longitudinal tilt axis that is parallel to the x-axis.

### B. Robotic Arm

FIGS. 23A to 23C illustrate different views of an exemplary robotic arm 210 according to some embodiments.

FIG. 23A illustrates that the robotic arm 210 includes a plurality of links 302 (e.g., linkages). The links 302 are connected by one or more joints 304. Each of the joints 304 includes one or more degrees of freedom (DoFs).

In FIG. 23A, the joints 304 include a first joint 304-1 (e.g., a base joint or an A0 joint) that is located at or near a base 306 of the robotic arm 210. In some embodiments, the base joint 304-1 comprises a prismatic joint that allows the robotic arm 210 to translate along the bar 220 (e.g., along the y-axis). The joints 304 also include a second joint 304-2. In some embodiments, the second joint 304-2 rotates with respect to the base joint 304-1. The joints 304 also include a third joint 304-3 that is connected to one end of link 302-2. In some embodiments, the joint 304-3 includes multiple DoFs and facilitates both tilt and rotation of the link 302-2 tilt with respect to the joint 304-3.

FIG. 23A also shows a fourth joint 304-4 that is connected to the other end of the link 302-2. In some embodiments, the joint 304-4 comprises an elbow joint that connects the link 302-2 and the link 302-3. The joints 304 further comprise a pair of joints 304-5 (e.g., a wrist roll joint) and 304-6 (e.g., a wrist pitch joint), which is located on a distal portion of the robotic arm 210.

A proximal end of the robotic arm 210 may be connected to a base 306 and a distal end of the robotic arm 210 may be connected to an advanced device manipulator (ADM) 308 (e.g., a tool driver, an instrument driver, or a robotic end effector, etc.). The ADM 308 may be configured to control the positioning and manipulation of a medical instrument 212 (e.g., a tool, a scope, etc.).

The robotic arm 210 can also include a cannula sensor 310 for detecting presence or proximity of a cannula to the robotic arm 210. In some embodiments, the robotic arm 210 is placed in a docked state (e.g., docked position) when the cannula sensor 310 detects presence of a cannula (e.g., via one or more processors of the robotic medical system 200). In some embodiments, when the robotic arm 210 is in a docked position, the robotic arm 210 can execute null space motion to maintain a position and/or orientation of the cannula, as discussed in further detail below. Conversely, when no cannula is detected by the cannula sensor 310, the robotic arm 210 is placed in an undocked state (e.g., undocked position).

In some embodiments, and as illustrated in FIG. 23A, the robotic arm 210 includes an input or button 312 (e.g., a donut-shaped button, or other types of controls, etc.) that can be used to place the robotic arm 210 in an admittance mode (e.g., by depressing the button 312). The admittance mode is also referred to as an admittance scheme or admittance control. In the admittance mode, the robotic system 210 measures forces and/or torques (e.g., imparted on the robotic arm 210) and outputs corresponding velocities and/or positions. In some embodiments, the robotic arm 210 can be manually manipulated by a user (e.g., during a set-up procedure, or in between procedures, etc.) in the admittance mode. In some instances, by using admittance control, the operator need not overcome all of the inertia in the robotic medical system 200 to move the robotic arm 210. For example, under admittance control, when the operator imparts a force on the arm, the robotic medical system 200 can measure the force and assist the operator in moving the robotic arm 210 by driving one or more motors associated with the robotic arm 210, thereby resulting in desired velocities and/or positions of the robotic arm 210.

In some embodiments, the links 302 may be detachably coupled to the medical tool 212 (e.g., to facilitate ease of mounting and dismounting of the medical tool 212 from the robotic arm 210). The joints 304 provide the robotic arm 210 with a plurality of degrees of freedom (DoFs) that facilitate control of the medical tool 212 via the ADM 308. In an embodiment as shown in FIG. 23 including multiple robotic arms, each robotic arm can hold its own respective medical tool and pivot the medical tool about a remote center of motion.

FIG. 23B illustrates a front view of the robotic arm 210. FIG. 23C illustrates a perspective view of the robotic arm 210. In some embodiments, the robotic arm 210 includes a second input or button 314 (e.g., a push button) that is distinct from the button 312 in FIG. 23A, for placing the robotic arm 210 in an impedance mode (e.g., by a single press or continuous press and hold of the button 314). In this example, the button 314 is located between the joint 304-5 and the joint 304-6. The impedance mode is also referred to as impedance scheme or impedance control. In the impedance mode, the robotic medical system 200 measures displacements (e.g., changes in position and velocity) and outputs forces and/or torques to facilitate manual movement of the robotic arm. In some embodiments, the robotic arm 210 can be manually manipulated by a user (e.g., during a set-up procedure) in the impedance mode. In some embodiments, under the impedance mode, the operator's movement of one part of a robotic arm 210 may cause motion in one or more joints and/or links throughout the robotic arm 210.

In some embodiments, for admittance control, a force sensor or load cell can measure the force that the operator is applying to the robotic arm 210 and move the robotic arm 210 in a way that feels light. Admittance control may feel lighter than impedance control because, under admittance control, one can hide the perceived inertia of the robotic arm 210 because motors in the controller can help to accelerate the mass. In contrast, with impedance control, the user is responsible for most if not all mass acceleration, in accordance with some embodiments.

In some circumstances, depending on the position of the robotic arm 210 relative to the operator, it may be inconvenient to reach the button 312 and/or the button 314 to activate a manual manipulating mode (e.g., the admittance mode and/or the impedance mode). Accordingly, under these circumstances, it may be convenient for the operator to trigger the manual manipulation mode other than by buttons.

In some embodiments, the robotic arm 210 includes a single button (e.g., the button 312 or 314) that can be used to place the robotic arm 210 in the admittance mode and/or the impedance mode (e.g., by using different presses, such as a long press, a short press, press and hold etc.). In some embodiments, the robotic arm 210 can be placed in impedance mode by a user pushing on arm linkages (e.g., the links 302) and/or joints (e.g., the joints 304) and overcoming a force threshold. In some embodiments, the admittance mode and the impedance mode are common in that they both allow the user to grab the robotic arm 210 and command motion by directly interfacing with it.

In some embodiments, the robotic arm 210 includes an input control for activating an arm follow mode. For example, in some embodiments, the robotic arm 210 can include a designate touch point that is located on a link 302 or a joint 304 of the robotic arm (e.g., an outer shell of the link 302 or a button 316). User interaction (e.g., user touch, contact, etc.) with the designate touch point activates the arm follow mode. In some embodiments, the robotic arm 210 includes multiple touch points. User interaction with any (e.g., one or more) of the touch points activates the arm follow mode.

During a medical procedure, it can be desirable to have the ADM 308 of the robotic arm 210 and/or a remote center of motion (RCM) of the tool 212 coupled thereto kept in a static pose (e.g., position and/or orientation). An RCM may refer to a point in space where a cannula or other access port through which a medical tool 212 is inserted is constrained in motion. In some embodiments, the medical tool 212 includes an end effector that is inserted through an incision or natural orifice of a patient while maintaining the RCM. In some embodiments, the medical tool 212 includes an end effector that is in a retracted state during a setup process of the robotic medical system.

In some circumstances, the robotic medical system 200 can be configured to move one or more links 302 of the robotic arm 210 within a "null space" to avoid collisions with nearby objects (e.g., other robotic arms), while the ADM 308 of the robotic arm 210 and/or the RCM are maintained in their respective poses (e.g., positions and/or orientations). The null space can be viewed as the set of joint states through which a robotic arm 210 can move that does not result in movement of the ADM 308 and/or RCM, thereby maintaining the position and/or the orientation of the medical tool 212 (e.g., within a patient). In some embodiments, a robotic arm 210 can have multiple positions and/or configurations available for each pose of the ADM 308.

For a robotic arm 210 to move an instrument to a desired pose in space, in certain embodiments, the robotic arm 210 may have at least six DoFs - three DoFs for translation (e.g., X, Y, and Z positions) and three DoFs for rotation (e.g., yaw, pitch, and roll). In some embodiments, each joint 304 may provide the robotic arm 210 with a single DoF, and thus, the robotic arm 210 may have at least six joints to achieve freedom of motion to position the ADM 308 at any pose in space. To further maintain the ADM 308 of the robotic arm 210 and/or the remote center or motion in a desired pose, the robotic arm 210 may further have at least one additional "redundant joint." Thus, in certain embodiments, the system may include a robotic arm 210 having at least seven joints 304, providing the robotic arm 210 with at least seven DoFs. In some embodiments, the robotic arm 210 may include a subset of joints 304 each having more than one degree of freedom thereby achieving the additional DoFs for null space motion. However, depending on the embodiment, the robotic arm 210 may have a greater or fewer number of DoFs.

Furthermore, as described with respect to FIG. 12, the bar 220 (e.g., adjustable arm support) can provide several degrees of freedom, including lift, lateral translation, tilt, etc. Thus, depending on the embodiment, a robotic medical system can have many more robotically controlled degrees of freedom beyond just those in the robotic arms 210 to provide for null space movement and collision avoidance. In a respective embodiment of these embodiments, the end effectors of one or more robotic arms (and any tools or instruments coupled thereto) and a remote center along the axis of the tool can advantageously maintain in pose and/or position within a patient.

A robotic arm 210 having at least one redundant DoF has at least one more DoF than the minimum number of DoFs for performing a given task. For example, a robotic arm 210 can have at least seven DoFs, where one of the joints 304 of the robotic arm 210 can be considered a redundant joint, in accordance with some embodiments. The one or more redundant joints can allow the robotic arm 210 to move in a null space to both maintain the pose of the ADM 308 and a position of an RCM and avoid collision(s) with other robotic arms or objects.

In some embodiments, the robotic medical system 200 can be configured to perform collision avoidance to avoid collision(s), e.g., between adjacent robotic arms 210, by taking advantage of the movement of one or more redundant joints in a null space. For example, when a robotic arm 210 collides with or approaches (e.g., within a defined distance of) another robotic arm 210, one or more processors of the robotic medical system 200 can be configured to detect the collision or impending collision (e.g., via kinematics). Accordingly, the robotic medical system 200 can control one or both of the robotic arms 210 to adjust their respective joints within the null space to avoid the collision or impending collision. In an embodiment including at least a pair of robotic arms, a base of one of the robotic arms and its end effector can stay in its pose, while links or joints therebetween move in a null space to avoid collisions with an adjacent robotic arm.

### C. Real-Time 3-D Renderings that Represent Status of the Robotic Medical System i. Guiding and/or Controlling Robotic Positional Changes

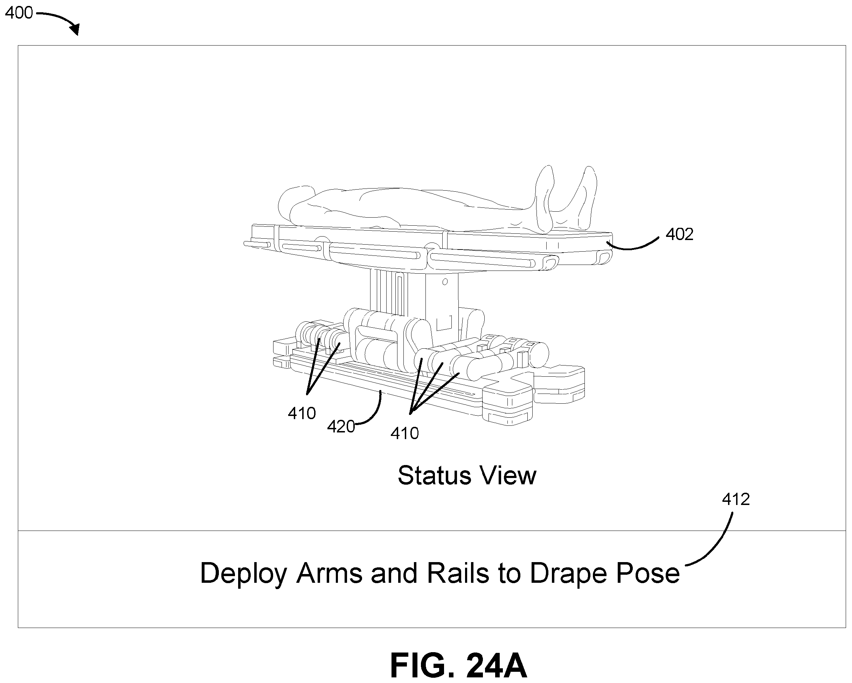

FIGS. 24A to 24C illustrate 3-D renderings (e.g., 3-D models, visual representations, etc.) of a robotic medical system (e.g., the robotic medical system 200 as depicted in FIGS. 21 and 22) during a setup stage of a procedure in accordance with some embodiments.

As used herein, a "3-D rendering" can include a 3-D graphic, a 3-D representation, or a computer-generated 3-D model (e.g., to scale or not to scale) of the robotic medical system 200 or a part thereof, such as one or more robotic arms, links and/or joints of a robotic arm, arm support(s), and/or a patient support platform of the robotic medical system 200. In some embodiments, the 3-D rendering includes an isometric view and/or a perspective view of the robotic medical system 200. In some embodiments, the 3-D rendering corresponds to a live (e.g., real-time) 3-D animated scene of the robotic medical system 200, which includes the robotic arms 210, adjustable arm supports (e.g., bars) 220, and/or patient support platform 204. In some embodiments, the 3-D scene includes a field of view (e.g., from a virtual camera) that is dynamically updated depending on the stage (or step) of the pre-programmed workflow. In some embodiments, the 3-D rendering can include an image (e.g., a two-dimensional (2-D) image) that is rendered to provide the user with a perception of depth. For example, the 3-D rendering can be a 2-D image that is color-rendered (e.g., using different color tones) to indicate depths (and as a result, provide a sense of depth or distance) of the robotic arms and the patient support platform. Additionally or alternatively, the 3-D rendering may include occlusion of one or more portions of the robotic arms and the patient support platform to indicate depth of the robotic arms and the patient support platform. In some embodiments, the 3-D rendering can include an image (e.g., a 2-D image) that is shaded to show the character and/or contour of different surfaces of the robotic system (e.g., surfaces of the robotic arms, surfaces of the bars, surfaces of the patient support platform, etc.).

FIG. 24A shows a 3-D rendering 400 that includes a visual representation 402 corresponding to the patient support 202, visual representations 410 corresponding to the robotic arms 210, and a visual representation 420 corresponding to the adjustable arm support(s) 220. The 3-D rendering 400 reflects a real-time status of the robotic medical system 200, in which the robotic arms 210 and the adjustable arm supports 220 are in a stowed configuration prior to setup. In FIG. 24A, the 3-D rendering 400 also includes a visual representation corresponding to a patient.

In some embodiments, the robotic medical system 200 generates the 3-D rendering in real-time (e.g., on-the-fly) in accordance with a determination of the status of the robotic medical system 200. For example, the robotic medical system 200 can use data that is determined by sensors and/or encoders that are included in robotic medical system 200, to determine corresponding positions and/or orientation of joints and/or links of the robotic arms 210, pose(s) (e.g., positions and/or orientations) of the arm support(s) 220, and/or a position and/or orientation of the patient support platform 202. In some embodiments, the robotic medical system 200 generates (e.g., constructs) 3-D representation(s) of the robotic medical system using the sensor data and displays the 3-D representation(s). In some embodiments, the robotic medical system 200 also determines changes in the positions and/or orientations of the robotic arms, arm supports, and/or patient support platform (e.g., in real-time, continuously, constantly, such as every 5 seconds, 10 seconds, 30 seconds, etc.), and updates the 3-D rendering in accordance with the changes in the positions and/or orientations of the robotic arms, arm supports, and/or patient support platform, to reflect status changes in the robotic medical system 200.

Additionally or alternatively, in some embodiments, the robotic medical system 200 stores (e.g., locally, on a remote computer system, etc.) a library (e.g., a database, a stock, etc.) of images corresponding to different configurations (e.g., position and/or orientation) of the robotic medical system. The robotic medical system 200 can use the sensors and/or encoders attached thereto to determine, in real-time, the positions and/or orientation of the joints and/or links of the robotic arms 210, poses of the arm supports 220, and/or a position of the patient support platform 202, and select an image from the stored images that most closely matches the determined configuration for display as a 3-D rendering.

In some embodiments, the 3-D rendering is displayed on one or more displays of the robotic medical system 200. The one or more displays can be located on a tower viewer, surgeon viewer, a bed/tower pendant of the robotic medical system 200. In some embodiments, the one or more displays can be located on any pendant (e.g., a remote pendant) that is communicatively connected to and used as part of the robotic medical system 200.

In some embodiments, the workflow includes a setup stage, and an intra-operative stage, and a post-operative stage. Each of the stages can include one or more respective steps. For example, the setup stage can include steps such as: deploying the robotic arms 210 and arm supports 220 (e.g., rails, bars, etc.) from a stowed state to a deployed state, draping the robotic arms 210 and arm supports 220, positioning the robotic arms 210 and the arm supports 220 in a pre-docking pose, manually adjusting the bars 220 and/or arms 210, establishing boundary condition(s) that are specific to a particular surgery and/or based on accessor(ies) that are utilized for the procedure, docking the robotic arms 210, attaching instrument(s) to the robotic arm(s) 210, and optimizing the position of the arm supports 220.

In some embodiments, for each step of the workflow, the robotic medical system 200 defines a particular perspective (e.g., viewpoint). In some embodiments, the particular perspective that may be most helpful / informative to the user in terms of monitoring or completing the step is selected. In some embodiments, the robotic medical system 200 stores information representing perspectives selected for (or associated with) respective steps of the workflow. In some embodiments, a step of the workflow can include (or be associated with) multiple (e.g., different) viewpoints (e.g., different fields of view). The one or more displays can include a user interface that provides a toggle element that, when selected, enables a user to toggle between the different viewpoints so as to better visualize the positions of components of the robotic medical system. In some embodiments, the one or more displays concurrently present renderings for the multiple viewpoints.

In some embodiments, the 3-D rendering includes visual indicators to convey a state of the robotic medical system 200 during the setup stage of a procedure. For example, in FIG. 24A, a text field 412 ("Deploy Arms and Rails to Drape Pose") is displayed concurrently with the 3-D rendering 400. In some implementations, the text field 412 guides a user through the next step of the procedure.

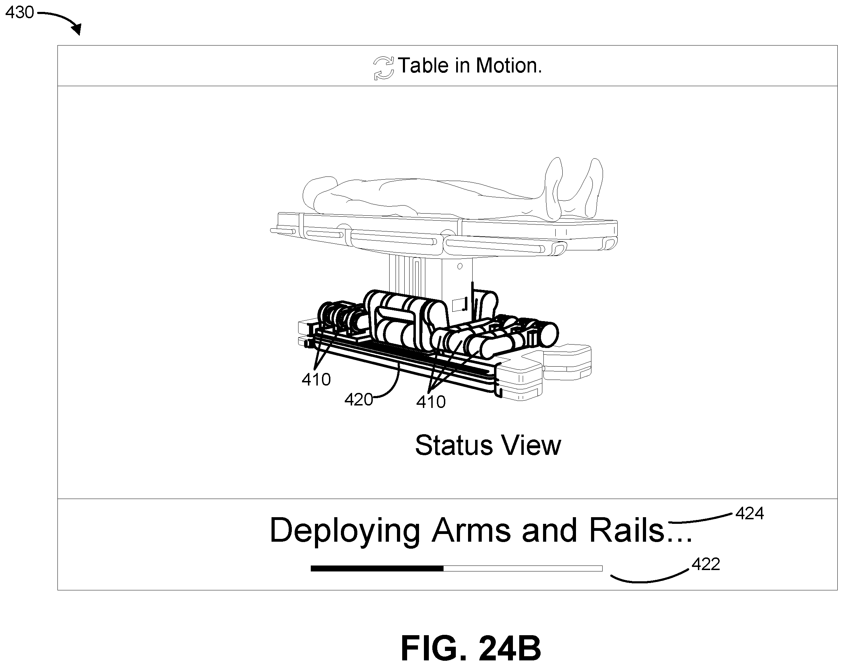

FIG. 24B illustrates an updated 3-D rendering 430 that is displayed in response to user selection of the visual representations 410 and/or 420. In some embodiments, the user selection of the visual representations 410 and/or 420 correspond to a user instruction to control the robotic arms 210 and/or arm supports 220 (e.g., using the tower pendant). In some embodiments, in response to the user selection, the robotic medical system 200 renders the visual representations 410 and 420 in a visually distinctive manner (e.g., highlighted, shaded, displayed with a different color, including visual effects or animations such as pulsing, and/or other visual emphasis) from other portions of the robotic system in the rendering. For example, in response to user selection of one or more portions of the 3-D rendering, the selected portions of the 3-D rendering model are highlighted (e.g., using a color such as blue, yellow, red, orange, or any other color, which is different from the rest of the rendering) to indicate the user selection (e.g., selection of one or more components or portions of the robotic medical system 200 for control from, for example, the tower pendant).

In some embodiments, user selection of a portion of the 3-D rendering comprises direct user selection, such as a user tap and/or touch on a touchscreen display, user click of a portion of the 3-D rendering using a mouse, etc. The user selection is interpreted by the robotic medical system 200 (e.g., the one or more processors) as portions to be robotically controlled by the robotic medical system 200.

In some embodiments, user selection of a portion of the 3-D rendering comprises indirect selection. For example, the 3-D rendering can be displayed together with a list of steps (e.g., text fields) of the procedure workflow. The user can indirectly select a portion of the 3-D rendering by selecting the text field corresponding to a particular workflow step. In response, the robotic medical system 200 identifies one or more portions of the robotic medical system corresponding to the selected workflow step, and causes (e.g., executes) robotic movement on the identified portions.

In some embodiments, in accordance with user selection of one or more portions of the robotic medical system in the 3-D rendering, the robotic medical system 200 causes (e.g., executes) robotic movement of the selected portions in accordance with the pre-programmed workflow step.

FIG. 24B also shows a progress bar 422 that is displayed concurrently with the 3-D rendering 430 as robotic movement is executed on the robotic medical system 200, to indicate progress to completion of a movement being controlled. In some embodiments, a text field 424 (e.g., "Deploying Arms and Rails") is displayed with the 3-D rendering 430. The text field 424 describes a current step of the workflow that is being executed by the robotic medical system 200.

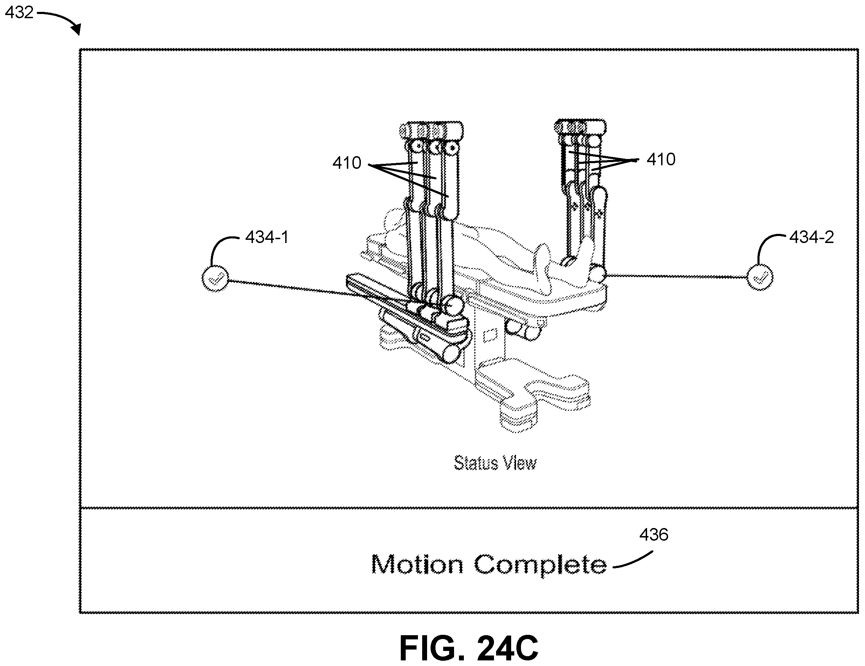

FIG. 24C shows a 3-D rendering 432 in which the visual representations 410 corresponding to the robotic arms 210 are in a substantially vertical pose. The 3-D rendering 432 matches (e.g., substantially matches or corresponds to) a real-time status of the robotic medical system 200 in which robotic-controlled movement to the drape pose has been completed and the robotic arms 210 have been straightened. In some embodiments, the visual representations 410 corresponding to the robotic arms are displayed in a manner that is visually distinct (e.g., highlighted, shaded, displayed with a different color, includes visual effects such as pulsing and/or other visual emphasis) from that used to depict the 3-D rendering 420 and/or the 3-D rendering 410 in FIG. 24B, to indicate completion of a step. For example, portions of the 3-D rendering can change from a first color to a second color (e.g., from white to blue) in response to user selection, and change from the second color to a third color (e.g., from blue to green) upon completion of a movement or step, to communicate status to a user. In some embodiments, as illustrated in FIG. 24C, the 3-D rendering 432 includes checkmark badges 434 (or other visual indicators), such as 434-1 and 434-2, to indicate completion of the movement (e.g., robotic movement) of the robotic arms 210 and/or adjustable arm support 220. In some embodiments, the 3-D rendering 432 is accompanied by (e.g., displayed with) text 436 (e.g., "Motion Complete") indicating completion of a step. In some embodiments, the robotic medical system 200 also synchronizes movement and/or completion statuses with audio tones (e.g., the robotic medical system 200 may output one or more audio tones to indicate a particular status of the robotic medical system 200).

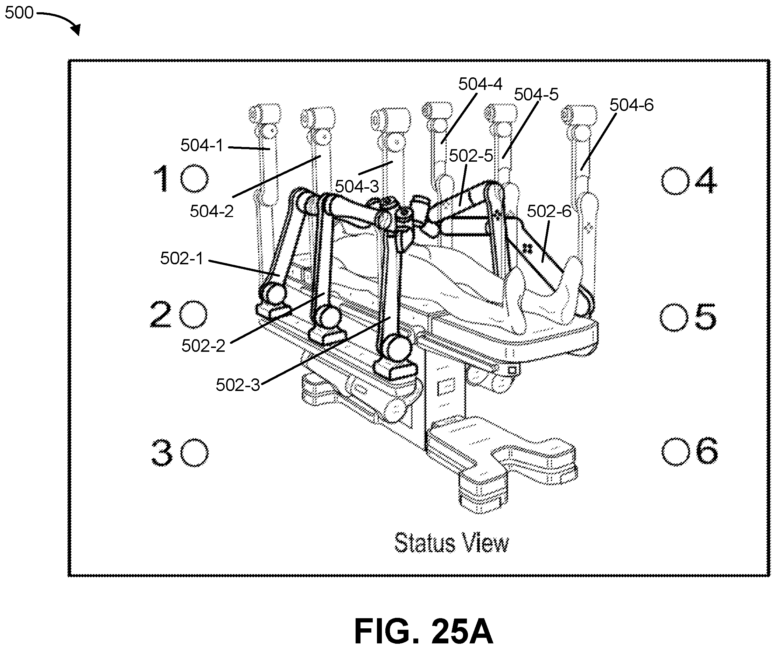

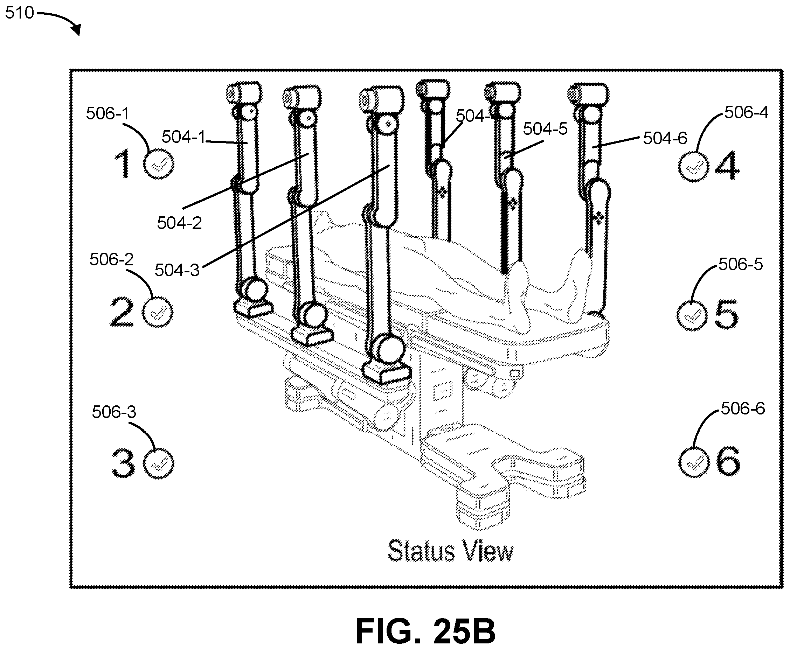

FIGS. 25A and 25B illustrate 3-D renderings of a robotic medical system 200 during a post-operative (e.g., teardown) stage of a procedure, in accordance with some embodiments.

In some embodiments, the post-operative stage includes steps such as removing surgical tools that are attached to the robotic arms, undocking the robotic arms, leveling the patient support platform and/or arm supports, adjusting the robotic arms for stowing, undraping the robotic arms and/or bar, and/or stowing the robotic arms and bars. The undraping step can involve straightening the robotic arms back to a "known position" prior to removing the drapes.

FIG. 25A shows a 3-D rendering 500 of the robotic medical system 200, in which the actual positions of the robotic arms 210 are visually represented by elements 502 (e.g., 502-1 through 502-6) in the rendering 500. The 3-D rendering 500 includes visual representations 504 (e.g., 504-1 through 504-6) of the "target positions" of the robotic arms 210, corresponding to where the robotic arms 210 need to be manually moved to at bedside. In some embodiments, the visual representations 504 of the "target positions" are rendered as a "ghosted" view (e.g., semi-transparent having a transparency of at least 10%, such as 10%, 20%, 30%, 40%, 50%, 60%, 70%, 80%, 90%, or 95%, or within any range between two of the aforementioned values).

FIG. 25B shows that upon completion of the movement (e.g., robotic movement and/or manual movement) of the robotic arms 210 to the target positions (e.g., straightened known positions), the robotic medical system 200 displays an updated 3-D rendering 510 in which the elements 502 are located at positions of visual representations 504 of the target positions, indicating that the robotic arms 210 have moved to the target positions. In some embodiments, the robotic medical system 200 ceases to display either the visual representations 504 of the target positions or the elements 502 (partly because the visual representations 504 overlap with the elements 502). In some embodiments, upon completion of the step, the robotic arms in the 3-D rendering 510 are represented in a different manner (e.g., as a different color, different line thickness, highlighted, outlined, shaded, etc.) from other portions of the 3-D rendering 510. In some embodiments, the 3-D rendering 510 also includes one or more checkmarks 506 (e.g., 506-1 through 506-6) to reflect successful completion of this step. For example, each check mark 506 may indicate successful completion of movement for a particular component of the robotic medical system 200 (e.g., check mark 506-1 indicates successful completion of movement of a robotic arm corresponding to the visual representation 504-1 and check mark 506-2 indicates successful completion of movement of a robotic arm corresponding to the visual representation 504-2, etc.). In contrast, the absence of a check mark, as shown in FIG. 25A, may indicate that movement of a particular component of the robotic medical system 200 has not been completed (e.g., the movement may be in progress) (e.g., the absence of check mark 506-1 in FIG. 25A indicates that movement of a robotic arm corresponding to the visual representation 504-1 has not been completed and the absence of check mark 506-2 in FIG. 25A indicates that movement of a robotic arm corresponding to the visual representation 504-2 has not been completed).

### ii. 3-D Renderings that Reflect Robotic State Changes Arising from Manual Interaction

In some embodiments, the real-time renderings are used to provide status updates based on manual interactions between a user and a portion of the robotic medical system. The real-time renderings can also be used to convey completion of manual actions such as docking the robotic arm(s) 210 to respective cannulas, and/or attaching instruments to the robotic arm(s) 210.

In some embodiments, in accordance with a determination that the robotic medical system 200 is executing a particular step of the workflow, the robotic medical system 200 adjusts a field of view (e.g., a viewpoint, a perspective, an angle, etc.) of a virtual camera to focus on a specific portion (e.g., a subset of robotic arms, a particular joint and/or link of a robotic arm, an advanced device manipulator (ADM), a tool driver, an instrument driver, or a robotic end effector, a tool or medical instrument that is attached to the robotic arm, etc.) of the robotic medical system. As a result, the robotic medical system 200 updates the 3-D rendering to display the specific portion of the robotic medical system from the adjusted field of view. In some embodiments, the robotic medical system stores (and/or updates) information indicating the adjusted field of view (e.g., orientation of the camera) (e.g., in the computer readable storage medium 382).

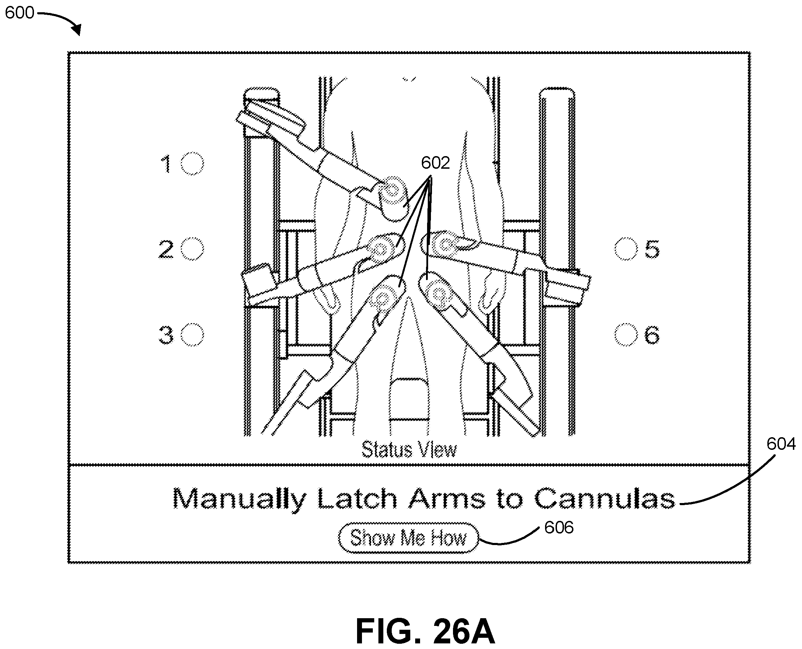

FIGS. 26A to 26D illustrate 3-D renderings that reflect real-time status of a robotic medical system arising from manual interactions, according to some embodiments.

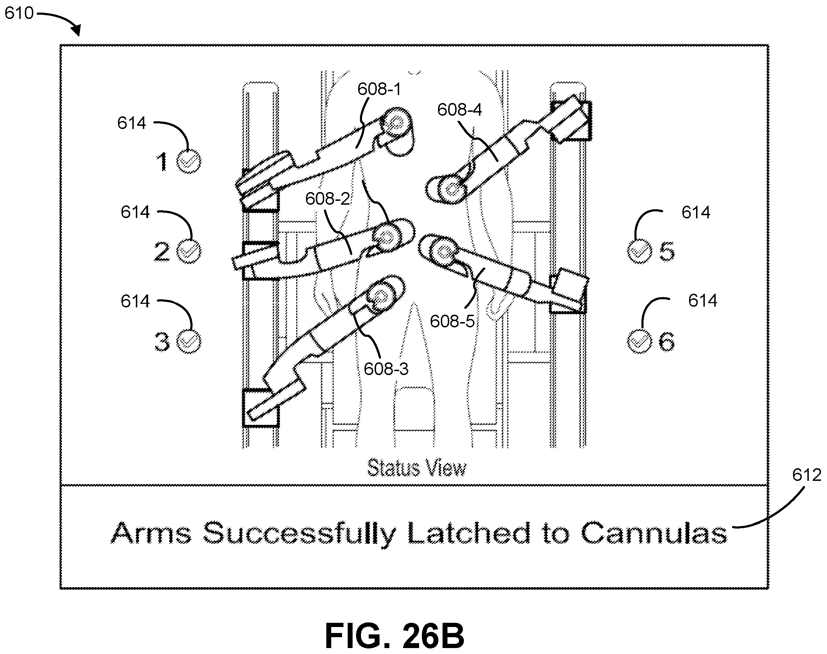

FIGS. 26A and 26B illustrate the process in which the robotic arms 210 are manually docked to their respective cannulas. In some embodiments, during the docking step, the robotic medical system 200 changes the camera view dynamically (e.g., from an isometric view to a plan view) to display a rendering 600 that focuses on visual representations 602 corresponding to the ADMs 308, as illustrated in FIG. 26A. In some embodiments, the rendering 600 is displayed with (or the rendering 600 includes) text 604 (e.g., "Manually Latch Arms to Cannulas") describing the manual action to be performed by a user. In some embodiments, the rendering 600 is displayed with (or the rendering 600 includes) an affordance 606 (e.g., "Help" / "Show Me How" button) that, when selected by the user (e.g., based on user interaction with a touch-sensitive display or any other input device), provides guidance (e.g., in the form of video, audio, and/or text, etc.) that guides the user through completion of the step.

FIG. 26B shows an updated rendering 610 that is displayed (e.g., automatically, in real time, etc.) upon successful completion of the manual docking step. In this example, the representations 608 (e.g., 608-1 through 608-5) in the rendering 610 reflect actual positions of the robotic arms 210, which typically move during the manual step of latching the robotic arms 210 to their respective cannulas. In some embodiments, representations 608 are displayed with a different color to reflect successful completion of the manual step. The updated rendering 610 can be displayed with (or include) text 612 and/or checkmark badges 614 to indicate successful completion of this manual step.

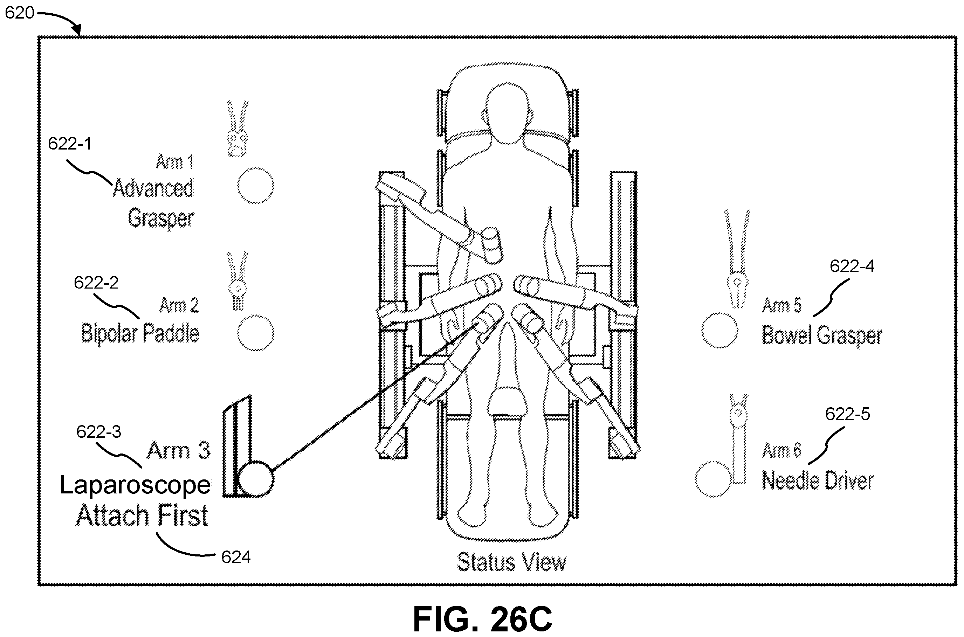

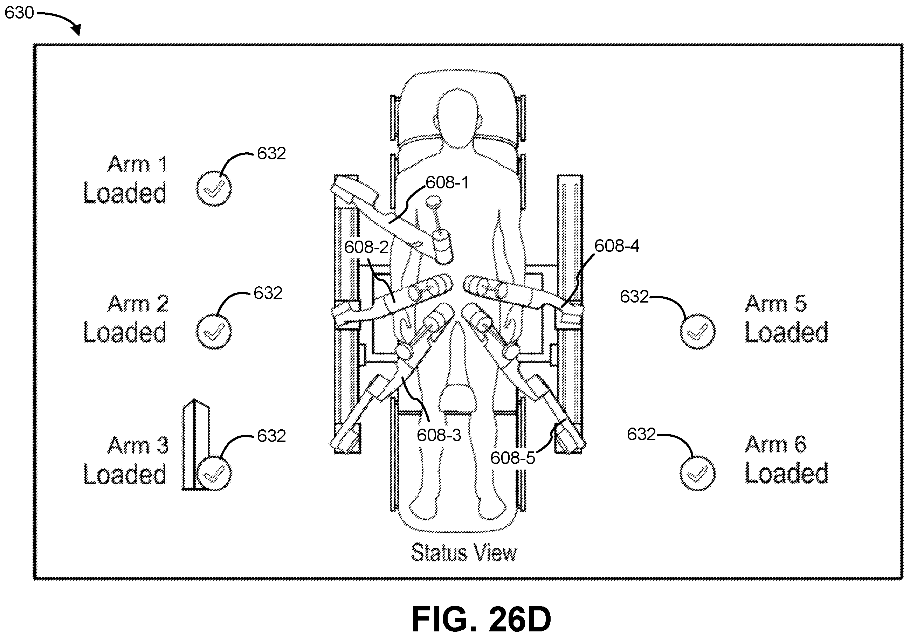

FIGS. 26C and 26D illustrate renderings that depict manual attachment of instruments (e.g., medical instruments or tools) to the robotic arms 210 in accordance with some embodiments. FIG. 26C shows a rendering 620 (e.g., an instrument model, a view, etc.) that includes identifications of instruments 622 (e.g., 622-1 through 622-5) to be attached to the robotic arms 210. In some embodiments, the rendering also includes, or indicates, an order 624 (e.g., a sequence, such as "Laparoscope - attach First") in which the instruments are to be attached. In some embodiments, each of the instruments includes a unique identifier that can be tracked via RFID tags. After an instrument is fully connected and latched to a robotic arm 210, the robotic medical system 200 (e.g., via the one or more processors) can identify the presence of the instrument on the robotic arm 210 as well as the instrument type to which it corresponds. FIG. 26D shows that upon successful completion of the manual step, the robotic medical system 200 displays an updated view 630 (e.g., an updated instrument model) in which each of the robotic arms that has been loaded with an instrument is displayed with a checkmark 632, indicating the successful completion of the manual step with the respective robotic arm.

### iii. 3-D Renderings to Show Error State

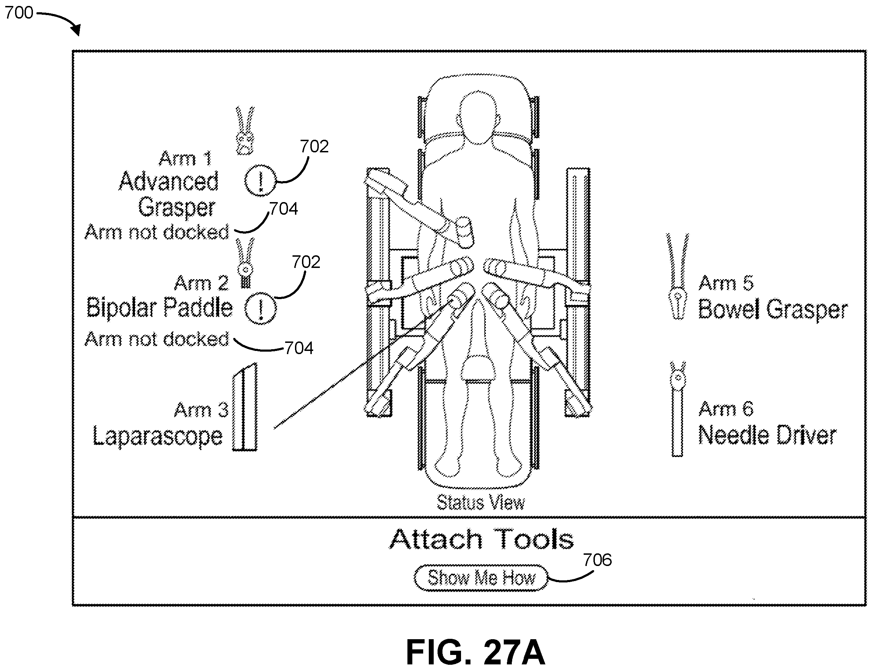

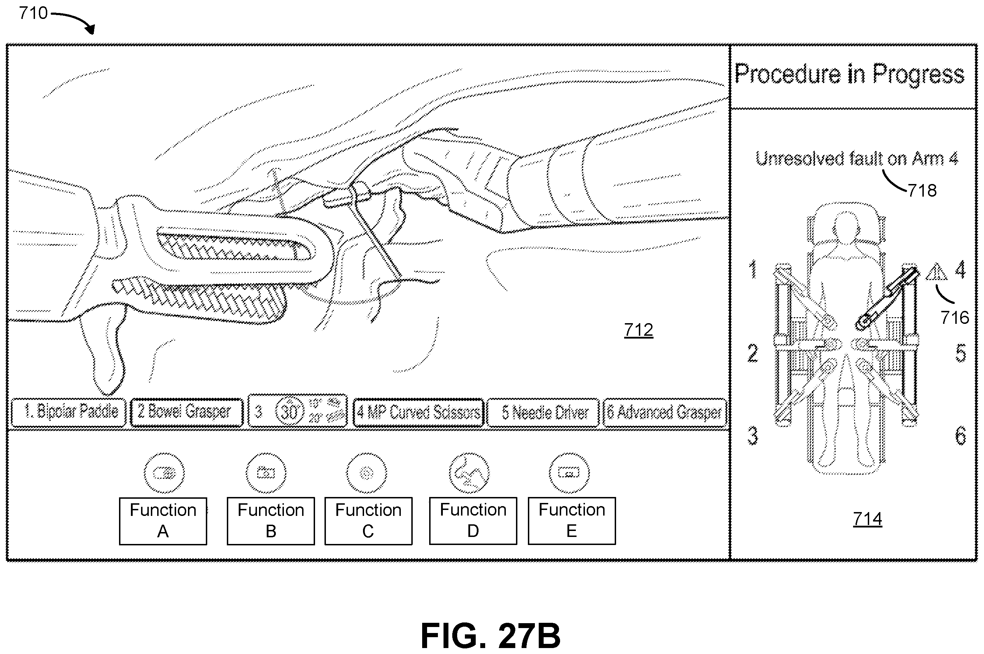

FIGS. 27A and 27B illustrate the use of 3-D renderings to communicate an error state of the robotic medical system 200 in accordance with some embodiments.

FIG. 27A depicts a 3-D rendering 700 corresponding to a workflow step of manual attachment of instruments (e.g., medical tools) to robotic arms. In some embodiments, in accordance with a determination that a user has not completed a workflow step (e.g., the user has skipped the workflow step), the robotic medical system 200 can display a rendering 700 with error information (e.g., the rendering 700 shows error state(s) 702 or error indicator, location(s) (e.g., identification of robotic arms) within the robotic medical system where the error(s) have been detected, and/or information 704 describing details of the error, such as a cause of the error). In some embodiments, the rendering 700 also includes a user-selectable affordance 706 (e.g., a button) that, when selected by the user, provides guidance (e.g., in the form of video, audio, and/or text, etc.) that guides the user through completion of the tool attachment step.

FIG. 27B illustrates a view 710 that is displayed by the robotic medical system 200 (e.g., on a user interface, a tower pendant, etc.) during a medical procedure (e.g., during an intra-operative stage), in accordance with some embodiments. In some embodiments, the view 710 corresponds to a live view 712 (e.g., captured by a camera that is attached to a robotic arm 210) of the medical procedure. In some embodiments, the view 710 includes a rendering 714 that depicts an intra-operative status of the robotic medical system 200 in-real time, to communicate faults, errors, and/or status during the procedure. In the example of FIG. 27B, the rendering 714 includes a warning symbol 716 next to robotic arm 4 and an accompanying description 718 indicating that there is an unresolved fault on robotic arm 4.

### iv. Supporting Graphics Overlaid on 3-D Renderings for Additional Guidance / Target Positions

In accordance with some embodiments of the present disclosure, graphics (e.g., graphical representations) can be generated and overlaid on the 3-D renderings to help guide a user to understand what needs to be done to the robotic medical system to complete a workflow. In some embodiments, the graphic can include a 0-D object such as a point, a 1-D object such as a line, and/or a 2-D object such as an area or a region of interest. In some embodiments, the graphic can represent a "target position" showing where the one or more robotic arms need to be moved to at the end of a workflow step.

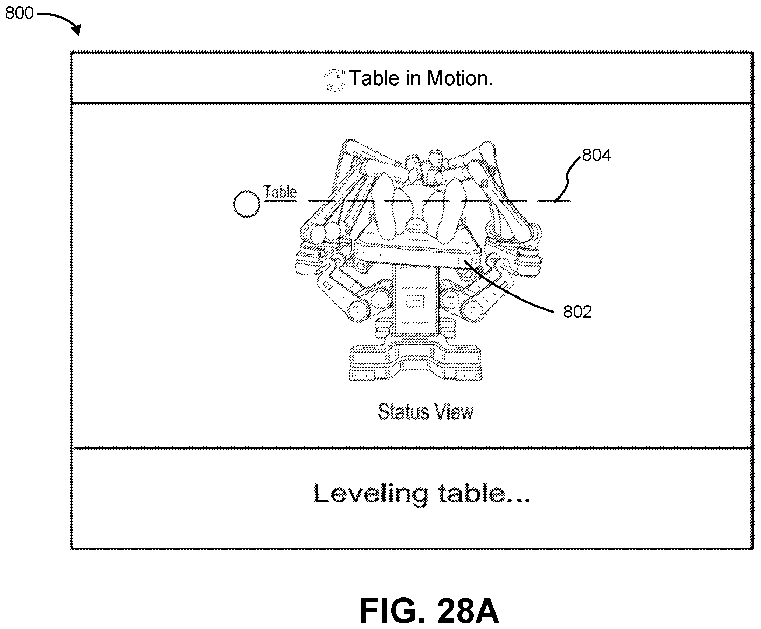

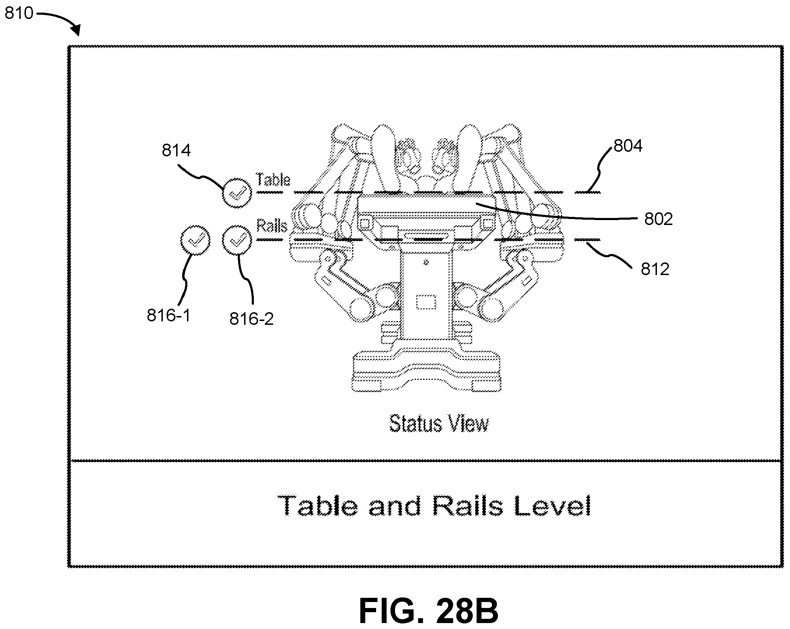

FIGS. 28A and 28B illustrate 3-D renderings depicting a real-time status of the robotic medical system 200 during an intra-operative (e.g., surgical) stage, where the patient support platform and/or arm supports are leveled out to a horizontal position from a tilted position (e.g., to facilitate surgical operations).

FIG. 28A shows a 3-D rendering 800 corresponding to a real-time status of the robotic medical system 200, where the patient support platform 202 (represented as element 802 in the rendering) is tilted (e.g., rolled) with respect to a longitudinal axis. Overlaid on the 3-D rendering is a guide line 804 (e.g., a graphic, a level line, a horizontal line, etc.) indicating a horizontal position of the patient support platform (e.g., table), to guide the user to complete the leveling step. For example, the user can look at a relative angle between the representation 802 and the guide line 804, and then try to adjust the patient support platform 202 (e.g., by holding a user interface control on the display, or activating a button on the robotic medical system 200) in the correct direction so that the representation 802 lines up with the guide line 804 (which corresponds to the patient support platform 202 becoming level (also called having a horizonal position)).

In some embodiments, while the patient support platform 202 is adjusted toward a horizontal position, the representation 804 is continuously updated to reflect the change in tilt angle of the patient support platform 202. In some embodiments, as the user adjusts the patient support platform 202, the guide line 804 remains stationary (e.g., fixed) while the representation 802 is continuously updated. In some embodiments, as the user adjusts the patient support platform 202, the guide line 804 remains level (e.g., horizontal) but changes in height (e.g., a vertical position) to match a height of the representation 802, which changes with the tilt angle change of the patient support platform 202.

FIG. 28B illustrates a 3-D rendering 810 that depicts completion of the step of leveling the patient support platform and arm supports (e.g., rails). FIG. 28B shows that when the step is completed, the rendering 810 is updated to include a checkmark 814 (e.g., a green checkmark, a checkmark badge etc.) adjacent to the guide line 804, indicating the completion of the leveling of the patient support platform. FIG. 28B also shows that the rendering 810 is updated to include checkmarks 816 adjacent to the guide line 812 corresponding to the arm supports (e.g., rails) to indicate completion of the leveling of the arm supports. In this example, there are two checkmarks 816, each corresponding to a respective arm support 220 (e.g., a left art support and a right arm support).

### v. Pre-Rendered Visuals to Convey Robotic Status

In some circumstances, a series of pre-rendered images (e.g., 2-D visuals or pre-rendered 3-D images) of the robotic medical system, including the patient support platform and/or robotic arms, can be used to convey robotic status/selections/completion/faults. The images of the robotic medical system will update upon completion of a movement to a new position. This may consume less computational power than generating the 3-D renderings in real time, which in turn allows presentation of the graphical representation of the robotic medical system in electronic components with limited graphical computation resources (e.g., on the pendant hardware).

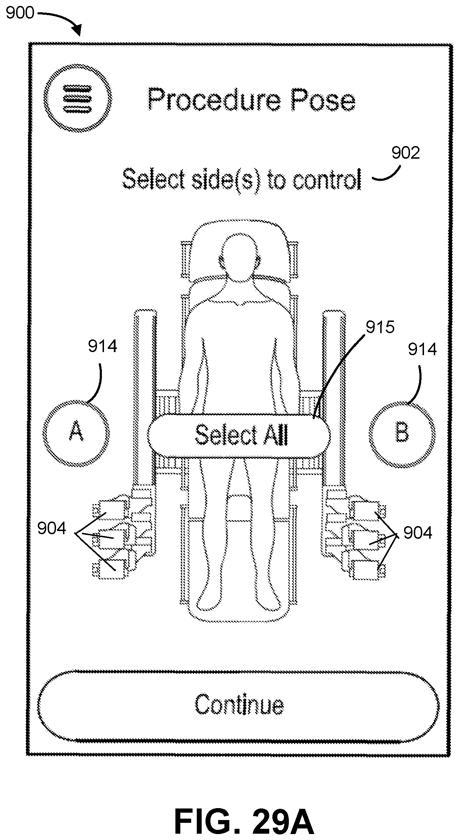

FIGS. 29A to 29C illustrate the use of pre-rendered images to convey a status of the robotic medical system in accordance with some embodiments.

FIG. 29A illustrates a pre-rendered 2-D image 900 of a procedure pose and a prompt 902 to the user to select side(s) of the robotic medical system that the user would like to control. In this example, a user selects the representations 904 of the robotic arms on both sides of the patient support platform in the image 900 (e.g., by tapping on the robotic arms on both sides of the patient support platform, tapping the labels 914 (e.g., "A" and "B"), or by tapping the label 915 (e.g., "Select All") in the image 900).

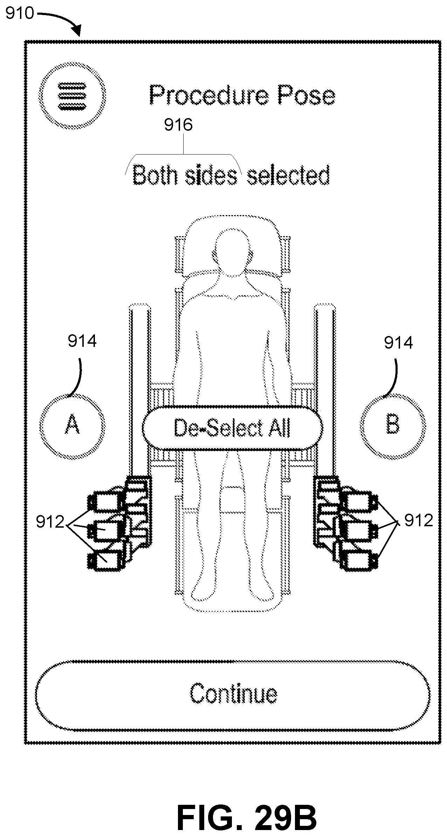

FIG. 29B illustrates that, in accordance with the user selection, the robotic medical system 200 displays a pre-rendered image 910, in which representations 912 of the robotic arms are rendered differently (e.g., with a different color, different line thickness, highlighted, visually emphasized, etc.) from the representations 904 in the image 900, to indicate (and reflect) the user selection. In some embodiments, the labels 914 (e.g., "A" and "B") and/or the text 916 (e.g., "Both Sides") in the image 910 are rendered differently (e.g., visually emphasized using, for example, color, font, size, and/or styles) from the labels and/or text in the image 900, to reflect user selection of the robotic arms and/or to movement of the robotic arms 210 in accordance with the user selection.

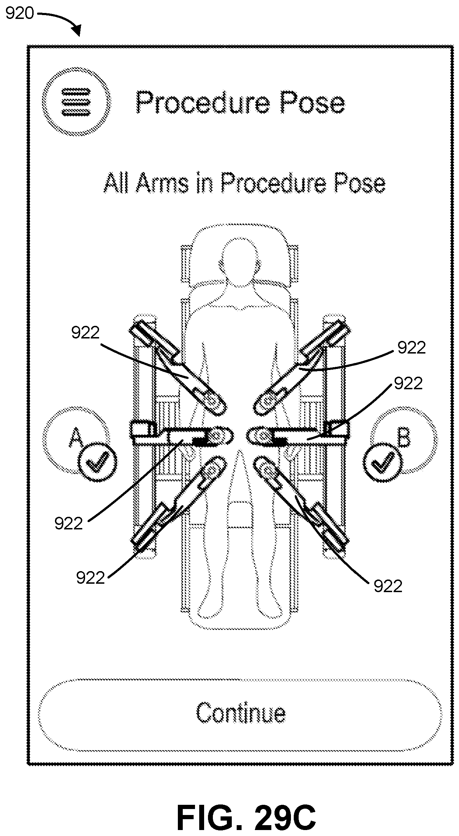

FIG. 29C illustrates a pre-rendered image 920 that is displayed after the robotic medical system 200 has moved the robotic arms 210 to the procedure pose. In the image 920, the representations 922 of the robotic arms are rendered differently (e.g., with a different color, different line thickness, highlighted, visually emphasized, etc.) from other portions of the robotic medical system depicted in the image (and/or from the representations 912 in the image 910, and/or the representations 904 in the image 900), to visually emphasize the new position of the robotic arms.

In some embodiments, the pre-rendered images (such as the images 900, 910, and 920) are stored locally on the robotic medical system 200 and/or remotely on a computer system that is communicatively connected with the robotic medical system 200. The images are retrieved by the robotic medical system 200 on-the-fly in accordance with a determination of the real-time status of the robotic medical system. Using the transition from FIG. 29A to FIG. 29B as an example, the robotic medical system 200 can detect user selection of the representations of the robotic arms on both sides of the patient support platform. In accordance with the detection, the robotic medical system 200 retrieves the pre-rendered image 910, which includes the representations 912, the labels 914, and/or the text 916. When the movement of the robotic arms 210 is completed, the robotic medical system 200 retrieves (e.g., selects) the image 920, which is an image corresponding to completion of movement of the robotic arms on both sides of the patient support platform, and displays the image 920.

### D. Exemplary Processes for Real-time 3-D Robotic Status

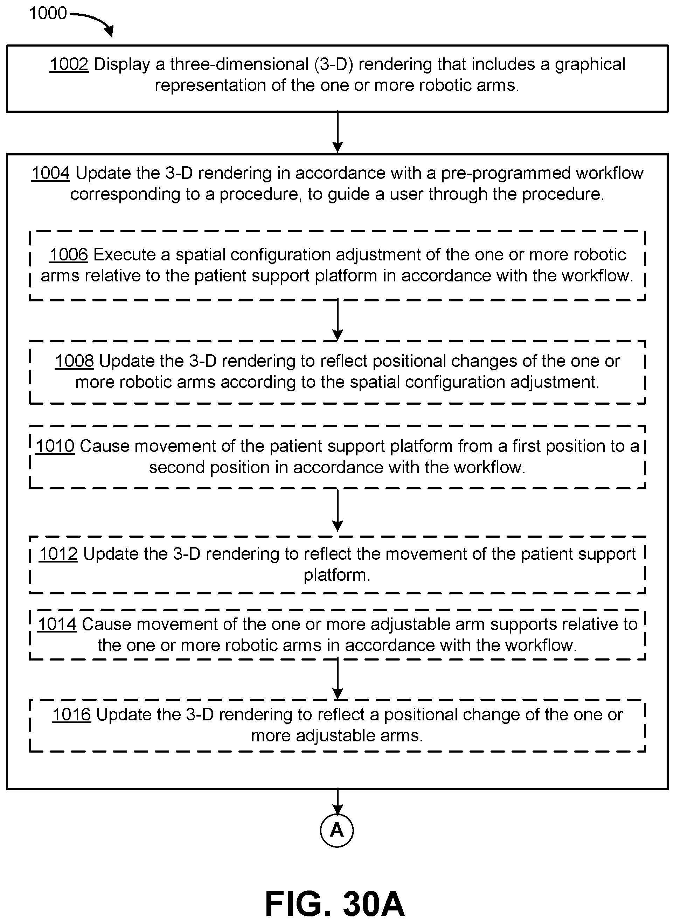

FIGS. 30A to 30E illustrate a flowchart diagram for a method 1000 performed by one or more processors (e.g., one or more processors 380) of a robotic medical system (e.g., the robotic medical system 200 as illustrated in FIGS 21 and 22 or a robotic surgery platform, etc.), in accordance with some embodiments. The robotic medical system comprises one or more processors and memory that stores instructions for execution by the one or more processors.

The robotic medical system includes one or more robotic arms (e.g., the robotic arms 210 in FIGS. 21, 22, 23A, 23B, and 23C). In some embodiments, the one or more robotic arms are used to perform a medical procedure. Each of the robotic arms is capable of holding a respective instrument. In some embodiments, the one or more robotic arms are coupled to an adjustable arm support (e.g., adjustable arm support or bar 220).

The robotic medical system includes one or more displays (e.g., an interactive display, a touchscreen display, a display that includes a user interface etc.). In some embodiments, the one or more displays include one or more interactive viewers for displaying a 3-D rendering of the one or more robotic arms. In some embodiments, the one or more displays are located on a tower viewer, surgeon viewer, a bed/tower pendant of the robotic medical system. In some embodiments, the one or more displays are located on any pendant (e.g., a remote pendant) that is used as part of the robotic medical system.

The robotic medical system displays (1002) (e.g., generates and displays) (e.g., on the one or more displays) a three-dimensional (3-D) rendering that includes a graphical representation of the one or more robotic arms.

The robotic medical system updates (1004) (e.g., changes, refreshes, etc.) the 3-D rendering (e.g., automatically, in real time, once every 10 seconds, 30 seconds, 60 seconds, etc., without user without user intervention, etc.) in accordance with a pre-programmed (e.g., predefined) workflow corresponding to a procedure (e.g., a medical procedure), to guide a user (e.g., a surgeon, a surgeon assistant, patient side staff, etc.) through the procedure (e.g., medical procedure, one or more stages of the procedure, etc.).

In some embodiments, updating the 3-D rendering includes rotating the 3-D rendering in space (e.g., about a vertical axis), thus presenting a view of the robotic medical system at various angles (e.g., viewpoints) (e.g., updating the 3-D rendering includes presenting a first view of the robotic medical system from a first viewpoint and subsequently replacing the first view of the robotic medical system with a second view of the robotic medical system from a second view point that is distinct from the first view point). In some embodiments, updating the 3-D rendering includes rotating the 3-D rendering and pausing at a scene (e.g., a field of view, a viewpoint, etc.) that includes specific elements to accomplish a step of the workflow process. In some embodiments, updating the 3-D rendering includes varying (e.g., changing, adjusting, etc.) a field of view (e.g., an angle) of the robotic medical system (e.g., a view as seen by a virtual camera of the robotic medical system).

In some embodiments, the 3-D rendering includes an isometric view of the robotic medical system. In some embodiments, updating the 3-D rendering includes panning (e.g., dynamically, automatically, in real-time) the 3-D rendering from the isometric view to a plan view, to focus on specific elements of the robotic arms (e.g., a particular joint, a particular link, a device manipulator, a tool that is attached onto the robotic arm, etc.), or a portion of the adjustable arm support and/or patient support platform.

In some embodiments, the robotic medical system includes a patient support platform (e.g., patient support platform 202, such as table, bed, etc.). The robotic medical system executes (1006) a spatial configuration adjustment of the one or more robotic arms (e.g., an adjustment or a change in positions and/or orientations of joints and/or links of the robotic arms) relative to the patient support platform in accordance with the workflow. The robotic medical system updates (1008) the 3-D rendering (e.g., automatically, in real-time, etc.) to reflect positional changes of the one or more robotic arms according to the spatial configuration adjustment (e.g., to accurately reflect the actual system state of the robotic arms and/or underlying bar relative to the patient support platform).

In some embodiments, the robotic medical system causes (1010) (e.g., executes) movement of the patient support platform from a first position to a second position in accordance with the workflow. For example, the movement can include a translational, rotational, and/or tilt movement. The robotic medical system updates (1012) the 3-D rendering (e.g., automatically, in real-time, etc.) to reflect the movement of the patient support platform.

In some embodiments, the robotic medical system includes one or more adjustable arm supports (e.g., arm supports 220, underlying bars, etc.) that are movably coupled to the one or more robotic arms. Each of the adjustable arm supports is capable of tilt, rotation, and/or translation. The robotic medical system causes (1014) (e.g., executes) movement of the one or more adjustable arm supports relative to the one or more robotic arms in accordance with the workflow. For example, the movement can include a translational, rotation, and/or tilt movement), The robotic medical system updates (1016) the 3-D rendering (e.g., automatically, in real-time, etc.) to reflect a positional change of the one or more adjustable arms (e.g., a positional change relative to the one or more robotic arms).

In some embodiments, the robotic medical system includes a plurality of robotic arms that are movably coupled to one adjustable arm support.

In some embodiments, the robotic medical system includes a plurality of robotic arms and at least two adjustable arm supports. Each of the robotic arms is movably coupled to a respective arm supports.

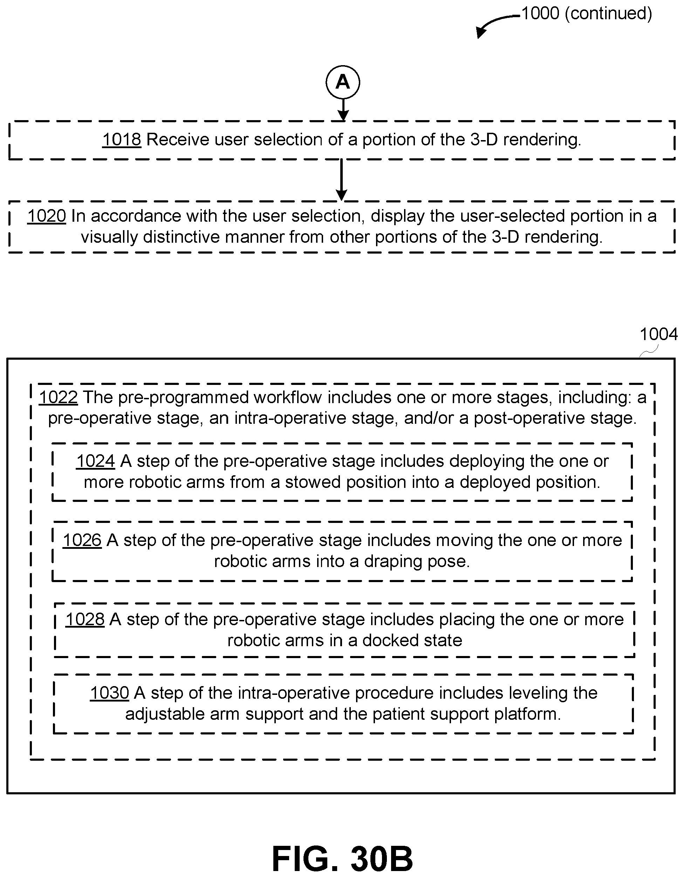

In some embodiments, the robotic medical system receives (1018, FIG. 30B) (e.g., via the one or more displays) user selection of a portion of the 3-D rendering. For example, as discussed with respect to FIG. 24A, the user selection can include direct user selection and/or indirect user selection. In accordance with the user selection, the robotic medical system displays (1020) the user-selected portion in a visually distinctive manner (e.g., highlighted, shaded, in a different color, with visual effects such as pulsing and/or other visual emphasis, in a different font size and/or font color etc.) from other portions of the 3-D rendering.

In some embodiments, the pre-programmed workflow includes (1022) one or more stages, including: a pre-operative stage (e.g., a setup stage), an intra-operative stage (e.g., an intra-operation stage), and/or a post-operative stage (e.g., a teardown stage).

In some embodiments, the updated 3-D rendering communicates the status of the robotic medical system, such as poses (e.g., positions and/or orientations) of arms and/or arm support(s), instruments attached thereto, warning / error messages, etc., during the various stages of the medical procedure.

In some embodiments, a step of the pre-operative stage includes deploying (1024) the one or more robotic arms from a stowed position into a deployed position.

In some embodiments, a step of the pre-operative stage includes moving (1026) the one or more robotic arms into a draping pose.

In some embodiments, a step of the pre-operative stage includes placing (1028) the one or more robotic arms in a docked state. For example, in some embodiments, the robotic arms are docked to one or more cannulas whereby they can be subsequently coupled to one or more corresponding instruments (e.g., robotically-controlled medical instruments or tools, such as an endoscope, a laparoscope, and/or any another instruments that may be used during surgery). In some embodiments, with the robotic arms docked to the corresponding cannulas, the robotic system determines a remote center of motion (RCM) and/or a port of entry with respect to each of the robotic arms.)

In some embodiments, the robotic medical system includes an adjustable arm support (e.g., an underlying bar) that is movably coupled to the one or more robotic arms. The robotic medical system includes a patient support platform (e.g., a bed, table, etc.). A step of the intra-operative procedure includes leveling (1030) the adjustable arm support and the patient support platform (e.g., moving the adjustable arm support and the patient support platform to a horizonal position, an untilted position, etc.). In some embodiments, leveling of the adjustable arm support and/or patient support platform occurs during teardown from a procedure (e.g., as a step in the post-operative stage).

In some embodiments, each of the one or more stages of the pre-programmed workflow includes one or more respective (e.g., distinct) steps. For example, as discussed above, the setup stage can include steps such as: deploying the robotic arms and arm supports from a stowed state to a deployed state, draping the robotic arms and arm supports, and docking the robotic arms. The post-operative (e.g., teardown) stage can include steps such as removing tools that are attached to the robotic arms, undocking the robotic arms, and leveling the patient support platform and arm supports (e.g., bars, rails, etc.).

In some embodiments, the robotic medical system updates the 3-D rendering in accordance with a determination (e.g., identification) of a step that is being executed by the robotic medical system.

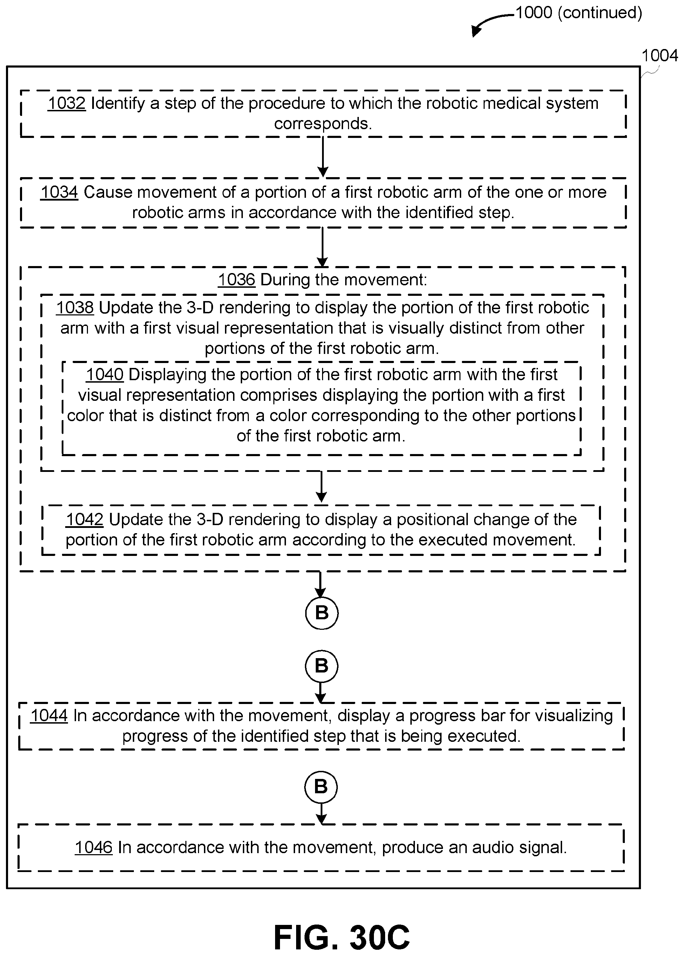

In some embodiments, the robotic medical system identifies (1032, FIG. 30C) (e.g., determines) a step of the (medical) procedure to which the robotic medical system corresponds. The robotic medical system causes (1034) (e.g., executes) movement (e.g., robotic movement, automatic movement, movement without user intervention, etc.) of a portion (e.g., a link, a joint, etc.) of a first robotic arm of the one or more robotic arms in accordance with the identified step. During (1036) the movement, the robotic medical system updates (1038) the 3-D rendering (e.g., generates and displays an updated 3-D rendering) (e.g., continuously, periodically, such as once every 5 second, 10 seconds, etc.) to display (e.g., show, illustrate, etc.) the portion of the first robotic arm with a first visual representation that is visually distinct from other portions of the first robotic arm (e.g., and/or from the other robotic arms). For example, the robotic medical system can display the portion of the first robotic arm with a first visual representation that includes highlighting, shading, a different color, and/or visual effects such as pulsing and/or other visual emphasis, etc. In some embodiments, displaying the portion of the first robotic arm with the first visual representation includes displaying (1040) the portion with a first color that is distinct from a color corresponding to the other portions of the first robotic arm (e.g., and from the other robotic arms). During the movement, the robotic medical system updates (1042) the 3-D rendering to display (e.g., show, illustrate, reflect, etc.) a positional change of the portion of the first robotic arm according to the movement.

For example, in some embodiments, the robotic medical system continuously determines (e.g., every second, every 3 seconds, every 10 seconds, etc.) the positions of joints and/or links of the robotic arm and/or its underlying bar while executing movement of the robotic arm, and generates and displays a 3-D rendering in accordance with the determination.

In some embodiments, where computational power is limited (e.g., on the tower pendant hardware), the robotic medical system can use a series of layered pre-rendered 2-D visuals of the patient support platform and robotic arms to convey robotic status /selections / completion. For example, the robotic medical system can store (e.g., either locally or remotely) 2-D images of the robotic system and update the images upon completion of a movement to a new position.

In some embodiments, in accordance with the executed movement, the robotic medical system displays (1044) (e.g., concurrently, simultaneously with the 3-D rendering) a progress bar (e.g., status bar, a progress indicator, a graphical control element, etc.) for visualizing progress of the identified step that is being executed, as illustrated in FIG. 24B.

In some embodiments, in accordance with the movement, the robotic medical system produces (1046) (e.g., activates, generates and outputs, etc.) an audio signal (e.g., an audio output) (e.g., via the tower viewer, pendant, etc.). In some embodiments, the audio output is synchronous with the executed movements.

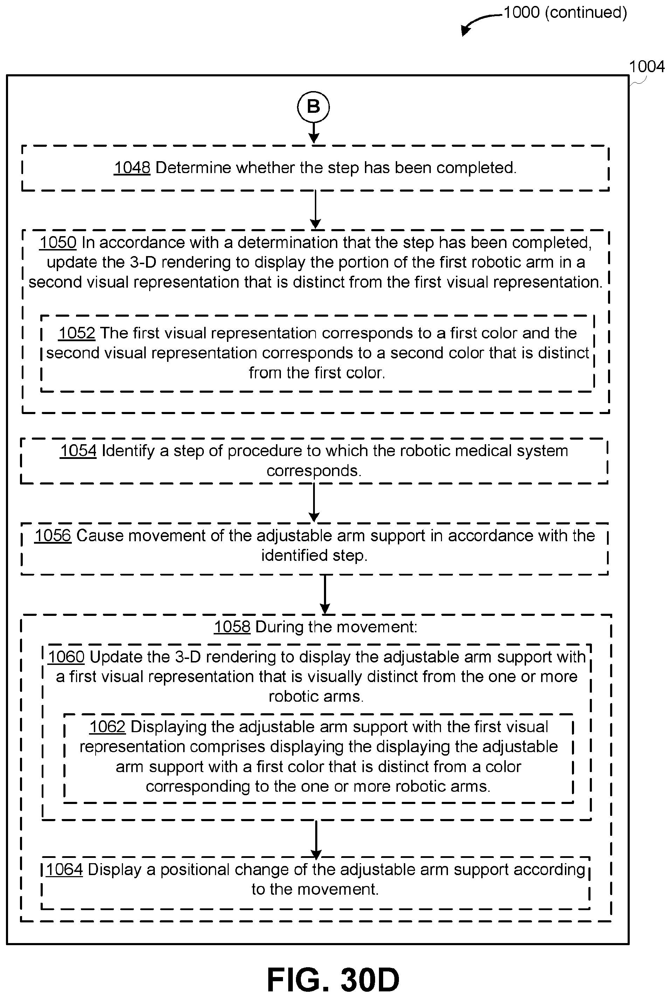

In some embodiments, the robotic medical system determines (1048, FIG. 30D) (e.g., detects) (e.g., using one or more sensors and/or encoders) whether the step has been completed, In accordance with a determination that the step has been completed, the robotic medical system updates (1050) the 3-D rendering to display the portion of the first robotic arm in a second visual representation that is distinct from the first visual representation. For example, in some embodiments, the second visual representation can have a different color, can undo (e.g., remove) a highlighted, shaded, and/or emphasis that was found in the first visual representation. In some embodiments, the second visual representation includes adding checkmark badge(s) to reflect completion of the step.

In some embodiments, the first visual representation corresponds (1052) to a first color and the second visual representation corresponds to a second color that is distinct from the first color.

In some embodiments, the robotic medical system stores the start and end positions for each of the joints of the robotic arms, for each of the steps of the workflow. The robotic medical system determines (e.g., using sensors and encoders located on the arms) an actual position of the joints, and compares the actual positions with the stored positions, to determine whether a step has been completed.

In some embodiments, after determining that a step of the workflow process has been completed, the robotic system proceeds (e.g., automatically) to the subsequent step of the workflow process. In accordance with the subsequent step, the virtual camera can automatically pan to a scene (e.g., having an angle, a field of view, a viewpoint, etc.) to focus on specific element(s) that are important for the subsequent step.

In some embodiments, the robotic medical system includes an interactive display. After a step of the workflow process has been completed, a user can select a user-selectable option (e.g., an icon) on the interactive display, which instructs (e.g., causes) the robotic medical system to proceed to the next step of the workflow.

Referring again to FIG. 30D, in some embodiments, the robotic medical system includes an adjustable arm support that is movably coupled to the one or more robotic arms are coupled. The robotic medical system identifies (1054) a step of procedure to which the robotic medical system corresponds. The robotic medical system causes (1056) (e.g., executes, activates) movement of the adjustable arm support in accordance with the identified step. During (1058) the movement, the robotic medical system updates (1060) (e.g., continuously, periodically, etc.) the 3-D rendering to display the adjustable arm support with a first visual representation that is visually distinct from the one or more robotic arms.

In some embodiments, displaying the adjustable arm support with the first visual representation comprises displaying (1062) the adjustable arm support with a first color that is distinct from a color corresponding to the one or more robotic arms.

In some embodiments, the robotic medical system displays (1064) a positional change of the adjustable arm support (e.g.., relative to the one or more robotic arms) according to the executed movement.

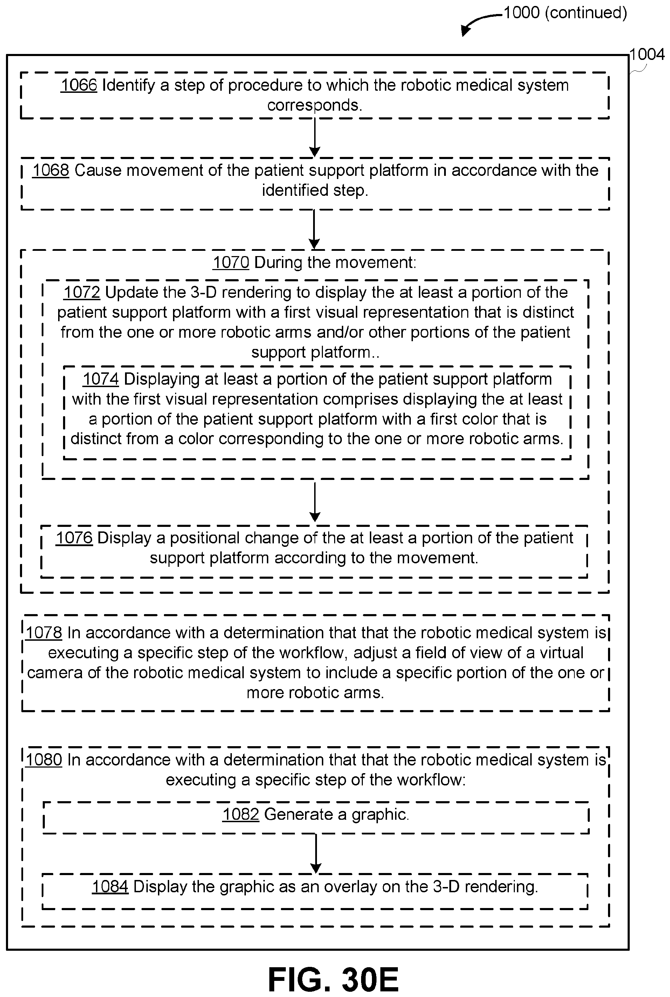

With reference to FIG. 30E, in some embodiments, the robotic medical system includes a patient support. The robotic medical system identifies (1066) a step of procedure to which the robotic medical system corresponds. The robotic medical system causes (1068) (e.g., executes, activates, etc.) movement of at least a portion of the patient support platform. During (1070) the movement, the robotic medical system updates (1072) the 3-D rendering to display the at least a portion of the patient support platform with a first visual representation that from the one or more robotic arms and/or other portions of the patient support platform.

In some embodiments, displaying the at least a portion of the patient support platform with the first visual representation displaying (1074) the adjustable arm support with a first color that is distinct from a color corresponding to the one or more robotic arms.

In some embodiments, the robotic medical system displays (1076) a positional change of the portion of the at least a portion of the patient support platform according to the executed movement.

In some embodiments, in accordance with a determination that the robotic medical system is executing a specific step of the workflow, the robotic medical system adjusts (1078) a field of view (e.g., a viewpoint, a perspective, an angle, etc.) of a virtual camera of the robotic medical system (e.g., the field of view of the virtual camera corresponds to a view of the robotic medical system as seen by a user on the display) to include (e.g., to focus on) a specific portion of the one or more robotic arms (e.g., a particular joint, a particular link, an advanced device manipulator (ADM), a tool driver, an instrument driver, or a robotic end effector, a tool or medical instrument that is attached to the robotic arm, etc.).

In some embodiments, a specific step of the workflow can include placing the robotic arms in a docked state, attaching one or more tools on the robotic arms, optimizing a pose (e.g., position and/or orientation) of the robotic arms, or underlying bar, and/or a step that requires manual actions by a user, etc. As an example, the specific step can be a docking step in which the robotic arms are placed in a docked state. During docking, the virtual camera changes dynamically to focus on the ADMs. The robotic arms of the 3-D rendering (e.g., model) will update position and state from white to green with a checkmark badge to reflect successful completion of this manual step.

In some embodiments, in accordance with a determination (1080) that the robotic medical system is executing a specific step of the workflow, the robotic medical system generates (1082) a graphic (e.g., a visual indicator, a visual marker, that is distinct from the 3-D rendering). The robotic medical system displays (1084) the graphic as an overlay (e.g., on top of) on the 3-D rendering. For example, as discussed in FIGS. 28A and 28B, in some embodiments, the graphic can include a 0-D object such as a point, a 1-D object such as a line, and/or a 2-D object such as a region of interest. In some embodiments, the graphic can represent a "target position" showing where the one or more robotic arms need to be moved to at the end of a step.

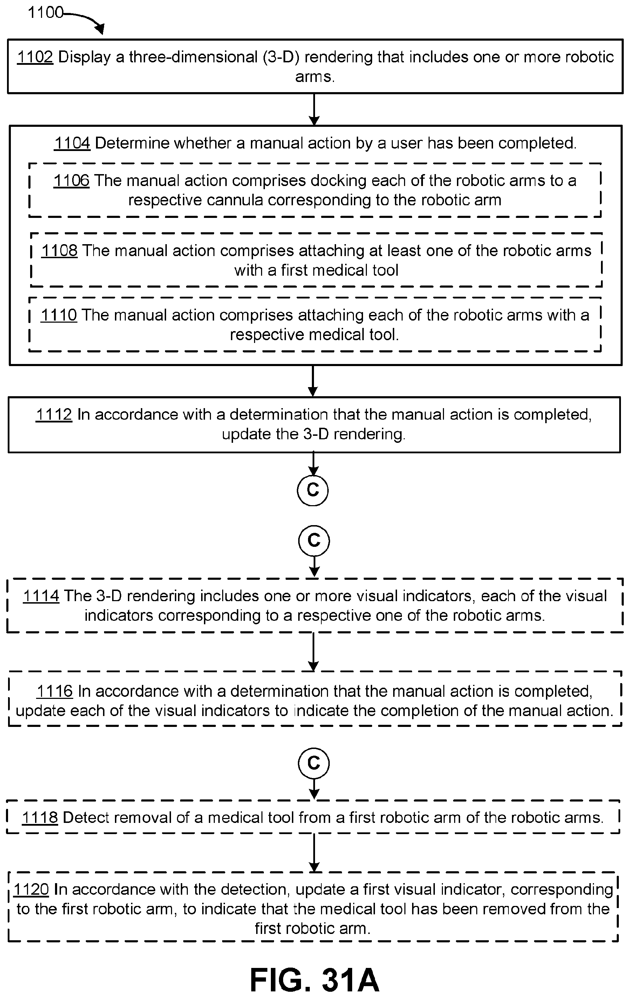

FIGS. 31A to 31C illustrate a flowchart diagram for a method 1100 performed by one or more processors of a robotic medical system (e.g., the robotic medical system 200 as illustrated in FIGS. 21 and 22, or a robotic surgery platform, etc.), in accordance with some embodiments. The robotic medical system comprises one or more processors and memory that stores instructions for execution by the one or more processors.

The robotic medical system includes one or more robotic arms (e.g., the robotic arms 210 in FIGS. 21, 22, 23A, 23B, 23C, 24A, 24B, 24C, 26A, 26B, 26C, and 26D). In some embodiments, the one or more robotic arms are used to perform a medical procedure. Each of the robotic arms is capable of holding a respective instrument. In some embodiments, the one or more robotic arms are coupled to an adjustable arm support (e.g., adjustable arm support or bar 220).

The robotic medical system includes one or more displays (e.g., an interactive display, a touchscreen display, a display that includes a user interface etc.) In some embodiments, the one or more displays include one or more interactive viewers for displaying a 3-D rendering of the one or more robotic arms. In some embodiments, the one or more displays are located on a tower viewer, surgeon viewer, a bed/tower pendant of the robotic medical system. In some embodiments, the one or more displays are located on any pendant (e.g., a remote pendant) that is used as part of the robotic medical system.

The robotic medical system displays (1102) (e.g., on the one or more displays) a three-dimensional (3-D) rendering that includes the one or more robotic arms.

The robotic medical system determines (1104) (e.g., detects) (e.g., automatically, in real time, etc.) whether a manual action by a user has been completed.

In some embodiments, the manual action corresponds to a step of a workflow for a medical procedure.

In some embodiments, the workflow includes one or more stages, including: a pre-operative stage (e.g., a setup stage), an intra-operative stage (e.g., an intra-operation stage), and/or a post-operative stage (e.g., a teardown stage).

In some embodiments, the manual action comprises docking (1106) each of the robotic arms to a respective cannula corresponding to the robotic arm.

In some embodiments, an action that can happen alongside with docking the robotic arms is to "stow away" one or more robotic arms that were deployed but are not necessary for the procedure (i.e., arms that are not docked but were deployed). If robotic arm(s) need to be stowed, they would be manually "twisted and tucked" into a position that that is least disruptive to bedside users during the procedure. In some embodiments, updating the 3-D rendering includes updating the 3-D rendering to reflect a respective position of one or more robotic arms that have been stowed away, or updating the 3-D rendering to exclude robotic arm(s) that have been stowed away.

In some embodiments, the manual action comprises attaching (1108) at least one of the robotic arms with a first medical tool (e.g., medical instrument). In some embodiments, the first medical tool (e.g., or each of the medical tools that is attached to a respective robotic arm) includes a unique identifier that can be tracked via RFID tags. When the first medical tool is fully connected and latched to the robotic arm, the one or more processors can identify the presence of the first medical tool and the tool type corresponding to the first medical tool.

In some embodiments, the manual action comprises attaching (1110) each of the robotic arms with a respective medical tool.

In some embodiments, in accordance with a determination that the manual action is completed, the robotic medical system updates (1112) (e.g., changes, refreshes, etc.) (e.g., automatically, in real time, without user interaction, without user intervention, etc.) the 3-D rendering.

In some embodiments, the 3-D rendering includes (1114) one or more visual indicators, each of the visual indicators corresponding to a respective one of the robotic arms. For example, a visual indicator can indicate an instrument to be attached to the respective arm, a sequence in which tools are to be attached to the robotic arms, a status of the robotic arm (e.g., whether the robotic arm is being deployed, has been docked, etc.). In accordance with a determination that the manual action is completed, the robotic medical system updates (1116) (e.g., in real time, automatically, without user intervention, etc.) each of the visual indicators to indicate the completion of the manual action.

In some embodiments, updating each of the visual indicators includes adding a checkmark (e.g., having a predefined color) to each of the visual indicators, to indicate completion of the manual action. In some embodiments, updating each of the visual indicators includes updating text corresponding to each of the visual indicators to indicate a status of the robotic arm (e.g., whether the robotic arm is docked, or whether an instrument is loaded onto the robotic arm, etc.)

In some embodiments, the manual action comprises attaching each of the robotic arms with a respective medical tool. The robotic medical system detects (1118) (e.g., determine) (e.g., automatically, in real time, without user intervention, etc.) removal of a medical tool (e.g., manually removed, un-attached, etc.) from a first robotic arm of the robotic arms. In accordance with the detection, the robotic medical system updates (1120) a first visual indicator corresponding to the first robotic arm (e.g., by removing the checkmark corresponding to the robotic arm), to indicate that the medical tool has been removed from the first robotic arm.

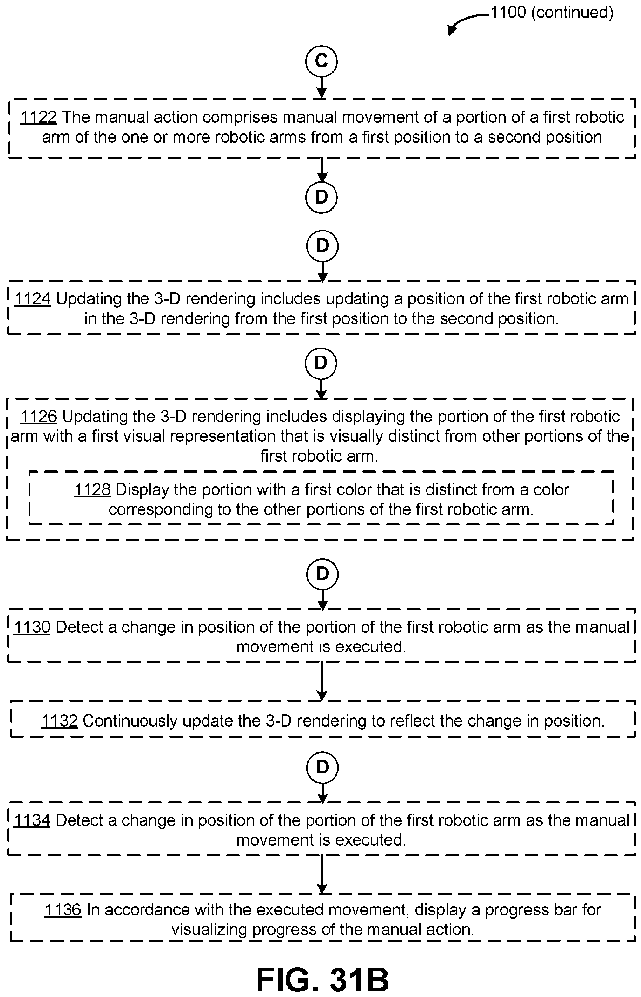

In some embodiments, the manual action comprises manual movement (1122, FIG. 31B) of a portion of a first robotic arm (e.g., one or more joints and/or links of the first robotic arm) of the one or more robotic arms from a first position to a second position.

In some embodiments, updating the 3-D rendering includes updating (1124) a position of the first robotic arm from in the 3-D rendering from the first position to the second position.

In some embodiments, the robotic medical system stores positional information (e.g., configuration information) of the robotic arms for each of the workflow steps. The robotic medical system may determine a current workflow step of the robotic medical system or receive user input that the robotic medical system is at a particular workflow step. As an example, in accordance with a determination or user input that the robotic medical system is at an arm docking step of the workflow, the system checks for completion of the cannula being attached to the robotic arm. When the system detects that the manual action has been completed, the checkbox next to the arms will turn green once the system detects that the step has been completed.

In some embodiments, updating the 3-D rendering includes displaying (1126) the portion of the first robotic arm with a first visual representation that is visually distinct from other portions of the first robotic arm.

In some embodiments, displaying the portion of the first robotic arm with the first visual representation includes displaying (1128) the portion with a first color that is distinct from a color corresponding to the other portions of the first robotic arm.

In some embodiments, the robotic medical system detects (1130) a change in position of the portion of the first robotic arm as the manual movement is executed. The robotic medical system continuously updates (1132) the 3-D rendering to reflect the change in position.

In some embodiments, the robotic medical system detects (1134) a change in position of the portion of the first robotic arm as the manual movement is executed. In accordance with the executed movement, the robotic medical system displays (1136) (e.g., concurrently, simultaneously with the 3-D rendering) a progress bar (e.g., status bar, a progress indicator, a graphical control element, etc.) for visualizing progress of the manual action.

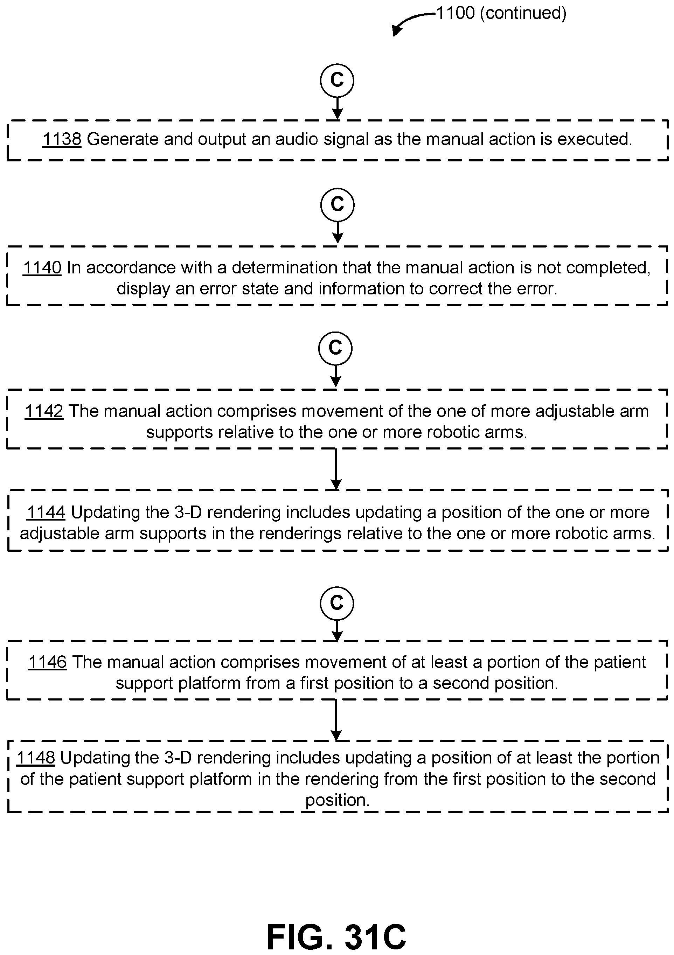

In some embodiments, the robotic medical system generates (1138, FIG. 31C) and outputs an audio signal as the manual action is executed.

In some embodiments, in accordance with a determination that the manual action is not completed, the robotic medical system displays (1140) an error state and information to correct the error. For example, in accordance with a determination that that a user has skipped a workflow step before successful completion, the robotic medical system can display a 3-D rendering to show an error state, a location where the error has occurred, and/or information on what went wrong.

In some embodiments, the robotic medical system includes one of more adjustable arm supports that are moveably coupled to the one or more robotic arms. The 3-D rendering includes renderings (e.g., representations, graphics, etc.) of the one or more adjustable arm supports.

In some embodiments, the manual action comprises movement (1142) of the one of more adjustable arm supports relative to the one or more robotic arms. Updating the 3-D rendering includes updating (1144) positions of the one or more adjustable arm supports in the renderings relative to the one or more robotic arms.

In some embodiments, the robotic medical system includes a patient support platform. The 3-D rendering includes a rendering of the patient support platform.

In some embodiments, the manual action comprises movement (1146) of at least a portion of the patient support platform from a first position to a second position. Updating the 3-D rendering includes updating (1148) a position of at least the portion of the patient support platform in the rendering from the first position to the second position.

### 3. Implementing Systems and Terminology.

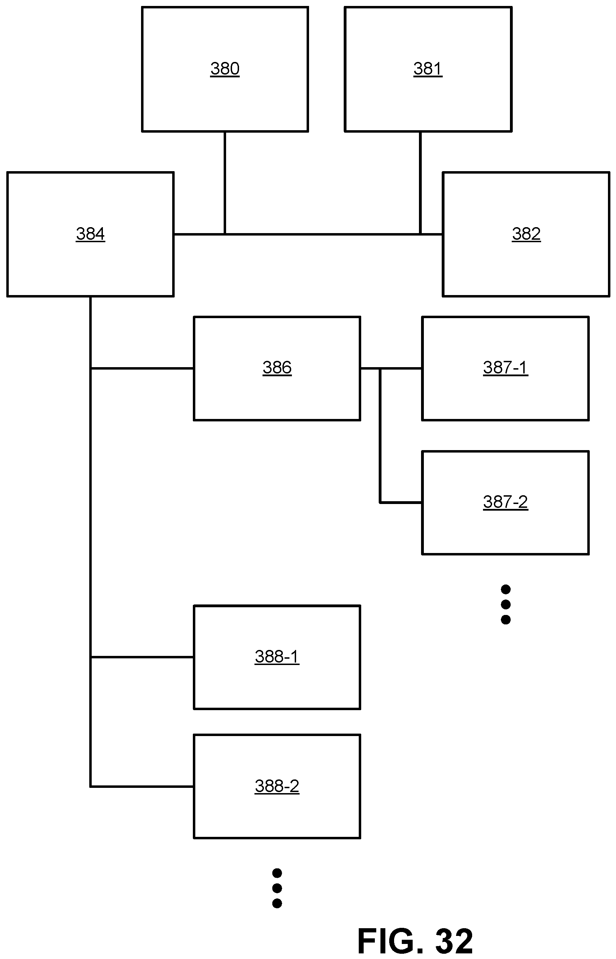

FIG. 32 is a schematic diagram illustrating electronic components of a robotic medical system in accordance with some embodiments.

The robotic medical system includes one or more processors 380, which are in communication with a computer readable storage medium 382 (e.g., computer memory devices, such as random-access memory, read-only memory, static random-access memory, and non-volatile memory, and other storage devices, such as a hard drive, an optical disk, a magnetic tape recording, or any combination thereof) storing instructions for performing any methods described herein (e.g., operations described with respect to FIGS. 30A-30E and 31A-31C). The one or more processors 380 are also in communication with an input/output controller 384 (via a system bus or any suitable electrical circuit). The input/output controller 384 receives sensor data from one or more sensors 388-1, 388-2, etc., and relays the sensor data to the one or more processors 380. The input/output controller 384 also receives instructions and/or data from the one or more processors 380 and relays the instructions and/or data to one or more actuators, such as first motors 387-1 and 387-2, etc. In some embodiments, the input/output controller 384 is coupled to one or more actuator controllers 386 and provides instructions and/or data to at least a subset of the one or more actuator controllers 386, which, in turn, provide control signals to selected actuators. In some embodiments, the one or more actuator controller 386 are integrated with the input/output controller 384 and the input/output controller 384 provides control signals directly to the one or more actuators 387 (without a separate actuator controller). Although FIG. 32 shows that there is one actuator controller 386 (e.g., one actuator controller for the entire mobile medical platform, in some embodiments, additional actuator controllers may be used (e.g., one actuator controller for each actuator, etc.). In some embodiments, the one or more processors 380 are in communication with one or more displays 381 for displaying information (e.g., three-dimensional renderings) as described herein.

It should be noted that the terms "couple," "coupling," "coupled" or other variations of the word couple as used herein may indicate either an indirect connection or a direct connection. For example, if a first component is "coupled" to a second component, the first component may be either indirectly connected to the second component via another component or directly connected to the second component.

The functions for transitioning to a manual manipulation mode described herein may be stored as one or more instructions on a processor-readable or computer-readable medium. The term "computer-readable medium" refers to any available medium that can be accessed by a computer or processor. By way of example, and not limitation, such a medium may comprise random access memory (RAM), read-only memory (ROM), electrically erasable programmable read-only memory (EEPROM), flash memory, compact disc read-only memory (CD-ROM) or other optical disk storage, magnetic disk storage or other magnetic storage devices, or any other medium that can be used to store desired program code in the form of instructions or data structures and that can be accessed by a computer. It should be noted that a computer-readable medium may be tangible and non-transitory. As used herein, the term "code" may refer to software, instructions, code or data that is/are executable by a computing device or processor.

The methods disclosed herein comprise one or more steps or actions for achieving the described method. The method steps and/or actions may be interchanged with one another without departing from the scope of the disclosure. In other words, unless a specific order of steps or actions is required for proper operation of the method that is being described, the order and/or use of specific steps and/or actions may be modified without departing from the scope of the disclosure.

As used herein, the term "plurality" denotes two or more. For example, a plurality of components indicates two or more components. The term "determining" encompasses a wide variety of actions and, therefore, "determining" can include calculating, computing, processing, deriving, investigating, looking up (e.g., looking up in a table, a database or another data structure), ascertaining and the like. Also, "determining" can include receiving (e.g., receiving information), accessing (e.g., accessing data in a memory) and the like. Also, "determining" can include resolving, selecting, choosing, establishing and the like.

The phrase "based on" does not mean "based only on," unless expressly specified otherwise. In other words, the phrase "based on" describes both "based only on" and "based at least on."

## Claims

1. A robotic medical system (200), comprising: one or more robotic arms (210);
one or more displays (381);
one or more processors (380); and
memory storing instructions that, when executed by the one or more processors (380), cause the one or more processors (380) to:
display a three-dimensional (3-D) rendering that includes a graphical representation of the one or more robotic arms (210); and
update the 3-D rendering (400) in accordance with a pre-programmed workflow corresponding to a procedure, to guide a user through the procedure,
wherein the pre-programmed workflow includes one or more stages, including:
a pre-operative stage, an intra-operative stage, and/or a post-operative stage, wherein each of the one or more stages of the pre-programmed workflow includes one or more respective steps,
wherein the memory further includes instructions that, when executed by the one or more processors (380), cause the one or more processors to (380):
identify a step of the procedure to which the robotic medical system (200) corresponds;
cause movement of a portion of a first robotic arm of the one or more robotic arms (210) in accordance with the identified step; and
during the movement, update the 3-D rendering (400) to:
display the portion of the first robotic arm with a first visual representation that is visually distinct from other portions of the first robotic arm; and
display a positional change of the portion of the first robotic arm according to the executed movement.

2. The robotic medical system (200) of claim 1, further comprising: a patient support platform (202); and
the memory further includes instructions that, when executed by the one or more processors (380), cause the one or more processors (380) to:
execute a spatial configuration adjustment of the one or more robotic arms (210) relative to the patient support platform (202) in accordance with the workflow; and
update the 3-D rendering (400) to reflect positional changes of the one or more robotic arms (210) according to the spatial configuration adjustment,
optionally wherein the memory further includes instructions that, when executed by the one or more processors (380), cause the one or more processors (380) to:
cause movement of the patient support platform (202) from a first position to a second position in accordance with the workflow; and
update the 3-D rendering (400) to reflect the movement of the patient support platform (202).

3. The robotic medical system (200) of claim 1, further comprising:
one or more adjustable arm supports (220) that are movably coupled to the one or more robotic arms (210); and
the memory further includes instructions that, when executed by the one or more processors (380), cause the one or more processors (380) to:
cause movement of the one or more adjustable arm supports (220) relative to the one or more robotic arms (210) in accordance with the workflow; and
update the 3-D rendering (400) to reflect a positional change of the one or more adjustable arm supports (220).

4. The robotic medical system (200) of claim 1, wherein the memory further includes instructions that, when executed by the one or more processors (380), cause the one or more processors (380) to:
receive user selection of a portion of the 3-D rendering (400); and
in accordance with the user selection, display the user-selected portion in a visually distinctive manner from other portions of the 3-D rendering (400).

5. The robotic medical system (200) of claim 1, wherein a step of the pre-operative stage includes deploying the one or more robotic arms (210) from a stowed position into a deployed position.

6. The robotic medical system (200) of claim 1, wherein a step of the pre-operative stage includes moving the one or more robotic arms (210) into a draping pose.

7. The robotic medical system (200) of claim 1, wherein a step of the pre-operative stage includes placing the one or more robotic arms (210) in a docked state.

8. The robotic medical system (200) of claim 1, further comprising:
an adjustable arm support (220) that is movably coupled to the one or more robotic arms (210); and
a patient support platform (202),
wherein a step of the intra-operative procedure includes leveling the adjustable arm support (220) and the patient support platform (202).

9. The robotic medical system (200) of claim 1, wherein the memory further includes instructions
optionally:
wherein displaying the portion of the first robotic arm with the first visual representation comprises displaying the portion with a first color that is distinct from a color corresponding to the other portions of the first robotic arm, or
wherein the memory further includes instructions that, when executed by the one or more processors (380), cause the one or more processors (380) to during the movement, display a progress bar (422) for visualizing progress of the identified step that is being executed, or
wherein the memory further includes instructions that, when executed by the one or more processors (380), cause the one or more processors (380) to, in accordance with the movement, produce an audio signal, or
wherein the memory further includes instructions that, when executed by the one or more processors (380), cause the one or more processors (380) to:
determine whether the step has been completed; and
in accordance with a determination that the step has been completed, update the 3-D rendering (400) to display the portion of the first robotic arm in a second visual representation that is distinct from the first visual representation,
optionally wherein the first visual representation corresponds to a first color and the second visual representation corresponds to a second color that is distinct from the first color.

10. The robotic medical system (200) of claim 1, further comprising:
an adjustable arm support (220) that is movably coupled to the one or more robotic arms (210) are coupled; and
the memory further includes instructions that, when executed by the one or more processors (380), cause the one or more processors (380) to:
identify a step of procedure to which the robotic medical system (200) corresponds;
cause movement of the adjustable arm support (220) in accordance with the identified step; and
during the movement, update the 3-D rendering (400) to:
display the adjustable arm support (220) with a first visual representation that is visually distinct from the one or more robotic arms (210); and
display a positional change of the adjustable arm support (220)
according to the movement,
optionally wherein displaying the adjustable arm support (220) with the first visual representation comprises displaying the displaying the adjustable arm support (220) with a first color that is distinct from a color corresponding to the one or more robotic arms (210).

11. The robotic medical system (200) of claim 1, further comprising:
a patient support platform (202);
the memory further includes instructions that, when executed by the one or more processors (380), cause the one or more processors (380) to:
identify a step of procedure to which the robotic medical system (200) corresponds;
cause movement of at least a portion of the patient support platform (202); and
during the movement, update the 3-D rendering (400) to:
display the at least a portion of the patient support platform (202) with a first visual representation that is visually distinct from the one or more robotic arms (210) and/or other portions of the patient support platform (202); and display a positional change of the at least a portion of the patient support platform (202) according to the movement, optionally wherein displaying the at least a portion of the patient support platform (202) with the first visual representation comprises displaying the at least a portion of the patient support platform (202) with a first color that is distinct from a color corresponding to the one or more robotic arms (210).

12. The robotic medical system (200) of claim 1, wherein the memory further includes instructions that, when executed by the one or more processors (380), cause the one or more processors (380) to:
in accordance with a determination that the robotic medical system (200) is executing a specific step of the workflow, adjust a field of view of a virtual camera of the robotic medical system (200) to include a specific portion of the one or more robotic arms (210).

13. The robotic medical system (200) of claim 1, wherein the memory further includes instructions that, when executed by the one or more processors (380), cause the one or more processors (380) to:
in accordance with a determination that the robotic medical system (200) is executing a specific step of the workflow:
generate a graphic (804); and
display the graphic (804) as an overlay on the 3-D rendering (400).

## Patentansprüche

1. Medizinisches Robotersystem (200), umfassend: einen oder mehrere Roboterarme (210);
eine oder mehrere Anzeigen (381);
einen oder mehrere Prozessoren (380); und
einen Speicher, der Anweisungen speichert, die, wenn sie durch den einen oder die mehreren Prozessoren (380) ausgeführt werden, den einen oder die mehreren Prozessoren (380) veranlassen zum:
Anzeigen eines dreidimensionalen (3-D) Rendering, das eine grafische Darstellung des einen oder der mehreren Roboterarme (210) einschließt; und
Aktualisieren des 3-D-Rendering (400) gemäß einem vorprogrammierten Arbeitsablauf, der einem Eingriff entspricht, um einen Benutzer durch den Eingriff zu führen,
wobei der vorprogrammierte Arbeitsablauf eine oder mehrere Stadien einschließt, einschließlich:
ein präoperatives Stadium, ein intraoperatives Stadium und/oder ein postoperatives Stadium, wobei jedes des eines oder der mehreren Stadien des vorprogrammierten Arbeitsablaufs einen oder mehrere jeweilige Schritte einschließt,
wobei der Speicher ferner Anweisungen einschließt, die, wenn sie durch den einen oder die mehreren Prozessoren (380) ausgeführt werden, die Prozessoren veranlassen zum (380):
Identifizieren eines Schritts des Eingriffs, dem das medizinische Robotersystem (200) entspricht;
Veranlassen einer Bewegung eines Abschnitts eines ersten Roboterarms des einen oder der mehreren Roboterarme (210) gemäß dem identifizierten Schritt; und
während der Bewegung, Aktualisieren des 3-D-Rendering (400) zum:
Anzeigen des Abschnitts des ersten Roboterarms mit einer ersten visuellen Darstellung, die sich von anderen Abschnitten des ersten Roboterarms visuell unterscheidet; und
Anzeigen einer Positionsänderung des Abschnitts des ersten Roboterarms gemäß der ausgeführten Bewegung.

2. Medizinisches Robotersystem (200) nach Anspruch 1, ferner umfassend: eine Patientenstützplattform (202); und
der Speicher ferner Anweisungen einschließt, die, wenn sie durch den einen oder die mehreren Prozessoren (380) ausgeführt werden, den einen oder die mehreren Prozessoren (380) veranlassen zum:
Ausführen einer räumlichen Konfigurationseinstellung des einen oder der mehreren Roboterarme (210) relativ zu der Patientenstützplattform (202) gemäß dem Arbeitsablauf; und
Aktualisieren des 3-D-Rendering (400), um Positionsänderungen des einen oder der mehreren Roboterarme (210) gemäß der räumlichen Konfigurationseinstellung zu reflektieren, optional wobei der Speicher ferner Anweisungen einschließt, die, wenn sie durch den einen oder die mehreren Prozessoren (380) ausgeführt werden, den einen oder die mehreren Prozessoren (380) veranlassen zum:
Veranlassen der Bewegung der Patientenstützplattform (202) von einer ersten Position in eine zweite Position gemäß dem Arbeitsablauf; und
Aktualisieren des 3-D-Rendering (400), um die Bewegung der Patientenstützplattform (202) zu reflektieren.

3. Medizinisches Robotersystem (200) nach Anspruch 1, ferner umfassend:
eine oder mehrere einstellbare Armstützen (220), die mit dem einen oder den mehreren Roboterarmen (210) bewegbar gekoppelt sind; und
der Speicher ferner Anweisungen einschließt, die, wenn sie durch den einen oder die mehreren Prozessoren (380) ausgeführt werden, den einen oder die mehreren Prozessoren (380) veranlassen zum:
Veranlassen der Bewegung der einen oder der mehreren einstellbaren Armstützen (220) relativ zu dem einen oder den mehreren Roboterarmen (210) gemäß dem Arbeitsablauf; und
Aktualisieren des 3-D-Rendering (400), um eine Positionsänderung der einen oder der mehreren einstellbaren Armstützen (220) zu reflektieren.

4. Medizinisches Robotersystem (200) nach Anspruch 1, wobei der Speicher ferner Anweisungen einschließt, die, wenn sie durch den einen oder die mehreren Prozessoren (380) ausgeführt werden, den einen oder die mehreren Prozessoren (380) veranlassen zum:
Empfangen einer Benutzerauswahl eines Abschnitts des 3-D-Rendering (400); und
gemäß der Benutzerauswahl, Anzeigen des von dem Benutzer ausgewählten Abschnitts in einer visuell von anderen Abschnitten unterscheidbaren Weise des 3-D-Rendering (400).

5. Medizinisches Robotersystem (200) nach Anspruch 1, wobei ein Schritt des präoperativen Stadiums ein Ausfahren des einen oder der mehreren Roboterarme (210) aus einer eingezogenen Position in eine ausgefahrene Position einschließt.

6. Medizinisches Robotersystem (200) nach Anspruch 1, wobei ein Schritt des präoperativen Stadiums das Bewegen des einen oder der mehreren Roboterarme (210) in eine Abdeckungspose einschließt.

7. Medizinisches Robotersystem (200) nach Anspruch 1, wobei ein Schritt des präoperativen Stadiums ein Platzieren des einen oder der mehreren Roboterarme (210) in einen angedockten Zustand einschließt.

8. Medizinisches Robotersystem (200) nach Anspruch 1, ferner umfassend:
eine einstellbare Armstütze (220), die mit dem einen oder den mehreren Roboterarmen (210) bewegbar gekoppelt ist; und
eine Patientenstützplattform (202),
wobei ein Schritt des intraoperativen Eingriffs ein Nivellieren der einstellbaren Armstütze (220) und der Patientenstützplattform (202) einschließt.

9. Medizinisches Robotersystem (200) nach Anspruch 1, wobei der Speicher ferner Anweisungen einschließt
optional:
wobei das Anzeigen des Abschnitts des ersten Roboterarms mit der ersten visuellen Darstellung das Anzeigen des Abschnitts mit einer ersten Farbe umfasst, die sich von einer Farbe unterscheidet, die den anderen Abschnitten des ersten Roboterarms entspricht, oder
wobei der Speicher ferner Anweisungen einschließt, die, wenn sie durch den einen oder die mehreren Prozessoren (380) ausgeführt werden, den einen oder die mehreren Prozessoren (380) veranlassen, während der Bewegung eine Fortschrittsanzeige (422) für ein Visualisieren des Fortschritts des identifizierten Schritts, der ausgeführt wird, anzuzeigen, oder
wobei der Speicher ferner Anweisungen einschließt, die, wenn sie durch den einen oder die mehreren Prozessoren (380) ausgeführt werden, den einen oder die mehreren Prozessoren (380) veranlassen, gemäß der Bewegung ein Audiosignal zu produzieren, oder
wobei der Speicher ferner Anweisungen einschließt, die, wenn sie durch den einen oder die mehreren Prozessoren (380) ausgeführt werden, den einen oder die mehreren Prozessoren (380) veranlassen zum:
Bestimmen, ob der Schritt abgeschlossen wurde; und
gemäß einer Bestimmung, dass der Schritt abgeschlossen wurde, Aktualisieren des 3-D-Rendering (400), um den Abschnitt des ersten Roboterarms in einer zweiten visuellen Darstellung anzuzeigen, die sich von der ersten visuellen Darstellung unterscheidet,
optional wobei die erste visuelle Darstellung einer ersten Farbe entspricht und die zweite visuelle Darstellung einer zweiten Farbe entspricht, die sich von der ersten Farbe unterscheidet.

10. Medizinisches Robotersystem (200) nach Anspruch 1, ferner umfassend:
eine einstellbare Armstütze (220), die mit dem einen oder den mehreren Roboterarmen (210) bewegbar gekoppelt ist, gekoppelt sind; und
der Speicher ferner Anweisungen einschließt, die, wenn sie durch den einen oder die mehreren Prozessoren (380) ausgeführt werden, den einen oder die mehreren Prozessoren (380) veranlassen zum:
Identifizieren eines Schritts eines Eingriffs, dem das medizinische Robotersystem (200) entspricht;
Veranlassen der Bewegung der einstellbaren Armstütze (220) gemäß dem identifizierten Schritt; und
während der Bewegung, Aktualisieren des 3-D-Rendering (400) zum:
Anzeigen der einstellbaren Armstütze (220) mit einer ersten visuellen Darstellung, die sich von dem einen oder den mehreren Roboterarmen (210) visuell unterscheidet; und
Anzeigen einer Positionsänderung der einstellbaren Armstütze (220) gemäß der Bewegung,
optional wobei das Anzeigen der einstellbaren Armstütze (220) mit der ersten visuellen Darstellung das Anzeigen der einstellbaren Armstütze (220) mit einer ersten Farbe umfasst, die sich von einer Farbe unterscheidet, die dem einen oder den mehreren Roboterarmen (210) entspricht.

11. Medizinisches Robotersystem (200) nach Anspruch 1, ferner umfassend:
eine Patientenstützplattform (202);
der Speicher ferner Anweisungen einschließt, die, wenn sie durch den einen oder die mehreren Prozessoren (380) ausgeführt werden, den einen oder die mehreren Prozessoren (380) veranlassen zum:
Identifizieren eines Schritt eines Eingriffs, dem das medizinische Robotersystem (200) entspricht;
Veranlassen der Bewegung von mindestens einem Abschnitt der Patientenstützplattform (202); und
während der Bewegung, Aktualisieren des 3-D-Rendering (400) zum:
Anzeigen des mindestens einen Abschnitts der Patientenstützplattform (202) mit einer ersten visuellen Darstellung, die sich von dem einen oder den mehreren Roboterarmen (210) und/oder anderen Abschnitten der Patientenstützplattform (202) visuell unterscheidet; und Anzeigen einer Positionsänderung des mindestens einen Abschnitts der Patientenstützplattform (202) gemäß der Bewegung, optional wobei das Anzeigen des mindestens einen Abschnitts der Patientenstützplattform (202) mit der ersten visuellen Darstellung das Anzeigen des mindestens einen Abschnitts der Patientenstützplattform (202) mit einer ersten Farbe umfasst, die sich von einer Farbe unterscheidet, die dem einen oder den mehreren Roboterarmen (210) entspricht.

12. Medizinisches Robotersystem (200) nach Anspruch 1, wobei der Speicher ferner Anweisungen einschließt, die, wenn sie durch den einen oder die mehreren Prozessoren (380) ausgeführt werden, den einen oder die mehreren Prozessoren (380) veranlassen zum:
gemäß einer Bestimmung, dass das medizinische Robotersystem (200) einen spezifischen Schritt des Arbeitsablaufs ausführt, Einstellen eines Sichtfelds einer virtuellen Kamera des medizinischen Robotersystems (200), um einen spezifischen Abschnitt des einen oder der mehreren Roboterarme (210) einzuschließen.

13. Medizinisches Robotersystem (200) nach Anspruch 1, wobei der Speicher ferner Anweisungen einschließt, die, wenn sie durch den einen oder die mehreren Prozessoren (380) ausgeführt werden, den einen oder die mehreren Prozessoren (380) veranlassen zum:
gemäß einer Bestimmung, dass das medizinische Robotersystem (200) einen spezifischen Schritt des Arbeitsablaufs ausführt:
Erzeugen einer Grafik (804); und
Anzeigen der Grafik (804) als eine Überlagerung auf dem 3-D-Rendering (400).

## Revendications

1. Système médical robotique (200), comprenant : un ou plusieurs bras robotiques (210) ;
un ou plusieurs affichages (381) ;
un ou plusieurs processeurs (380) ; et
de la mémoire stockant des instructions qui, lorsqu'elles sont exécutées par le ou les processeurs (380), amènent le ou les processeurs (380) à :
afficher une restitution tridimensionnelle (en 3D) qui comporte une représentation graphique du ou des bras robotiques (210) ; et
mettre à jour la restitution en 3D (400) conformément à un flux de travail préprogrammé correspondant à une procédure, pour guider un utilisateur au cours de la procédure,
dans lequel le flux de travail préprogrammé comporte une ou plusieurs phases, comportant :
une phase préopératoire, une phase peropératoire et/ou une phase postopératoire, dans lequel chacune parmi la ou les phases du flux de travail préprogrammé comporte une ou plusieurs étapes respectives,
dans lequel la mémoire comporte en outre des instructions qui, lorsqu'elles sont exécutées par le ou les processeurs (380), amènent le ou les processeurs à (380) :
identifier une étape de la procédure à laquelle le système médical robotique (200) correspond ;
amener un mouvement d'une partie d'un premier bras robotique du ou des bras robotiques (210) conformément à l'étape identifiée ; et
pendant le mouvement, mettre à jour la restitution en 3D (400) pour :
afficher la partie du premier bras robotique avec une première représentation visuelle qui est visuellement distincte d'autres parties du premier bras robotique ; et
afficher un changement de position de la partie du premier bras robotique selon le mouvement exécuté.

2. Système médical robotique (200) selon la revendication 1, comprenant en outre : une plate-forme de support de patient (202) ; et
la mémoire comporte en outre des instructions qui, lorsqu'elles sont exécutées par le ou les processeurs (380), amènent le ou les processeurs (380) à :
exécuter un ajustement de configuration spatiale du ou des bras robotiques (210) par rapport à la plate-forme de support de patient (202) conformément au flux de travail ; et
mettre à jour la restitution en 3D (400) pour refléter les changements de position du ou des bras robotiques (210) selon l'ajustement de configuration spatiale, facultativement dans lequel la mémoire comporte en outre des instructions qui, lorsqu'elles sont exécutées par le ou les processeurs (380), amènent le ou les processeurs (380) à :
amener un mouvement de la plate-forme de support de patient (202) d'une première position à une seconde position conformément au flux de travail ; et
mettre à jour la restitution en 3D (400) pour refléter le mouvement de la plate-forme de support de patient (202).

3. Système médical robotique (200) selon la revendication 1, comprenant en outre :
un ou plusieurs supports de bras ajustables (220) qui sont accouplés de manière mobile au ou aux bras robotiques (210) ; et
la mémoire comporte en outre des instructions qui, lorsqu'elles sont exécutées par le ou les processeurs (380), amènent le ou les processeurs (380) à :
amener un mouvement du ou des supports de bras ajustables (220) par rapport au ou aux bras robotiques (210) conformément au flux de travail ; et
mettre à jour la restitution en 3D (400) pour refléter un changement de position du ou des supports de bras ajustables (220).

4. Système médical robotique (200) selon la revendication 1, dans lequel la mémoire comporte en outre des instructions qui, lorsqu'elles sont exécutées par le ou les processeurs (380), amènent le ou les processeurs (380) à :
recevoir une sélection utilisateur d'une partie de la restitution en 3D (400) ; et
conformément à la sélection utilisateur, afficher la partie sélectionnée par l'utilisateur d'une manière visuellement distincte des autres parties de la restitution en 3D (400).

5. Système médical robotique (200) selon la revendication 1, dans lequel une étape de la phase préopératoire comporte le déploiement du ou des bras robotiques (210) d'une position rangée à une position déployée.

6. Système médical robotique (200) selon la revendication 1, dans lequel une étape de la phase préopératoire comporte un déplacement du ou des bras robotiques (210) dans une posture de placement de champ opératoire.

7. Système médical robotique (200) selon la revendication 1, dans lequel une étape de la phase préopératoire comporte le placement du ou des bras robotiques (210) dans un état arrimé.

8. Système médical robotique (200) selon la revendication 1, comprenant en outre :
un support de bras ajustable (220) qui est accouplé de manière mobile au ou aux bras robotiques (210) ; et
une plate-forme de support de patient (202),
dans lequel une étape de la procédure peropératoire comporte la mise à niveau du support de bras ajustable (220) et de la plate-forme de support de patient (202).

9. Système médical robotique (200) selon la revendication 1, dans lequel la mémoire comporte en outre des instructions
facultativement :
dans lequel l'affichage de la partie du premier bras robotique avec la première représentation visuelle comprend l'affichage de la partie avec une première couleur qui est distincte d'une couleur correspondant aux autres parties du premier bras robotique, ou
dans lequel le mémoire comporte en outre des instructions qui, lorsqu'elles sont exécutées par le ou les processeurs (380), amènent le ou les processeurs (380) à, pendant le mouvement, afficher une barre de progression (422) permettant de visualiser l'avancement de l'étape identifiée qui est en cours d'exécution, ou
dans lequel la mémoire comporte en outre des instructions qui, lorsqu'elles sont exécutées par le ou les processeurs (380), amènent le ou les processeurs (380) à, conformément au mouvement, produire un signal audio, ou
dans lequel la mémoire comporte en outre des instructions qui, lorsqu'elles sont exécutées par le ou les processeurs (380), amènent le ou les processeurs (380) à :
déterminer si l'étape est achevée ; et
conformément à une détermination que l'étape est achevée, mettre à jour la restitution en 3D (400) pour afficher la partie du premier bras robotique dans une seconde représentation visuelle qui est distincte de la première représentation visuelle,
facultativement dans lequel la première représentation visuelle correspond à une première couleur et la seconde représentation visuelle correspond à une seconde couleur qui est distincte de la première couleur.

10. Système médical robotique (200) selon la revendication 1, comprenant en outre :
un support de bras ajustable (220) qui est accouplé de manière mobile au ou aux bras robotiques (210) qui sont accouplés ; et
la mémoire comporte en outre des instructions qui, lorsqu'elles sont exécutées par le ou les processeurs (380), amènent le ou les processeurs (380) à :
identifier une étape de procédure à laquelle le système médical robotique (200) correspond ;
amener un mouvement du support de bras ajustable (220) conformément à l'étape identifiée ; et
pendant le mouvement, mettre à jour la restitution en 3D (400) pour :
afficher le support de bras ajustable (220) avec une première représentation visuelle qui est visuellement distincte du ou des bras robotiques (210) ; et
afficher un changement de position du support de bras ajustable (220) selon le mouvement,
facultativement dans lequel l'affichage du support de bras ajustable (220) avec la première représentation visuelle comprend l'affichage de l'affichage du support de bras ajustable (220) avec une première couleur qui est distincte d'une couleur correspondant au ou aux bras robotiques (210).

11. Système médical robotique (200) selon la revendication 1, comprenant en outre :
une plate-forme de support de patient (202) ;
la mémoire comporte en outre des instructions qui, lorsqu'elles sont exécutées par le ou
les processeurs (380), amènent le ou les processeurs (380) à :
identifier une étape de procédure à laquelle le système médical robotique (200) correspond ;
amener un mouvement d'au moins une partie de la plate-forme de support de patient (202) ; et
pendant le mouvement, mettre à jour la restitution en 3D (400) pour :
afficher l'au moins une partie de la plate-forme de support de patient (202) avec une première représentation visuelle qui est visuellement distincte du ou des bras robotiques (210) et/ou d'autres parties de la plate-forme de support de patient (202) ; et afficher un changement de position de l'au moins une partie de la plate-forme de support de patient (202) selon le mouvement, facultativement dans lequel l'affichage de l'au moins une partie de la plate-forme de support de patient (202) avec la première représentation visuelle comprend l'affichage de l'au moins une partie de la plate-forme de support de patient (202) avec une première couleur qui est distincte d'une couleur correspondant au ou aux bras robotiques (210).

12. Système médical robotique (200) selon la revendication 1, dans lequel la mémoire comporte en outre des instructions qui, lorsqu'elles sont exécutées par le ou les processeurs (380), amènent le ou les processeurs (380) à :
conformément à une détermination que le système médical robotique (200) est en train d'exécuter une étape spécifique du flux de travail, ajuster un champ de vision d'une caméra virtuelle du système médical robotique (200) pour comporter une partie spécifique du ou des bras robotiques (210).

13. Système médical robotique (200) selon la revendication 1, dans lequel la mémoire comporte en outre des instructions qui, lorsqu'elles sont exécutées par le ou les processeurs (380), amènent le ou les processeurs (380) à :
conformément à une détermination que le système médical robotique (200) est en train d'exécuter une étape spécifique du flux de travail :
générer un graphique (804) ; et
afficher le graphique (804) en tant que recouvrement sur la restitution en 3D (400).
